# EUROPEAN PATENT SPECIFICATION

(11) **EP 4 608 465 B1**
(45) Date of publication and mention of the grant of the patent: **11.11.2026**
(21) Application number: 23813217.9
(22) Date of filing: 26.10.2023
(51) Int. Cl.: A61L 31/10, A61L 31/14, A61L 31/16, A61F 6/04, C09J 133/04

(54) **ENHANCED SENSATION CONDOM**
KONDOM MIT VERBESSERTER EMPFINDUNG
PRÉSERVATIF À SENSATION AMÉLIORÉE

(30) Priority: 26.10.2022 US 202263381071 P; 31.10.2022 US 202263381653 P; 02.04.2023 US 202363493761 P; 02.04.2023 US 202363493762 P; 10.05.2023 US 202363501237 P; 10.05.2023 US 202363501238 P
(43) Date of publication of application: 03.09.2025
(73) Proprietor: Nuceptive Labs, Inc., Boston, Massachusetts 02215 (US)
(72) Inventor: HEARON II, Michael Keith, Boston, Massachusetts 02215 (US); LARSON, David F., Boston, Massachusetts 02215 (US); GRINSTAFF, Mark W., Boston, Massachusetts 02215 (US); FITZGERALD, Danielle, Boston, Massachusetts 02215 (US)
(74) Representative: Elkington and Fife LLP
(86) International application number: PCT/US2023/077970
(87) International publication number: WO 2024/092168

(56) References cited:
- EP-B1- 0 746 289
- WO-A1-00/62723
- WO-A1-90/13420
- WO-A1-91/14461
- WO-A1-91/14462

## Description

### 1. BACKGROUND

A female diaphragm or cap is a device which acts as a barrier method of contraception. It fits inside the vagina and prevents sperm passing through the cervix. The female diaphragm does not prevent the spread of sexually transmitted infections (STIs). From a pleasure standpoint, the diaphragm provides for near-optimal contact between the penis and vagina.

Conventional male condoms are a latex or polyurethane device that completely cover the penis and attempt to trap sperm within it to prevent pregnancy as well as the spread of certain STIs. As condoms substantially cover the penis head and shaft, they decrease the sensation of pleasure. This reduction in pleasure reduces the use of condoms during sex and results in unwanted pregnancies or STI spread, resulting in substantially altered human livelihood in many situations and increasing overall healthcare costs.

Fractional male condoms are known in the art, i.e., condoms covering less than a conventional male condom. The GALACTIC CAP^{™} is a commercially available fractional condom marketed for pregnancy prevention (http://www.galacticcap.com/). It is made of polyurethane barrier layer with an adhesive backing and attaches to the penis head and shaft. However, it suffers several important limitations. It can only be applied in an aroused state and to the extent it still covers a significant portion of the penis shaft, user sensation of pleasure is correspondingly limited. The condom is made from a material that cannot be used with oil lubricants that are otherwise popular among users. The design is complex, making the condom difficult to apply and leading to the possibility of user error. The condom is also difficult (awkward, painful) to remove. As shown in the user instructions, removal requires the use of baby oil and the recommended (easiest) mode of removal requires the user to urinate into the condom, to inflate the same and then peel off. Even apart from the awkwardness this brings to the removal experience, it is also known to be painful. See, e.g., https://www.vice.com/en/article/znmmp4/i-tried-the-latest-in-cndom-technology-and-it-went-shockingly-well. Leaks have also been reported. The above limitations have significantly limited the impact of the GALATIC CAP^{™}'s appeal to consumers.

Other fractional condoms have been reported in the art, subject to similar limitations.

Wondaleaf Cap^{®} is a commercially available fractional condom that comprises an adhesive coating at the condom opening to stick onto the penile shaft and onto itself to form the proximal portion of the condom and two lateral tabs. The loose-fitting barrier layer is made of polyurethane. The user removes the condom by holding tabs on the side of the device and pulling the condom distally with counter pressure on the penile skin. Similar to GALATIC CAP^{™}, the device can only be applied in an aroused state and to the extent it still covers a significant portion of the penis shaft, user sensation of pleasure is correspondingly limited. The design is also complex, creating the potential for user error (https://www.wondaleaf.com/wondaleaf-cap/).

WO2014178661A describes a fractional condom with a barrier layer made from polyurethane and having a discontinuous adhesive layer and a pre-formed semen reservoir that protrudes from the device prior to use (i.e., the condom is non-planar prior to use). The adhesive is said to be double-sided tape, e.g., 3M double-sided tape, or generally a "medical adhesive layer" or pressure sensitive adhesive.

US 11,234,858 describes a body fluid collection device comprising a fitting component and a collection film applied to the penis via an adhesive layer. The collecting film provides a pre-formed semen reservoir (i.e., the condom is non-planar prior to use). The fitting component and the collection film are typically non-continuous, in a structure said to avoid semen leakage more effectively. The constituent material of the adhesive layer may include, for example, a pressure-sensitive adhesive.

WO 00/62723 Aldiscloses a condom comprising:a barrier layer suitable for prohibiting passage of semen and comprising an inner surface, and outer surface, and an adhesive layer adhered to at least a portion of the inner surface of the barrier layer;wherein:the condom is configured for adhesion of the adhesive layer to the glans of the penis of a human subject,the condom is configured to provide fractional coverage of the penis, wherein, when the condom is applied to the penis, the adhesive layer comprises an acrylic pressure sensitive adhesive which adheres to the glans of the penis, and the adhesive layer can be removed from the glans of the penis following application of a stimulus to the condom. The condom is sized and shaped such that, when applied to the penis, the condom does not contact the corona and the shaft of the penis. The condom comprises a semen reservoir having a rounded shape. The adhesive is present on the outer area of the inner surface.

There is a need for contraceptive devices that prevent pregnancy and provide increased sensation in comparison with that of existing contraceptive devices yet offers ease of application and removal.

### 2. SUMMARY

In one aspect, the present disclosure provides a condom comprising a barrier layer suitable for prohibiting passage of semen and comprising an inner surface and outer surface, and an adhesive layer adhered to at least a portion of the inner surface of the barrier layer; wherein the condom is configured for adhesion of the adhesive layer to the glans of the penis of a human subject, the condom is configured to provide fractional coverage of the penis (e.g., a glans sheath), and the adhesive layer comprises an adhesive that comprises a stimuli-responsive polymer formed from one or more monomers (e.g., acrylate or methacrylate monomers) and one or more polyfunctional crosslinkers (e.g., trifunctional crosslinkers), wherein the stimuli-responsive polymer is a crosslinked polymer having a heterogeneous crosslink network, and wherein when the condom is applied to the penis, the adhesive layer adheres to the glans of the penis, and the adhesive layer can be removed from the glans of the penis following application of a stimulus (e.g., a mechanical stimulus) to the condom. In some embodiments, the fractional condom is planar prior to use. In some embodiments, the barrier layer further comprises a self-forming semen reservoir. In some embodiments, the adhesive layer is coextensive with the barrier layer. In some embodiments, the adhesive layer is the sole means for securing the condom to the penis.

In some embodiments, the fractional coverage is limited to the tip of the penis. In some embodiments, the fractional coverage is limited to the head of the penis. In some embodiments, the fractional coverage does not include the shaft of the penis. In some embodiments, the fractional coverage includes the base of the penis or below the base of the penis.

In some embodiments, the condom permits on-demand delamination in response to a stimulus, wherein delamination produces substantially no pain to the user and leaves substantially no residue or residue that is removed in a facile manner.

In another aspect, the present disclosure provides a package comprising a condom described herein.

In still another aspect, the present disclosure provides a kit comprising (a) a condom described herein or a package comprising a condom described herein and (b) instructions for use.

In yet another aspect, the present disclosure provides a method of applying a condom of disclosed herein to the penis of a human subject comprising contacting the adhesive layer of the condom to the glans of the penis and applying pressure to the condom sufficient to adhere the condom to the glans of the penis.

In a further aspect, the present disclosure provides a method of removing a condom disclosed herein from the penis of a human subject comprising applying a stimulus to the condom whose adhesive layer is adhered to the glans of the penis and removing the condom from the glans of the penis.

### 3. BRIEF DESCRIPTION OF THE SEVERAL VIEWS OF THE DRAWINGS

These and other features, aspects, and advantages of the present disclosure will become better understood with regard to the following description, and accompanying drawings, where:

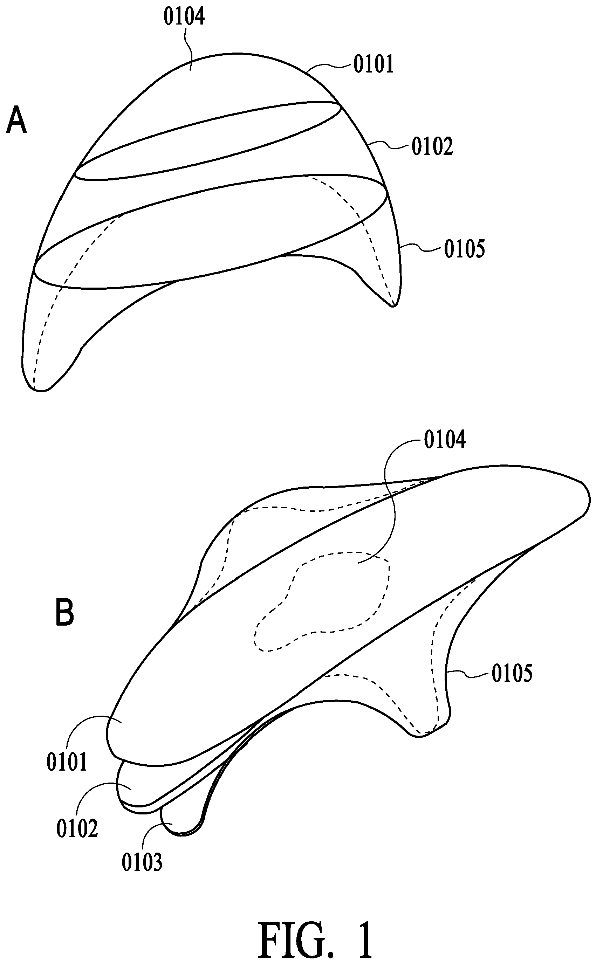

**FIG. 1A-B** shows perspective views of a complex-curvature condom (A) and planar condom (B). In panel A, a complex-curvature condom (one with non-zero gaussian curvature) comprises a continuous barrier layer (0101) and an adhesive layer (0102). An optional release liner (013) protects the adhesive layer prior to use to prevent sticking to itself or unintended surfaces and facilitates in user-handling. Optionally, the portion of the barrier layer covering the urethra meatus (opening) does not have adhesive (0104) to (a) mitigate unintended adhesion to the urethra and (b) provide a space for ejaculate fluids to flow into either a pre-formed or self-forming reservoir. In panel B, a planar condom, or one with zero gaussian curvature, comprises a continuous barrier layer (0101) and an adhesive layer (0102). An optional release liner (013) protects the adhesive layer prior to use to prevent sticking to itself or unintended surfaces and facilitates in user-handling. Optionally, a hole (0104) exists in the adhesive layer in the portion of the device covering the urethral meatus (opening), so as to (a) mitigate adhesion to the urethra and (b) provide a space for ejaculate fluids to flow into either a pre-formed or self-forming reservoir.
**FIG. 2** shows a cross-section of a condom comprised of a barrier layer (0201) and a two-adhesive pattern, consisting of a primary adhesive (0202) such as a water-soluble adhesive responsible for the primary attachment of the device to the glans, and a gasket adhesive (0203) that either enhances the sealing efficacy of the device or separates a water-soluble primary adhesive from aqueous vaginal or (pre-)ejaculate fluids.

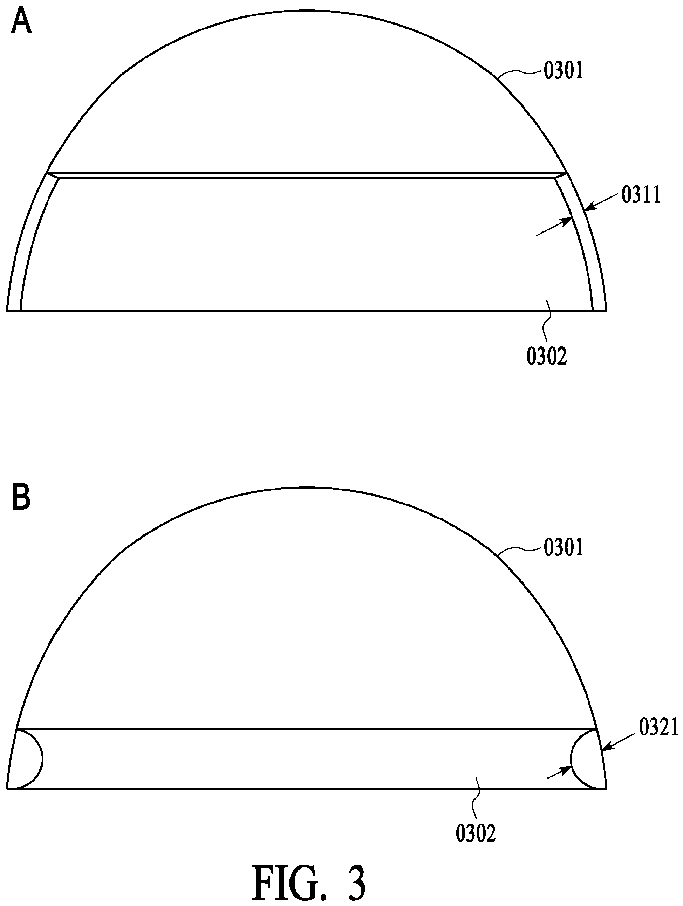

**FIG. 3A-B** shows cross-sections of an exemplary diaphragm described herein having (A) a constant thickness (0311) and (B) a non-constant thickness (0321).
**FIG. 4** shows a plot of adhesive strength of adhesives according to Example 10.
**FIG. 5** shows a graph of tensile strain capacity of adhesives according to Example 11.
**FIG. 6** shows a graph of tensile strain capacity of adhesives according to Example 11.
**FIG. 7** shows a graph of tensile strain capacity of adhesives according to Example 11.
**FIG. 8** shows a graph of peel strength of an adhesive according to Example 12 in a 180-degree peel test using a human skin substrate analog.
**FIG. 9** shows a graph of peel strength of adhesives according to Example 12 in a 180-degree peel test using a glass substrate.
**FIG. 10** shows a plot of tan(δ) for adhesives according to Example 13.
**FIG. 11** shows a plot of storage and loss modulus for adhesives according to Example 13.
**FIG. 12** shows a zoomed-in view of loss and storage moduli for L6 adhesive illustrating crossover between elastic and viscous regimes between at 0.05 MPA and 8 rad/sec.
**FIG. 13** shows a plot of the change in tan(δ) for adhesives according to Example 13 over the range of 0 °C to 50 °C.
**FIG. 14** shows DMA measurements of storage (G') and loss (G") moduli as a function of temperature for the L6 adhesive.
**FIG. 15** shows DMA measurements of tan(δ), the ratio of the loss modulus over the storage modulus, for the L6 adhesive.
**FIG. 16** shows DMA measurements of storage (G') and loss (G") moduli as a function of temperature for the L6 adhesive.
**FIG. 17** shows DMA measurements of tan(δ), the ratio of the loss modulus over the storage modulus, for the L6 adhesive.
**FIG. 18** shows DMA measurements of storage (G') and loss (G") moduli as a function of temperature for LMA and BA adhesives.
**FIG. 19** shows DMA measurements of tan(δ), the ratio of the loss modulus over the storage modulus, for LMA and BA adhesives.
**FIG. 20** shows the angular strain of the L6 adhesive at different temperatures.
**FIG. 21** shows the shear rate of the L6 adhesive at different temperatures.
**FIG. 22** shows the angular strain of the LMA and BA adhesives at different temperatures.
**FIG. 23** shows the shear rate of the LMA and BA adhesives at different temperatures.
**FIG. 24** shows the tack strength of the L6 adhesive at varied different temperatures.
**FIG. 25** shows the tack strength of LMA and BA adhesives at different temperatures.
**FIG. 26A-B** shows a perspective view of a condom. Panel B shows a cross-sectional side view of the embodiment of panel A.
**FIG. 27** shows a side view of a fractional condom.
**FIG. 28** shows a cross-sectional side view of fractional condom including a wrinkled barrier layer and a wrinkled adhesive layer.
**FIG. 29A-B** show cross-sectional perspective views of a fractional condom with a horizontally folded semen receptacle.
**FIG. 30A-B** show a planar condom adhered to a glans pre-ejaculation (A) and mid-ejaculation (B).
**FIG. 31** shows a plot of mechanical characteristics related to an embodiment's suitability for expansion and accommodation of ejaculate.
**FIG. 32** shows the gel fraction of the sol-gel analysis in Example 18 of various adhesives.
**FIG. 33** shows a Wong-Baker qualitative pain assessment for removing various condoms according to Example 19.

### 4. DETAILED DESCRIPTION

### 4.1. Definitions

When describing the embodiments of the present disclosure, the following terms, if present, have the following meanings, unless otherwise indicated. If not otherwise defined, terms have their customary meaning in the relevant art.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

"Adhesion," as used herein, refers to the ability of a composition or material to adhere or "stick" to a substrate (e.g., the skin). Adhesion is measured as adhesive force in Newtons (N). The higher the adhesive force, the higher will be the number of Newtons required to peel one object from the other.

"Adhesive," as used herein, refers to a composition or material that adheres to a substrate (e.g., the skin or a barrier layer).

"Bio-based," as used herein, refers to materials derived from natural sources.

"Biodegradable," as used herein, means used herein with reference to a composition or that can be degraded by living microorganisms like fungi or bacteria, without regard to a particular time frame or degradable by environmental conditions suitable for life such as ambient moisture and atmospheric conditions.

"Compostable," as used herein, means refers to a composition or article that require microorganisms, humidity, and heat to yield a finished compost product (CO2, water, inorganic compounds, and biomass). Compostable is distinguished from biodegradable in that compostable compositions and articles must break down into natural elements within a specific time frame. In one embodiment, the compositions and articles disclosed herein complies set forth by the U.S. Composting Council, Environmental Protection Agency, American Society for Testing and Materials (ASTM International), or Tuv Austria.

"Controlled porosity," as used herein, refers to pores which are normally closed that are stimulated to open, thereby allowing the passage of fluids as allowed by their rheological properties.

"Conventional condom," as used herein, refers to a condom that comprises a continuous elastic tubular wall with a closed distal end (tip) and an open proximal end, typically made of a thin, soft material such as latex or polyurethane, that when in use provides coverage on the penis head (i.e., the glans of the penis) and shaft. The length of a conventional condom in use is typically between about 7 and about 8 inches, but with commercially available products up to 9 inches and down to about 6.3 inches. Numerous condom brand manufacturers and products are known in the art. See, e.g., https://www.trojanbrands.com/en/products/condoms and https://www.durexusa.com/collections/condoms, each incorporated herein by reference.

"Crosslink Density," as used herein, refers to average molecular weight between crosslinks. (https://www.pcimag.com/articles/104955-calculation-of-crosslink-density-of-thermoset-polymers). Flory, et al., put forth theory in crosslink density in the 1940s.

"Curing," as used herein, refers to the chemical process of converting a macromolecule into a higher molecular weight polymer via crosslinking reactions.

"Debonding," as used herein, refers the mechanism of debonding may vary and include, for example, phase changes, chemical reactions, cross-linking and volumetric expansion.

"Elastomeric" or "elastomeric behavior," as used herein, refers to generally linear elastic or combined linear elastic and plastic deformation stress/strain behavior for a material as it is strained in a regime in a region above a key transition regime such that stress/strain hysteresis is generally conserved.

"Flexible" or "flexible behavior," as used herein, refers to behavior of a rigid, viscoelastic or elastomeric material that can be described as compliant or deformable to fit the demands of a specific engineering application.

"Fractional coverage," as used herein, refers to substantially less coverage of the penis that a conventional condom. In certain embodiments, the fractional condom disclosed herein does not contact or cover the shaft of the penis. In certain embodiments, the fractional condom does not contact or cover the corona and frenulum.

"Room temperature" (used interchangeably with "ambient temperature"), as used herein, refers to a temperature from 20-25°C.

"Selectively permeable," as used herein, refers to differential permeability. For example, a membrane contains channels or passages that allow specific molecules to pass through, either passively or actively, while not permitting passage of other molecules. Active transport through the membrane requires an energy input, which may be from a mechanical, acoustic, chemical, electrical, magnetic, pH change, ionic strength, thermal or optical source.

"Tg", as used herein, refers to the glass transition temperature of a polymer. At this temperature, polymers undergo a transition from glassy to rubbery state. Tg is an important feature of polymer behavior. It marks a region of dramatic changes in the physical and mechanical properties. Below a polymer's Tg, due to lack of mobility, the polymer is hard and brittle. Above a polymer's Tg, due to increased mobility, the polymer is soft and flexible.

"Stimuli-responsive," as used herein, refers to a change in physical, environmental, physico-chemical, thermomechanical, mechanical, thermal, energetic or other properties of a composition or material arising from exposure to a stimulus including, for example, a change in temperature, pH, ionic strength, environmental conditions including moisture, water immersion, exposure or humidity, solvent exposure, exposure to electromagnetic radiation including gamma rays, x-rays, ultraviolet rays, visible light, infrared waves, radio waves, ultrasonic waves, high humidity, magnetism, electricity, as well as mechanical forces, e.g., shear rate or peeling. In one embodiment, stimuli-responsive does not encompass application of liquid, such as baby oil, to the material to effect the change in properties of the material. In one embodiment, stimuli-responsive refers to a change in physical, environmental, physico-chemical, thermomechanical, mechanical, thermal, energetic or other properties of a composition or material previously applied to a substrate (e.g., skin) arising from exposure to a stimulus. A stimuli-responsive adhesive can be contrasted with a mechanically passive adhesive.

"Mechanically passive adhesive" refers to an adhesive that is that are designed to maintain their structural and mechanical properties after placement. Many pressure-sensitive adhesives are mechanically passive adhesives. In certain embodiments, the adhesive layer does not comprise a mechanically passive adhesive or pressure-sensitive adhesive.

"Shear rate," as used herein, refers to the rate at which a shearing deformation occurs. A shearing deformation is one whereby parallel lamellae (layers) of a fluid, gel or solid material moves past one another in a sliding manner. These layers may be discrete (possessing finite measurable thickness) or continuous (infinitesimally thin or not individually discernable). A shear rate may be a constant function in time, a monotonic function in time, a non-periodic non-monotonic or a periodic function in time. Periodic is understood to mean a function that is truly periodic in time, or is approximately periodic in time (e.g., a sinc function).

"Frequency responsive," as used herein, refers to the behavior of a material or fluid that changes under the application of a periodic or near-periodic force or displacement in time. This applied quantity can be called a "signal". The applied signal has a defined amplitude and frequency, and optionally phase, all of which may be constant or varying in time and/or space.

"Shear responsive," as used herein, refers to behavioral changes of a material (e.g., gel, solid, or liquid) under the application of different shear displacements or shear rates.

"Force responsive," as used herein, refers to behavioral changes of a material (e.g., gel, solid, or liquid) under the application of different magnitudes of force.

"Nonlinear force," as used herein, refers to a type of force in which the relationship between the force and its effect on a system is not proportional or does not follow a simple, linear equation.

"Network," as used herein with reference to a polymer, refers to macromolecular architecture formed by cross-linked polymer chains. Cross-links are covalent bonds or other strong interactions such as entanglements, supramolecular interactions, or physical interactions such as polymer chain interactions with crystalline or glassy phases.

"Interpenetrating network" ("IPN"), as used herein, refers to a unique type of polymer material that comprises two or more independent polymer networks, which are physically entangled but not covalently bonded to each other. Each network maintains its distinct identity, but the networks are intertwined at the molecular level, forming a composite material with properties derived from the combination of the individual networks.

"Semi-interpenetrating network" ("Semi-IPN"), as used herein, refers to a cross-linked or branched polymer network and an entangled linear or branched additional polymer or series of polymers.

"Heterogeneous network," as used herein, refers to a polymer network with a non-uniform crosslink density distribution.

"Gel Fraction" refers to the mass of a polymer remaining after washing with suitable solvent divided by initial mass. Means for determining the gel fraction are known in the art.

"Sol Fraction" refers to the mass of a polymer lost after washing with a substantial suitable solvent divided by initial mass. Means for determining the sol fraction are known in the art.

"Heterogeneous crosslinking," as used herein, refers to crosslinking distributed in a polymer network or system that is non-uniform.

"Monomer reactivity ratios," as used herein, refers to parameters used in polymer chemistry to describe the relative reactivity of two monomers in a copolymerization reaction. A copolymerization reaction involves the simultaneous polymerization of two different monomers, forming a copolymer with varying monomer compositions.

"Self-healing," as used herein, refers to a class of smart materials that possess the ability to autonomously repair themselves when subjected to mechanical damage or microcracks. This self-repair process can occur without the need for external stimuli or intervention, thereby enhancing the material's durability, reliability, and longevity. Self-healing can occur by flow of viscous or viscoelastic constituents.

"Enhanced sensation" or "enhanced pleasure," as used herein, refers to increased exposure of sensory neurons and/or increased exposed penile surface area and/or increased sexual arousal and/or increased simulation of sensory neurons. The sensation or pleasure may be the users, the partners or both (collectively, the "users").

"Preventing" (used interchangeably with "prohibiting"), as used herein, refers to reducing, minimizing, or eliminating the release of semen outside the barrier layer in the present disclosure in comparison to natural release during ejaculation.

"Plasticizer," as used herein, refers to an additive that, when added to a polymer, polymer blend, copolymer, copolymer blend, polymer network or copolymer network, results in thermomechanical behavior consistent with that understood to be associated with plasticization, namely, decrease in glass transition temperature, lowering of crystalline melting temperature, triggering of stress relaxation or resulting in increased or decreased adhesion strength. Plasticizers may be added in approximately about 1 wt%, about 2 wt%, about 3 wt% etc. increasing up to about 30 wt% or about 50 wt% or more to polymer, copolymer or network mixtures or blends. Examples of plasticizers for various polymer systems are known an include water, common solvents, small molecules such as phthalates, glycerol or fatty acid compounds, triacetin, poly(ethylene glycol) compounds that are liquid at room temperature with molecular weights ranging from 1 to 30 or more repeat units, vegetable oil, detergents and other common plasticizing agents. Low molecular weight oligomers can also plasticize high molecular weight or crosslinked polymers of the same or similar chemical composition.

"Substantially less," as used herein, refers to a reduction in covered penile surface area in comparison with that covered by a traditional condom such that enhanced sensation or pleasure occurs. Substantially less may be, for example, about 10%, about 20%, about 30%, about 40%, about 50%, about 60%, about 70%, about 80% or about 90% or less surface area.

"Elastomeric behavior," as used herein, refers to generally linear elastic or combined linear elastic and plastic deformation stress/strain behavior for a material as it is strained in a regime in a region above a key transition regime such that stress/strain hysteresis is generally conserved.

"Flexible behavior," as used herein, refers to behavior of a rigid, viscoelastic or elastomeric material that can be described as compliant or deformable to fit the demands of a specific engineering application.

"Self-form," as used herein, refers to natural expansion of barrier layer in the present disclosure upon forces resulting from application of the barrier layer to the penis and/or ejaculation such that barrier layer expands but does not substantially release seminal fluid outside its boundaries.

"Reduce" or "reduction," as used herein, refers to a decrease in a particular property. The reduction may be, for example, at least about 10%, at least about 20%, at least about 30%, at least about 40%, at least about 50%, at least about 60%, at least about 70%, at least about 80%, at least about 90% or at least about 99% or more. The decrease can be measured by any suitable means, for example, by any suitable method known in the art. In the case of coverage of a condom of an erect penis, reduction may be decreased erect penis surface area of, for example, at least about 10%, at least about 20%, at least about 30%, at least about 40%, at least about 50%, at least about 60%, at least about 70%, at least about 80%, at least about 90% or at least about 99% or more. In the case of coverage of a condom that primarily covers a portion of the head of the penis but exposes other sections of the head of the penis and the shaft of the penis, reduction may be decreased erect penis head surface area of, for example, at least about 10%, at least about 20%, at least about 30%, at least about 40%, at least about 50%, at least about 60%, at least about 70%, at least about 80%, at least about 90% or at least about 99% or more. In all cases, reduction of erect penis coverage may include exposure of sections of the penis that have high sensation/sensory neuron presence, including the frenulum.

"Enhanced" as used herein refers to an increase in a particular property. The enhancement may be, for example, at least about 10%, at least about 20%, at least about 30%, at least about 40%, at least about 50%, at least about 60%, at least about 70%, at least about 80%, at least about 90% or at least about 100% or more. The increase can be measured by any suitable means, for example, by any suitable method known in the art. In one embodiment, the condom disclosed herein enhances sexual pleasure compared to other condoms known in the art, including but not limited to conventional condoms. In one embodiment, the condom disclosed herein enhances sexual pleasure compared to other condoms known in the art, including, but not limited to, conventional condoms.

"Contraception device" (used interchangeably with "prophylactic device"), as used herein, refers to a mechanical barrier that prevents transmission of fluid during sexual intercourse. A condom is a male contraception device. A diaphragm is a female contraception device.

"Membrane," as used herein, refers to a layer or film of a solid, continuous polymer material.

"Subject," as used herein, refers to the person to which the contraceptive device is, or is intended to be, applied.

"Polymer," as used herein, refers substances composed of macromolecules, very large molecules with molecular weights ranging from a few thousand to as high as millions of grams/mole made up of simpler, repeating units, derived from lower molecular weight monomers. Polymer, as used herein, refers to both homopolymers and copolymers. Homopolymers are made from (i.e., they comprise) one type of monomer. Copolymers are made from (i.e., they comprise) two or more different kinds of monomers (e.g., a styrene-butadiene copolymer). The adhesive described herein may comprise one or more polymers, including but not limited to, stimuli-responsive polymers.

"Pressure-sensitive adhesive," as used herein, refers to an adhesive that gains adhesive performance or tack through polymer flow onto the surface onto which the adhesive is pressed to adhere using force. In certain embodiments herein, the adhesive does not comprise a pressure-sensitive adhesive. The term "conventional pressure sensitive adhesive", as used in the art, refers to any pressure any pressure sensitive adhesive known in the art, and in some embodiments, refers to any commercially available pressure sensitive adhesive referenced herein or to any pressure sensitive adhesive used in the Examples section herein.

### 4.2. Contraceptive Devices

In one aspect, the present disclosure provides contraceptive devices comprising an adhesive comprising a stimuli-responsive polymer, and optionally one or more other polymers, cross linkers and/or additives, wherein when the adhesive of the contraceptive device is adhered to the genital surface of a human subject, the adhesive delaminates from the genital surface upon application of a stimulus to the contraceptive device. In some embodiments, the contraceptive device is a condom, and the genital surface is the glans of the penis. In some embodiments, the contraceptive device is a diaphragm, and the genital surface is the vagina.

In some embodiments, the contraceptive device comprises a barrier layer suitable for preventing prohibiting passage of bodily fluids during sexual intercourse and comprising an inner surface and outer surface, and an adhesive layer adhered to at least a portion of the inner surface of the barrier layer; wherein the adhesive layer comprises an adhesive that comprises a stimuli-responsive polymer formed from one or more monomers and optionally one or more polyfunctional crosslinkers, wherein when the contraceptive device is applied to the genital surface of a human subject, the adhesive layer adheres to the genital surface, and the adhesive layer can be removed from the genital surface following application of a stimulus to the contraceptive device. In some embodiments, the contraceptive device is a condom, and the genital surface is the glans of the penis. In some embodiments, the contraceptive device is a diaphragm, and the genital surface is the vagina.

In some embodiments, the human subject is male, and the contraceptive device is applied to and adheres to the penis. In some embodiments, the human subject is female, and the contraceptive device is applied to and adheres to the vagina.

In some embodiments, the contraceptive device is a fractional condom. The fractional condoms of the present disclosure cover substantially less of the penis than conventional condoms, therein leaving increased penile surface area exposed and providing a framework for increased user sensation. Like a conventional condom, the fractional condom described herein reduces the chance of pregnancy by preventing sperm from reaching the eggs. Unlike conventional condoms, it does not cover the entire penis and correspondingly, may not prevent the spread of certain STIs.

In some embodiments, the fractional condom is configured for adhesion to the glans of the penis. In some embodiments, the fraction condom is configured for adhesion only to the glans of the penis. In some embodiments, the fractional condom is sized and shaped such that, when applied to the penis, the condom does not contact or cover the corona of the penis. In some embodiments, the condom does not contact or cover the frenulum of the penis. In some embodiments, the fractional condom is sized and shaped such that, when applied to the penis, the condom does not contact the shaft of the penis.

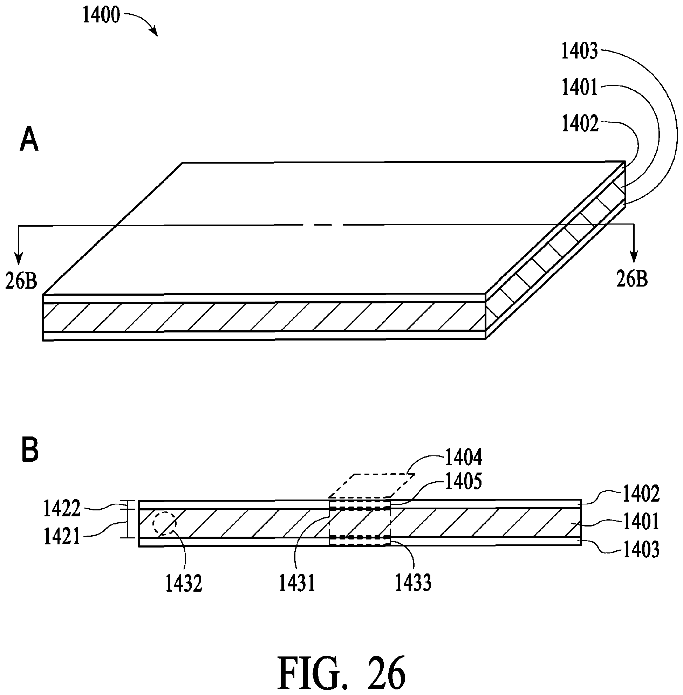

In some embodiments, the fractional condom has a planar geometry 1400. See, for example, **FIG. 26A** (which presents a perspective view of condom 1400). The device geometry is planar, such that it may be laid out upon a flat surface without significant stretching, wrinkling, folding, or creasing. The embodiment includes a planar adhesive layer 1401 coupled to a planar barrier layer 1402. A backing layer 1403 may couple to the adhesive layer such that the adhesive layer is between the backing layer and the barrier layer. As shown in **FIG. 26B** (which provides a side view of the embodiment of **FIG. 26A****),** such an embodiment may include a specific portion of the device for a semen reservoir, however any such reservoir does not extend vertically beyond the barrier layer (i.e., into area 1404). In other words, the thickness of the reservoir is not beyond the combined thickness of the adhesive and barrier layers and therefore is coplanar with the barrier layer (e.g., within area 1405), the adhesive layer, or a combination of the barrier and adhesive layers.

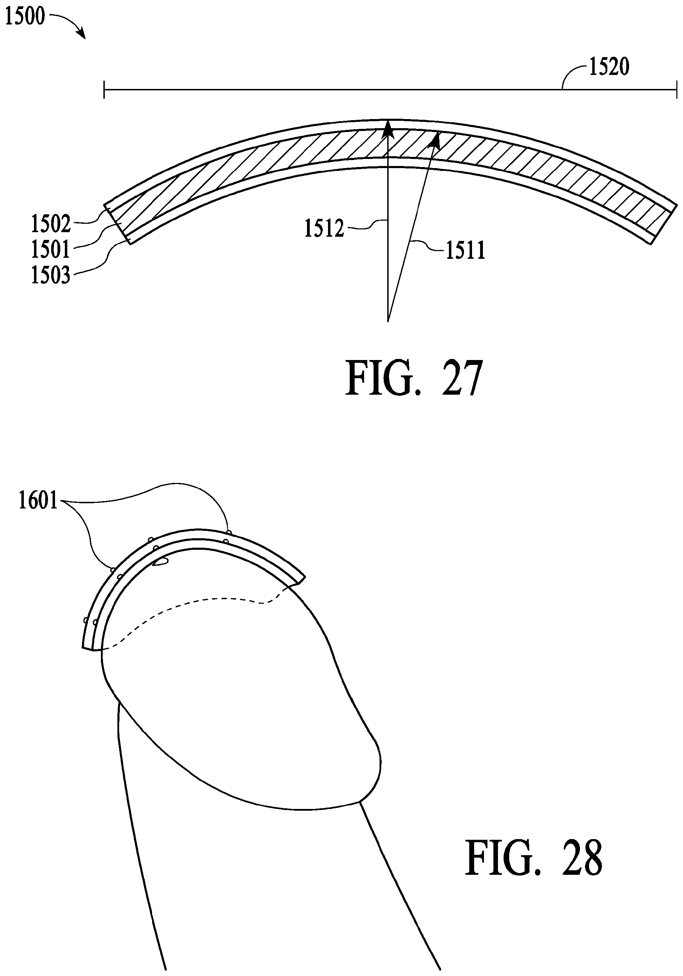

In some embodiments, the geometry of the condom's barrier layer may have one principal direction of curvature which may have a constant or varying radius of curvature, but zero curvature in any orthogonal direction. In other words, the Gaussian curvature of the adhesive layer is zero or approximately zero. In an embodiment as shown in **FIG. 27****,** a fractional condom 1500 includes an outer barrier layer 1502 coupled to an inner adhesive layer 1501 (which couples to backing layer 1503). The barrier layer includes a consistent radius of curvature 1512 for over 50% of the width 1520 of the barrier layer. The adhesive layer is coupled to the barrier layer and "nests" underneath the barrier layer, thereby adopting the curvature of the barrier layer albeit at a slightly smaller radius of curvature 1511.

In some embodiments, the semen reservoir is a self-forming semen reservoir, i.e., in response to application of the condom to the penis and/or ejaculation during use. Upon removal of the package, there is not necessarily a reservoir that protrudes outward away from outer portions of the condom's barrier layer. For example, see **FIG. 26B****.** In some embodiments, the reservoir is indistinguishable from other portions of the barrier and does not exist in a sense while residing in the condom packaging. However, a reservoir is formed in use through one or more of the following methods.

First, as the user ejaculates, the expressed fluid will press upon an internal surface of the device covering the vicinity of the urethra meatus (opening), causing a mechanical deformation of the barrier layer to accommodate the ejaculated fluid volume. The resultant reservoir, which may elastically or plastically deform the barrier and/or adhesive layers is thereby said to be self-forming. For example, by adjusting Young's modulus for the barrier layer, mechanical deformation may be adjusted to accommodate ejaculate.

In some embodiments, the condom may comprise a relatively thin barrier layer (e.g., less than 200 microns) (see thickness 1422) coupled to a relatively thick adhesive layer (e.g., greater than 300 microns) (see thickness 1421). The adhesive may not be present in area 1431 of the barrier layer (which covers the urethra meatus). The thickness of the adhesive 1421 creates a gap between the barrier layer and the glans, which is a void volume that may be filled with semen. For example, area 1431 may form a gap where no adhesive is located and the gap is formed between the urethra, adhesive layer portions, and barrier layer.

In some embodiments, adhesive may be present in area 1431 but may be perforated or thinned (as compared to outer edges of the adhesive in location 1432) to provide flexibility in the otherwise thick adhesion layer to better accommodate ejaculate. In some embodiments, adhesive may be present in area 1431 but a thin membrane 1433 may be included to shield the adhesive from the urethral opening. Embodiments where adhesive remains in area 1431 may facilitate ease of manufacturing by reducing the need for exact placement of adhesive on the barrier layer.

**FIG. 28** shows an embodiment that includes a barrier layer coupled to an adhesive layer. As the condom is applied to the glans of the penis, it will wrinkle and fold, with the user pressing the condom onto the skin of the glans, thereby sealing closed any folds or wrinkles due to the self-adhesive property of the adhesives. See, e.g., folds 1601. Such folds and wrinkles induce a void volume. The void volume may be further increased by pinching a portion of the barrier layer covering the urethra meatus.

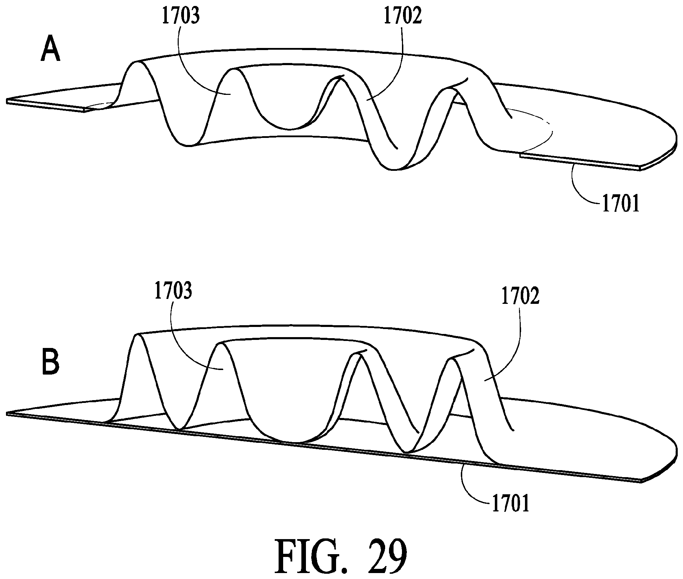

**FIG. 29A-B** show embodiments including a barrier layer 1702 coupled to an adhesive layer 1701. The reservoir 1703 is pre-formed into the barrier layer using a process compatible with the material. For example, such processes include dip coating for natural rubber latex barrier layers or vacuum/thermo-forming for thermoplastic elastomers barrier layers. The reservoir 1703 is folded using concentric folds in the horizontal plane prior to ejaculation. Upon ejaculation, the reservoir fills with fluid, causing it to both unfold and stretch to accommodate the ejaculate. The horizontal concentric folds of the reservoir both aide in manufacturability, reduce the thickness of the device for easy packaging and storage, and reduce the likelihood of the reservoir unfolding upon removal from the package, during application to the glans, or during intercourse pre-ejaculation in comparison to reservoirs with axial or vertical folding patterns.

The equilibrium pressure that must be contained by the device following an ejaculation of a specific volume can be tuned through the selection of a combination of the barrier layer (or barrier and adhesive layers) Young's modulus, Poisson's ratio, thickness, and initial void volume. The minimum limit of the equilibrium pressure is zero-gauge pressure measured across the reservoir's walls and occurs when the initial void volume is equal to or greater than the ejaculate volume. In an embodiment a relatively smaller sized preformed semen reservoir simplifies manufacturing, lowers materials cost, eases user experience, and is more aesthetically pleasing to users. When the initial void volume is less than the ejaculate volume, the device may stretch to accommodate the remainder of the ejaculate fluid. In the limit that the initial void volume is zero or approximately zero (meaning less than 0.01 mL or less than 0.1 mL or less than 0.2 mL or less than 0.5 mL or less than 1 mL), the reservoir is self-forming.

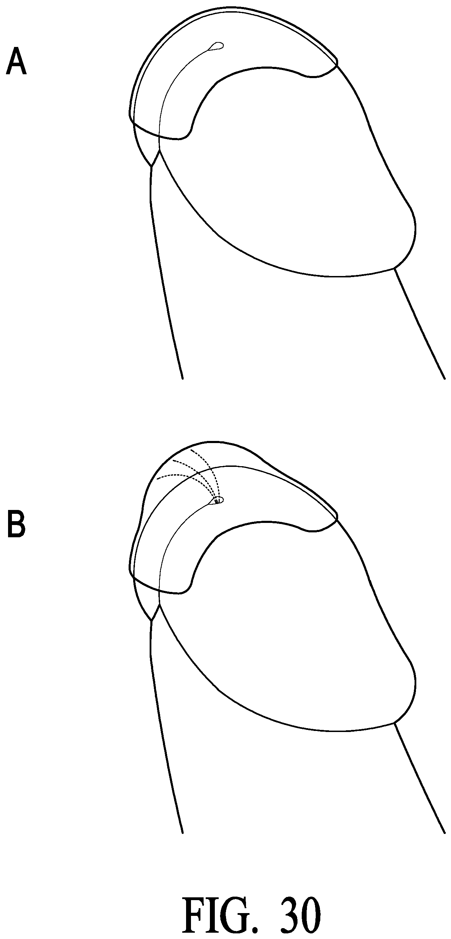

For example, **FIG. 30A** shows a planar condom attached to glans pre-ejaculation. **FIG. 30B** shows the condom attached to glans, mid-way through ejaculation (showing accumulation of ejaculate and the barrier layer stretching to accommodate the fluid).

While not being bound to any specific theory of operation, an embodiment with a "self-forming reservoir" illustrates a counterintuitive behavior of thin-walled (hyper-)elastic pressure vessel reservoirs, which may or may not exhibit plastic deformation behavior, in that there exists a value of the ratio of the reservoir volume divided by the initial (undeformed) reservoir volume such that the value of the equilibrium pressure contained by the reservoir is maximized. If the volume is decreased or increased from this amount, the internal equilibrium pressure contained by the reservoir will be reduced. In some cases, with increasing volume, the pressure reaches an approximately constant asymptotic value. As such, it is advantageous from the perspective of the fluid sealing behavior of the device to reduce the initial reservoir void volume to reduce the equilibrium pressure which will be established rapidly after ejaculation. Furthermore, it is advantageous from a physiological perspective to reduce the pressure exerted by the semen on the urethra to reduce the risk of back driving semen.

A spherical reservoir equilibrium pressure as a function of expanded ejaculate volume is now addressed. An exemplary spherical reservoir is considered under the assumptions of a thin-walled pressure vessel, a linear elastic material with Poisson's ratio of 0.5, and spherical symmetry. The relationship between pressure P and radius r of the reservoir is derived,$P \left(r\right) = \left(2E\left(r-r_0\right)t_0 r_0\right) / r 3$

In the expression above, E is the Young's modulus, t_0 is the initial thickness of the reservoir wall, r_0 is the initial radius of the reservoir. The volume of this reservoir is expressed as:$V \left(r\right) = 4 / 3 π r 3$

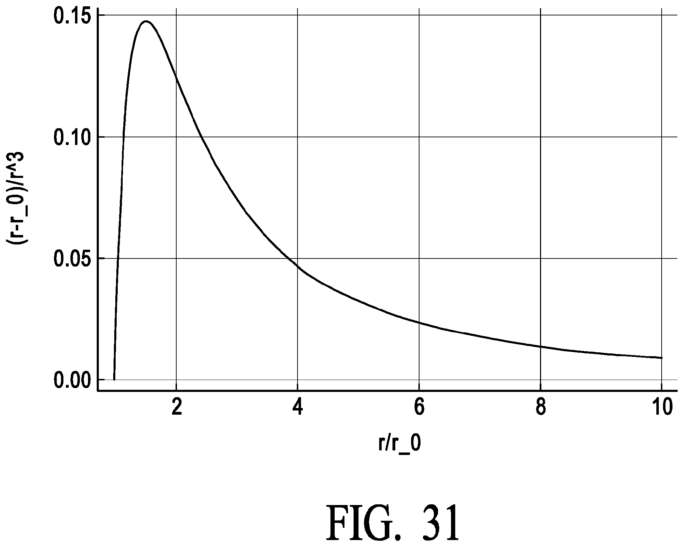

The functional shape of this curve is shown in **FIG. 31****,** and the peak at which maximum pressure occurs is:$r \left(P_maximum\right) = 3 / 2 r_ 0$

A smaller reservoir also enables the device to fit into smaller packages, enabling users to store and carry the devices more conveniently and more often. Furthermore, as an embodiment is planar without the need to unroll or unfold the device, manipulation and application is facilitated, particularly to those using the product with limited dexterity, mobility, or visual impairment (e.g., due to natural anatomical variation, injuries, or use in the dark).

In some embodiments, the condom is a fractional condom, such as the condom of **FIG. 26B****.** The condom includes a barrier layer coupled to an adhesive layer. The barrier layer may have a thickness 1422 between 25 to 200 microns and the adhesive layer may have a thickness 1421 between 25 to 750 microns. For example, the barrier layer (e.g., latex) may be between 30 and 150 microns in thickness while the adhesive layer is between 250 and 500 microns. While not being bound to any particular theory of operation, embodiments having an adhesive layer that is thicker than the barrier layer diminish or remove pain to the user during condom removal. Removal of the condom from the user is aided by the cohesive nature of a crosslinked adhesive described herein. The thick adhesive aides in a painless removal of the device from the glans through advantageous combinations of the storage and loss moduli, and material thickness, which both dissipates energy and transmits the applied force during removal to the skin in a favorable way as to mitigate experienced pain. Further, the ratio of thickness between the adhesion and barrier layers may also facilitate a pain-free removal of the device from the user.

Furthermore, a thick adhesive enables the adhesive to function as a compliant gasket which deforms and adapts to the user's skin motion during application, intercourse, ejaculation and pre-removal to provide a robust seal to entrap semen and pre-ejaculate fluids. In contrast, thinner adhesives must rely on the mechanics of the barrier layer to provide all of the compliance and deformation required to contain the fluid, particularly when considering the dynamic deformation of the glans skin and structure under the actions and loads of sexual intercourse.

In some embodiments, the contraceptive device has a shelf-life of at least 1 month, such as, for example, at least 2 months, at least 3 months, at least 4 months, at least 5 months, at least 6 months, at least 7 months, at least 8 months, at least 9 months, at least 10 months, at least 11 months, at least 12 months, or at least 24 months.

In some embodiments, the barrier performance of the contraceptive device is consistent with that demonstrated to be suitable for use in a relevant field of use.

In some embodiments, the contraceptive device is compatible with a personal lubricant. In some embodiments, the contraceptive device is compatible with an oil-based lubricant.

In some embodiments, the contraceptive device does not comprise an additional mechanical retaining means, as in U.S. Patent No. 5,421,350.

In some embodiments, the adhesive layer does not stick to itself, i.e., is not self-adhesive.

In some embodiments, the condom is a non-rigid, non-rolled, fractional condom comprising a first adhesive layer (e.g., a stimuli responsive polymer) and a second layer comprising a barrier and reservoir, wherein the condom does not contact the shaft or corona of the penis; wherein the adhesive layer is (a) coextensive with the barrier layer; (b) thicker than the barrier layer; and/or (c) the only means for securing the fractional condom to the penis. In some embodiments, the second layer is continuous, i.e., the barrier and the reservoir are continuous. In some embodiments, the reservoir is self-forming. In some embodiments, the stimulus is mechanical, e.g., shear rate or peeling. In some embodiments, the fractional condom consists of only two or three layers, the latter inclusive of a third backing layer. In some embodiments, the two or three layers comprise sublayers. In some embodiments, the condom comprises one or more additional means of mechanically securing the condom to the penis.

The first adhesive layer of the non-rigid, non-rolled, fractional condom described above may comprise any suitable stimuli-responsive polymer, e.g., any stimuli-responsive polymer described herein, and in some embodiments, a stimuli-responsive polymer comprising acrylate or methacrylate monomers (e.g., lauryl methacrylate) and optionally, a polyfunctional crosslinker (e.g., a tri-functional crosslinker such as TMPTA). The weight ratio of the polymer to the tri-functional crosslinker may be, for example, 99.1:0.9 and more particularly, 99.02: 08, 99.04:06, or 99.04:06. In some embodiments the adhesive is characterized by low-density, heterogeneous crosslinking.

The non-rigid, non-rolled, fractional condom described above may have one or more properties, as described herein including, but not limited to, a disproportionate response to a stimulus, such as a mechanical stimulus, such that a response is achievable using a lower intensity of stimulus. In some embodiments, the non-rigid, non-rolled, fractional condom described above may exhibit a lower peel strength at lower peel rates and a higher peel strength at higher peel rates, e.g., a peel strength that is about 10X, about 20X, about 30X, about 40X, about 50X, about 60X, about 70X, about 80X, about 90X, about 100X, about 2X, about 3X, about 4X, or about 5X or more less than a peel strength at a higher peel rate.

In some embodiments, the non-rigid, non-rolled, fractional condom may be removed with little or no pain, e.g., as measured using the Wong-Baker Qualitative Pain Assessment (WBQPA) on a scale of 0 (no pain) to 10 (maximum pain). In some embodiments, the pain is less than 4 on the WBQPA scale when the adhesive layer is removed by light peeling, e.g., less than 3, less than 2, less than 1 or 0. In some embodiments, the non-rigid, non-rolled, fractional condom may have a loss modulus from 0.1 MPa to 0.5 MPa.

In certain embodiments, the non-rigid, non-rolled, fractional condom described above leaves little or no residue on the skin after removal, e.g., less than about 10%, less than about 5%, less than about 1% or 0 residue.

In some embodiments, the rate of contraceptive device failure (clinical or nonclinical) when measured a subject or a group of subjects is less than about 10%, less than about 8%, less than about 6%, less than about 4%, less than about 2%, less than about 1.5%, less than about 1%, less than about 0.9%, less than about 0.8%, less than about 0.7%, less than about 0.6%, less than about 0.5%, less than about 0.4%, less than about 0.3%, less than about 0.2% or about 0.1% or less. Clinical failure refers to contraceptive devices, e.g., condoms, that break, tear, leak, or slip off completely after initial penetration and before final complete withdrawal. Nonclinical failure refers to contraceptive devices, e.g., condoms, that break, tear, leak, or partially slip.

In some embodiments, the leakage rate of the condom is less than about 6%, such as, for example, less than about 5%, less than about 4%, less than about 3%, less than about 2%, or less than about 1%.

### 4.2.1. Barrier Layer

In some embodiments, the barrier layer comprises a polymer membrane or film that exhibits elastomeric or flexible thermomechanical behavior. In some embodiments, the barrier layer is a membrane or film.

In some embodiments, the barrier layer comprises natural latex rubber, synthetic rubber, amorphous polyurethane, semi-crystalline polyurethanes including various thermoplastic polyurethanes, polyethylene, polypropylene, polydimethylsiloxane and other silicone rubbers, polyethylene terephthalate, poly(vinyl chloride), polyisoprene, vulcanized polyisoprene and other vulcanized or crosslinked rubbers, ethylene vinyl acetate, poly(vinyl acetate), elastomeric or flexible materials, or blends thereof. In some embodiments, the barrier layer comprises natural latex rubber, synthetic rubber, or polyurethanes.

In some embodiments, the barrier layer is not polyurethane.

In some embodiments, the barrier layer is not loose fitting.

In some embodiments, the barrier layer exhibits a stimuli-responsive behavior that enables selective permeability, controlled permeability, or controlled porosity. Exemplary stimuli of the stimuli-responsive barrier layer include temperature change, physico-chemical change, light, ultrasound, ionic strength change, pH change, magnetism, and mechanical force. In some embodiments, the stimulus of the stimuli-responsive barrier layer is different than the stimulus of the stimuli-responsive polymer of the adhesive layer. In some embodiments, the stimulus of the stimuli-responsive barrier layer is the same as the stimulus of the stimuli-responsive polymer of the adhesive layer.

In some embodiments, the barrier layer further comprises one or more additives, e.g., to enhance its properties. Exemplary additives include, but are not limited to, a polymer, ceramic, metal material or spherical, rod, disc, or other shaped structures. Additive materials include silicon oxide, metal oxides, iron oxides, metals, nitinol, ceramics, conducting polymers, etc. Additive materials maybe uniformly or non-uniformly dispersed or crosslinked within the material.

In some embodiments, the barrier layer has a thickness from 0.001 mm to 2 mm, such as, for example, from 0.001 mm to 1.5 mm, from 0.001 mm to 1 mm, from 0.001 mm to 0.5 mm, from 0.001 mm to 0.1 mm, from 0.001 mm to 0.01 mm. In some embodiments, the barrier layer has a thickness from 0.025 mm to 0.25 mm, such as, for example, from 0.025 mm to 0.2 mm, from 0.025 mm to 0.15 mm, from 0.025 mm to 0.1 mm, or from 0.025 mm to 0.05 mm. In some embodiments, the barrier layer has thickness of at least 0.01 mm, e.g., at least 0.05 mm, at least 0.10 mm, at least 0.15 mm, at least 0.25 mm, at least 0.3 mm, or at least 0.5 mm.

In some embodiments, the barrier layer has a thickness of less than about 200 microns, such as, for example, from about 40 to 100 microns, or about 40 microns, about 50 microns, about 60 microns, about 70 microns, about 80 microns, about 90 microns, or about 100 microns.

In some embodiments, the barrier layer has a thickness of less than about 180 microns, such as, for example, less than about 160 microns, less than about 140 microns, less than about 120 microns, less than about 100 microns, less than about 80 microns, less than about 60 microns, less than about 40 microns, or less than about 20 microns, but in each case greater than zero.

In some embodiments, the barrier layer has a planar or curved geometry selected from a square, circle, oval, hemisphere, rectangle, polygon, or curvilinear polygon. In some embodiments, the barrier layer is not tubular.

In some embodiments, the barrier layer has a first geometry before adhering to the penis and transforms to a second geometry upon application to the penis.

In some embodiments, the barrier layer has a circle geometry. In some embodiments, the radius of the circle is at least about 0.5 cm, e.g., about 1.0 cm, about 2.0 cm, about 3.0 cm, or about 5.0 cm. In some embodiments, the radius of the circle is about 0.5 cm, about 1.0 cm, about 2.0 cm, about 3.0 cm, about 5.0 cm or about 10.0 cm or greater.

In some embodiments, the barrier layer has a rectangle geometry. In some embodiments, the rectangle has a length from 0.5 cm to 5 cm and a width from 0.5 cm to 5 cm. In some embodiments, the rectangle has a length from 0.5 cm to 4 cm, from 0.5 cm to 3 cm, from 0.5 cm to 2 cm, from 0.5 cm to 1 cm, from 1 cm to 5 cm, from 1 cm to 4 cm, from 1 cm to 3 cm, from 1 cm to 2 cm, from 2 cm to 5 cm, from 2 cm to 4 cm, from 2 cm to 3 cm, from 3 cm to 5 cm, or from 4 cm to 5. In some embodiments, the rectangle has a width from 0.5 cm to 4 cm, from 0.5 cm to 3 cm, from 0.5 cm to 2 cm, from 0.5 cm to 1 cm, from 1 cm to 5 cm, from 1 cm to 4 cm, from 1 cm to 3 cm, from 1 cm to 2 cm, from 2 cm to 5 cm, from 2 cm to 4 cm, from 2 cm to 3 cm, from 3 cm to 5 cm, or from 4 cm to 5.

In some embodiments, the barrier layer has an oval geometry. In some embodiments, the oval has a primary radius of about 0.5 cm, about 1.0 cm, about 2.0 cm, about 3.0 cm, about 5.0 cm, or about 10.0 cm and a separate secondary radius of about 0.5 cm, about 1.0 cm, about 2.0 cm, about 3.0 cm, about 5.0 cm, or about 10.0 cm or greater.

In some embodiments, the barrier layer has primary dimensions of about 0.5 x 0.5 cm, about 1.0 x 1.0 cm, about 1.5 x 1.5 cm, about 2.5 x 2.5 cm, about 3.0 x 3.0 cm, or about 5.0 x 5.0 cm or any combination thereof in the case of a rectangular barrier layer.

In some embodiments, the barrier layer does not comprise one or more protrusions. Rather, according to this embodiment, the barrier layer is a conventional, simple geometric form (e.g., rectangle, oval, or circle).

In some embodiments, the barrier layer does not comprise one or more protrusions. Rather, according to this embodiment, the barrier layer is a conventional, simple geometric form (e.g., rectangle, oval, or circle). This is contrast, for example, to the protruding wings disclosed in WO2014178661A1.

In some embodiments, the barrier layer comprises a lubricant on the outer surface, e.g., the outer surface of the contraceptive device (condom or diaphragm). In some embodiments, the lubricant is selected from a water-based lubricant, silicon-based lubricant, and oil-based lubricant.

In some embodiments, the barrier layer comprises a spermicide on the outer surface of the barrier layer, i.e., the outer surface of the condom. In some embodiments, the spermicide is selected from Nonoxynol-9, octoxynol-9, benzalkonium chloride, lactic acid, menfegol, and combinations thereof.

In some embodiments, a condom further comprises a reservoir sized and shaped suitably for collecting semen ejaculated from the penis. In some embodiments, the reservoir is configured for distal to the urethral opening of the penis. In some embodiments, the reservoir is configured on the tip, along the side, or at the base, or below the base of the condom.

In some embodiments, the reservoir is continuous with the barrier layer, i.e., the reservoir and the barrier layer are part of the same structure/not separate structures and not adhered or otherwise connected by a connecting means.

In some embodiments, the reservoir self-forms when subjected to pressure from ejaculation by the penis and does not have a pre-defined geometry.

In some embodiments, the reservoir comprises a polymer coating that swells or gels when contacted with semen. In some embodiment, such swelling or gelling retains the sperm within the reservoir. Exemplary polymer coatings include, but are not limited to, chitosan, alginate, polyacrylic acid, crosslinked polyacrylic acid, sodium polyacrylate, crosslinked sodium polyacrylate, and combinations thereof.

In some embodiments, the reservoir is roughly spherical in nature and has a radius of, e.g., about 0.1 mm, about 0.25 mm, about 0.5 mm, about 0.75 mm, about 1.0 mm, about 2.0 mm, or about 5.0 mm or greater.

In some embodiments, the reservoir is roughly cylindrical in nature and has a radius of, e.g., about 0.1 mm, about 0.25 mm, about 0.5 mm, about 0.75 mm, about 1.0 mm, or about 2.0 mm or greater and a length of about 1.0 mm, about 2.0 mm, about 5.0 mm or about 10.0 mm or greater.

In some embodiments, a condom further comprises an elastomeric ring affixed to the outer portion of the inner surface of the barrier layer or the edge of the barrier layer. In some embodiments, the elastomeric ring encircles the base of the barrier layer and may be expandable by stretching to encompass and secure the barrier layer to the base of the penile head and may optionally exert contractile force that enhances adhesion of the condom to the penis and prevents stress concentrations or shear forces from removing the adhered barrier layer during mechanical perturbation such as that associated with sexual activity.

In some embodiments, the elastomeric ring has a cross-sectional diameter of at least 0.1 mm, such as, for example, at least 0.5 mm, at least 1.0 mm, at least 2.0 mm, or at least 3.0 mm or more. In some embodiments, the elastomeric ring has an overall diameter of at least 0.25 times (0.25x) the diameter of the barrier layer, such as, for example, from 0.25x to 1x, or 0.25x, 0.50x, 0.75x, or 1.0x.

In some embodiments, the elastomeric ring comprises raised rings or studs to enhance sexual sensation or pleasure.

In some embodiments, the condom further comprises one or more protruding arms connected to the elastomeric ring or the barrier layer. In some embodiments, the more protruding arms may be expandable by stretching to encompass and secure the barrier layer to the base of the penile head and may optionally exert contractile force that enhances adhesion of the condom to the penis and prevents stress concentrations or shear forces from removing the adhered barrier layer during mechanical perturbation such as that associated with sexual activity.

In some embodiments, the protruding arms may have an aspect ratio of about 1:1, about 1:2, about 1:5, about 1:10, about 1:20 or about 1:100 or greater. In some embodiments, there may be 1, 2, 3, 4, 5, 6 or more protruding arms present. In some embodiments, a protruding arm has a length of about 0.5 cm, about 1.0 cm, about 2.0 cm, about 3.0 cm, or about 5.0 cm or more, and a width of about 0.1 cm, about 0.2 cm, about 0.5 cm, about 1.0 cm or about 2.0 cm or more.

### 4.2.2. Adhesive Layer

The contraceptive devices of the present disclosure comprise an adhesive layer comprising an adhesive comprising a stimuli-responsive polymer and optionally, one or more additional polymers, crosslinkers and/or additives. In some embodiments, the adhesive has been previously applied to a surface (e.g., skin) and is stimuli-responsive in response to a stimulus encountered in the bound state. In some embodiments, the stimuli-responsive polymer is a mechanical action or force-responsive polymer.

In some embodiments, the adhesive layer is adhered to at least a portion of the barrier layer. In some embodiments, the adhesive layer is adhered to a portion of the inner surface of the barrier layer. In some embodiments, the adhesive layer is coextensive with the inner surface of the barrier layer, i.e., adhered to the entire surface of the barrier layer. In some embodiments, the adhesive area covers at least about 1% of the inner surface of the barrier layer, such as, for example, at least about 5%, at least about 10%, at least about 25%, at least about 33%, at least about 50%, at least about 66%, at least about 75%, or about 100%.

In some embodiments, the adhesive layer is coextensive with the barrier layer. In some embodiments, the adhesive layer is coextensive with the barrier layer but for a limited area associated with self-formation of a reservoir. In some embodiments, the adhesive layer is positioned in the front of the barrier layer. In some embodiments, the barrier layer and the reservoir are formed continuously, and (a) the adhesive layer is continuous with the barrier layer or (b) the adhesive layer is located at the front of the barrier layer.

In some embodiments, the adhesive layer extends to the elastomeric ring and/or protruding arm(s) of the condom. In some embodiments, the adhesive layer is the sole means for securing the condom to the penis.

In some embodiments, the adhesive layer comprises a cross-section that is uniform, non-uniform, circular, spherical, elliptical or ellipsoidal.

In some embodiments, the adhesive layer comprises one or more patterned configurations. Patterning may facilitate adhesive crack propagation or shear-responsive delamination. For example, adhesive patterning may enable sufficient adhesion of the barrier layer to the penis or vagina while also enabling the barrier layer to be peeled with minimal or no pain. Such patterning includes, but is not limited to, a continuous ring or repeating dot ring layer at the base of the barrier layer ranging from 0.1 to 3000 micrometers in length, more particularly 1 to 2000 micrometers in length, more particularly 20 to 2000 micrometers in length, and ranging from 0.1 to 3000 micrometers thick, more particularly 1 to 2000 micrometers thick, more particularly 5 to 1000 micrometers thick, more particularly 10 to 600 micrometers thick, with dot patterns covering between 10 and 100% of available adhesive area in base ring area, more particularly 20 and 100% of available adhesive area in base ring area, more particularly 30 and 100% of available area in base ring area.

In certain embodiments, the length of the patterning is from about 1 to 100 micrometers, from about 100 to about 200 micrometers, from about 200 to about 300 micrometers, from about 300 to about 400 micrometers, from about 400 to about 500 micrometers, from about 500 to about 600 micrometers, from about 600 to about 700 micrometers, from about 700 to about 800 micrometers, from about 800 to about 900 micrometers, from about 900 to about 1000 micrometers in length.

In certain embodiments, the length of the patterning is about between about 1000 and 1100 micrometers, about 1100 and about 1200 micrometers, about 1200 and about 1300 micrometers, about 1300 and about 1400 micrometers, about 1400 and about 1500 micrometers, about 1500 and about 1600 micrometers, about 1600 and about 1700 micrometers, about 1700 and about 1800 micrometers, about 1800 and about 1900 micrometers or about 1900 and about 200 micrometers in length.

In certain embodiments, the thickness of the patterning is between about 0.1 and about 500 micrometers, about 1 and about 1 and 100 micrometers, about 100 and about 200 micrometers, about 200 and about 300 micrometers, about 300 and about 400 micrometers, about 400 and about 500 micrometers, about 500 and about 600 micrometers, about 600 and about 700 micrometers, about 700 and about 800 micrometers, about 800 and about 900 micrometers, about 900 and about 1000 micrometers in thickness.

In certain embodiments, the thickness of the patterning is about between about 1000 and 1100 micrometers, about 1100 and about 1200 micrometers, about 1200 and about 1300 micrometers, about 1300 and about 1400 micrometers, about 1400 and about 1500 micrometers, about 1500 and about 1600 micrometers, about 1600 and about 1700 micrometers, about 1700 and about 1800 micrometers, about 1800 and about 1900 micrometers or about 1900 and about 200 micrometers in thickness.

Exemplary pattern configurations include rings, stripes, dots, and combinations thereof. Patterning may be uniform, non-uniform, or random in shape or size or location, or any combination thereof. In some embodiments, the one or more patterned configuration may cover about 1%, about 5%, about 10%, about 25%, about 33%, about 50%, about 66%, about 75% or about 100% of the adhesive layer.

In some embodiments, the patterned configuration comprises one or more rings or ring-like structures. The thickness of the rings can be, for example, about 0.1 mm, about 0.25 mm, about 0.5 mm, or about 1.0 mm or more. In some embodiments, the ring pattern covers at least about 1%, about 5%, about 10%, about 25%, about 33%, about 50%, about 66%, about 75%, or about 100% of the adhesive layer.

In some embodiments, the patterned configuration comprises dots (e.g., square, rectangular, or circular shaped dots). In some embodiments, the dot pattern covers at least about 1%, about 5%, about 10%, about 25%, about 33%, about 50%, about 66%, about 75%, or about 100% of the adhesive layer.

In some embodiments, the pattern configuration comprises stripes. In some embodiments, the strips can be of varying thickness, e.g., about 0.1 mm, about 0.25 mm, about 0.5 mm, or about 1.0 mm or more. In some embodiments, the stripes cover about 1%, about 5%, about 10%, about 25%, about 33%, about 50%, about 66%, about 75% or about 100% of the overall adhesive area within the barrier layer.

In some embodiments, the adhesive layer has a thickness in the nanometer scale or micron scale. In some embodiments, the adhesive layer has a thickness from 0.1microns to 3,000 microns, such as, for example, from 0.1 microns to 2,000 microns, from 0.1 microns to 1,000 microns, from 0.1 microns to 750 microns, from 0.1 microns to 500 microns, from 0.1 microns to 250 microns, from 1 microns to 2,000 microns, from 1 microns to 1,000 microns, from 1 microns to 750 microns, 1 microns to 500 microns, from 1 microns to 250 microns, from 25 microns to 2,000 microns, from 25 microns to 1,000 microns, from 25 microns to 750 microns, from 25 microns to 500 microns, from 25 microns to 250 microns, from 100 microns to 2,000 microns, from 100 microns to 1,000 microns, from 100 microns to 750 microns, from 100 microns to 500 microns, or from 100 microns to 250 microns.

In some embodiments, the adhesive layer has a thickness from about 0.01 microns to about 0.1 microns, from about 1.0 microns to about 5.0 microns, from about 10.0 microns to about 20.0 microns, from about 30.0 microns to about 50.0 microns, from about 100.0 microns to about 200 microns, from about 300 microns to about 400 microns, from about 600 microns to about 750 microns, or from about 1000 microns to about 2000 microns.

In some embodiments, the adhesive layer is from 25 to 750 microns thick, such as, for example, from about 200 to about 500 microns, or about 400 microns thick.

In some embodiment, the adhesive layer has a thickness of about 25 microns, about 50 microns, about 100 microns, about 150 microns, about 200 microns, about 250 microns, about 300 microns, about 350 microns, about 400 microns, about 450 microns, about 500 microns, about 550 microns, about 600 microns, about 650 microns, about 700 microns, or about 750 microns.

In some embodiments, the adhesive layer has a thickness from about 100 microns to about 600 microns, such as, for example, from about 200 microns to about 500 microns, or from about 300 to about 500 microns.

In some embodiments, the adhesive layer is thicker than the barrier layer. The adhesive layer may be, for example, about 1.5X, about 2X, about 2.5X, about 3X, about 3.5 X, about 4X, about 4.5X, about 5X, about 5.5X, about 6X, about 6.5X, about 7X, about 7.5X, about 8X, 8.5 X, about 9X, about 9.5X, or at least about 10X thicker than the barrier layer.

In some embodiments, the barrier layer is from about 40 to about 100 microns thick and the adhesive layer is from about 25 to about 750 microns thick. In some embodiments, the barrier layer is about 200 microns thick and the adhesive layer is about 400 microns thick.

In some embodiments, the adhesive layer is transparent.

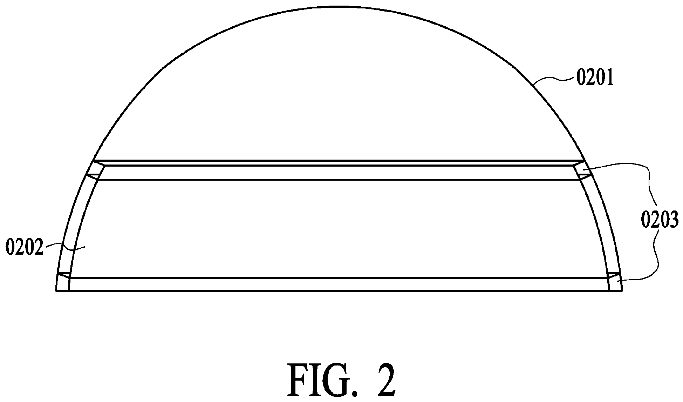

In some embodiments, the adhesive layer comprises multiple adhesives, blended together or applied separately to a barrier layer. For example, adhesive layer may include a hydrophobic, water-insoluble layer at its outer edges along the circumference of the barrier layer approximately 0.1 to 2 cm in length, more particularly 0.2 to 1 cm in length, more particularly 0.25 to 1 cm in length, and a hydrophilic, water-soluble layer above the hydrophobic adhesive layer as illustrated in **FIG. 2****.** In some embodiments, the hydrophobic outer adhesive layer may be stimuli-responsive.

In some embodiments, the adhesive comprises (1) a primary side chain optionally crystallizable side chain adhesive polymer, (2) optional additives, (3) optional additional polymers, and optionally, amorphous polymer blended phases or heterophases, and (4) optionally crosslinkers, which optionally are incorporated into polymer network uniformly and optionally are aggregated and serve as high stress concentration network sites to facilitate adhesive failure or desirable tack profile when desirable. In some embodiments, the adhesive comprises a blend or copolymer of amorphous polymers that may crystallize.

In some embodiments, the adhesive layer of the contraceptive devices described herein comprises an adhesive that comprises a stimuli-responsive polymer formed from one or more monomers and optionally one or more polyfunctional crosslinkers. In some embodiments, the adhesive consists of the stimuli-responsive polymer. In some embodiments, the adhesive consists essentially of the stimuli-responsive polymer. "Consists essentially of," as used herein with respect to the adhesive, refers to additional components that do not materially affect the basic characteristics of the adhesive, e.g., additives that do not have adhesive properties themselves.

In some embodiments, the adhesive does not include additional components, e.g., additives, adhesive polymers, and/or other polymers.

In some embodiments, the adhesive layer comprises a single layer. In some embodiments, the adhesive layer comprises multiple layers, e.g., 2, 3, 4, or 5 or more layers. The layers may be the same or different.

In some embodiments, the adhesive layer comprises two or three or more adhesive regions which may exhibit different mechanical, chemical and biological properties. These may include different solubility parameters in various solvents including water or vaginal fluids or ejaculate fluids. Reversible or irreversible adhesive behavior can be triggered by exposure to temperature changes, physico-chemical changes, light, ultrasound, ionic strength change, pH change, magnetic, electrical or mechanical forces as well as other stimuli or any combination thereof, either simultaneously or sequentially.

### 4.2.2.1 Adhesive Properties

In some embodiments, when the condom is applied to the penis, the adhesive layer adheres to the penis. In some embodiments, when the condom is applied to the glans of the penis, the adhesive layer adheres to the glans of the penis. In some embodiments, when the diaphragm is applied to the vagina, the adhesive layer adheres to the vagina.

Application of a stimulus to the contraceptive device causes the adhesive layer to become less adhesive or delaminate from the skin (e.g., the penis or vagina), thereby enabling selective delamination (i.e., on-demand delamination). Reversible or irreversible adhesive behavior can be triggered by exposure to temperature changes (e.g., a decrease in temperature), physico-chemical changes (e.g., dissolution), light, ultrasound, ionic strength change, pH change, magnetic, electrical, or mechanical actions or forces, as well as other stimuli or any combination thereof.

Properties for the stimuli-responsive polymer recited below may also apply to the adhesive, which can optionally contain additional components, e.g., additives and/or polymers (e.g., adhesive polymers or non-adhesive polymers).

In some embodiments, the stimuli-responsive polymer becomes less adhesive or delaminates from the vagina or penis (e.g., the glans of the penis) in 0.1 s to 60 s in response to the stimulus of the contraceptive device, such as, for example, from 1 s to 60 s, from 1 s to 30 s, from 1 s to 15 s, from 1 s to 10 s, from 1 s to 5 s, from 2 s to 30 s, or from 1 s to 15 s.

In some embodiments, the adhesive becomes less adhesive or delaminates from the vagina or penis (e.g., the glans of the penis) in 0.1 s to 60 s in response to the stimulus of the contraceptive device, such as, for example, from 1 s to 60 s, from 1 s to 30 s, from 1 s to 15 s, from 1 s to 10 s, from 1 s to 5 s, from 2 s to 30 s, or from 1 s to 15 s.

In some embodiments, the stimuli-responsive polymer delaminates from the vagina or penis faster than a non-stimuli-responsive polymer, e.g., a conventional pressure-sensitive adhesive polymer. In some embodiments, the stimuli-responsive polymer delaminates at least 2X faster than a non-stimuli-responsive polymer, e.g., a conventional pressure-sensitive adhesive polymer, such as, for example, at least 3X faster, at least 4X faster, at least 5X faster, at least 6X faster, at least 7X faster, at least 8X faster, at least 9X faster, or at least 10X faster.

In some embodiments, an adhesive comprising a stimuli-responsive polymer delaminates from the vagina or penis faster than an adhesive comprising a non-stimuli-responsive polymer, e.g., a conventional pressure-sensitive adhesive polymer. In some embodiments, an adhesive comprising a stimuli-responsive polymer delaminates at least 2X faster than an adhesive comprising a non-stimuli-responsive polymer, e.g., a conventional pressure-sensitive adhesive polymer, such as, for example, at least 3X faster, at least 4X faster, at least 5X faster, at least 6X faster, at least 7X faster, at least 8X faster, at least 9X faster, or at least 10X faster.

In some embodiments, the stimulus is selected from a temperature change, a physico-chemical change, light, ultrasound, an ionic strength change, a pH change, magnetism, a mechanical force, or mechanical action.

In some embodiments, the stimulus is a mechanical action, e.g., shear rate. In some embodiments, the shear rate is induced by peeling or pulling on the contraceptive device at different rates or frequencies.

In some embodiments, the adhesive is shear-rate responsive. In some embodiments, the stimuli-responsive polymer is shear-rate responsive. In such embodiments, the adhesive remains adhered upon application of higher shear rates and becomes less adhesive at lower shear rates. Exemplary higher shear rates include hard peeling or hard pulling. Exemplary lower shear rates include light peeling or light pulling. Higher and lower are taken with respect to a threshold about which a change in behavior is observed. In this manner, the adhesive layer and the contraceptive device can be easily removed from the penis or vagina following application of a stimulus to the contraceptive device.

In some embodiments, light peeling corresponds to a peel rate of 500 mm/min or less, such as, for example, 400 mm/min or less, 300 mm/min or less, 200 mm/min or less, 100 mm/min or less, or 50 mm/min or less. In some embodiments, light peeling corresponds to a peel rate of 50 mm/min to 500 mm/min, such as, for example, from 50 mm/min to 400 mm/min, from 50 mm/min to 300 mm/min, from 50 mm/min to 200 mm/min, from 50 mm/min to 100 mm/min, from 100mm/min to 500 mm/min, from 100 mm/min to 400 mm/min, from 100 mm/min to 300 mm/min, from 100 mm/min to 200 mm/min, from 200 mm/min to 500 mm/min, from 200 mm/min to 400 mm/min, from 200 mm/min to 300 mm/min, from 300 mm/min to 500 mm/min, from 300 mm/min to 400 mm/min, or from 400 mm/min to 500 mm/min.

In some embodiments, light peeling corresponds to a peel rate of 25 mm/s or less, such as, for example, 10 mm/s or less, 5 mm/s or less, 1 mm/s or less, 0.5 mm/s or less, 0.3 mm/s or less, or 0.1 mm/s or less. In some embodiments, light peeling corresponds to a peel rate from 0.01 mm/s to 25 mm/s, such as, for example, from 0.01 mm/s to 10 mm/s, from 0.01 mm/s to 5 mm/s, from 0.01 mm/s to 1 mm/s, from 0.01 mms/ to 0.5 mm/s, from 0.01 mm/s to 0.3 mm/s, from 0.01 mm/s to 0.1 mm/sec, from 0.1 mm/s to 25 mm/s, from 0.1 mm/s to 10 mm/s, from 0.1 mm/s to 5 mm/s, from 0.1 mm/s to 1 mm/s, from 0.1 mms/ to 0.5 mm/s, from 0.1 mm/s to 0.3 mm/s, from 1 mm/s to 25 mm/s, from 1 mm/s to 10 mm/s, or from 1 mm/s to 5 mm/s.

In some embodiments, the stimulus is a mechanical force. In some embodiments, the adhesive is force responsive. In some embodiments, the stimuli-responsive polymer is force responsive. In such embodiments, the adhesive remains adhered upon application of higher forces and becomes less adhesive upon application of lower forces. Higher and lower are taken with respect to a threshold about which a change in behavior is observed.

In some embodiments, the applied force that results in delamination of the stimuli-responsive polymer is, for example, from 0.01 to 0.1 N, from 0.1 to 1 N, from 1 to 10 N, from 10 to 100 N, or from 100 and 1000 N. In some embodiments, the applied force that results in delamination of the stimuli-responsive polymer is, for example, from 1 to 10 Pa, from 10 to 100 Pa, from 0.1 to 1 kPa, from 1 to 10 kPa, from 10 to 100 kPa, or from 0.1 to 1 MPa.

In some embodiments, the applied force that results in delamination of the adhesive is, for example, from 0.01 to 0.1 N, from 0.1 to 1 N, from 1 to 10 N, from 10 to 100 N, or from 100 and 1000 N. In some embodiments, the applied force that results in delamination of the adhesive is, for example, from 1 to 10 Pa, from 10 to 100 Pa, from 0.1 to 1 kPa, from 1 to 10 kPa, from 10 to 100 kPa, or from 0.1 to 1 MPa.

In some embodiments, the stimuli-responsive polymer has a lower peel strength at lower peel rates and a higher peel strength at higher peel rates. In some embodiments, the stimuli-responsive polymer has at least a 5% lower peel strength at a lower peel rate compared to the peel strength at a higher peel rate, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the stimuli-responsive polymer has a lower peel strength at a peel rate of 100 mm/min than at 200 mm/min. In some embodiments, the stimuli-responsive polymer has a lower peel strength at a peel rate of 100 mm/min than at 300 mm/min. In some embodiments, the stimuli-responsive polymer has a lower peel strength at a peel rate of 100 mm/min than at 400 mm/min. In some embodiments, the stimuli-responsive polymer has a lower peel strength at a peel rate of 100 mm/min than at 500 mm/min.

In some embodiments, the stimuli-responsive polymer has a lower peel strength at a peel rate of 1 mm/s than at 3 mm/s. In some embodiments, the stimuli-responsive polymer has a lower peel strength at a peel rate of 1 mm/s than at 5 mm/s. In some embodiments, the stimuli-responsive polymer has a lower peel strength at a peel rate of 1 mm/s than at 7 mm/s. In some embodiments, the stimuli-responsive polymer has a lower peel strength at a peel rate of 1 mm/s than at 10 mm/s.

In some embodiments, the stimuli-responsive polymer has at least a 5% lower peel strength at a peel rate of 100 mm/min than at 200 mm/min, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the stimuli-responsive polymer has at least a 5% lower peel strength at a peel rate of 100 mm/min than at 300 mm/min, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the stimuli-responsive polymer has at least a 5% lower peel strength at a peel rate of 100 mm/min than at 400 mm/min, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the stimuli-responsive polymer has at least a 5% lower peel strength at a peel rate of 100 mm/min than at 500 mm/min, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the stimuli-responsive polymer has at least a 5% lower peel strength at a peel rate of 1 mm/s than at 3 mm/s, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the stimuli-responsive polymer has at least a 5% lower peel strength at a peel rate of 1 mm/s than at 5 mm/s, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the stimuli-responsive polymer has at least a 5% lower peel strength at a peel rate of 1 mm/s than at 7 mm/s, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the adhesive has a lower peel strength at lower peel rates and a higher peel strength at higher peel rates. In some embodiments, the adhesive has at least a 5% lower peel strength at a lower peel rate compared to the peel strength at a higher peel rate, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the adhesive has a lower peel strength at a peel rate of 100 mm/min than at 200 mm/min. In some embodiments, the adhesive has a lower peel strength at a peel rate of 100 mm/min than at 300 mm/min. In some embodiments, the adhesive has a lower peel strength at a peel rate of 100 mm/min than at 400 mm/min. In some embodiments, the adhesive has a lower peel strength at a peel rate of 100 mm/min than at 500 mm/min.

In some embodiments, the adhesive has a lower peel strength at a peel rate of 1 mm/s than at 3 mm/s. In some embodiments, the adhesive has a lower peel strength at a peel rate of 1 mm/s than at 5 mm/s. In some embodiments, the adhesive has a lower peel strength at a peel rate of 1 mm/s than at 7 mm/s. In some embodiments, the adhesive has a lower peel strength at a peel rate of 1 mm/s than at 10 mm/s.

In some embodiments, the adhesive has at least a 5% lower peel strength at a peel rate of 100 mm/min than at 200 mm/min, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the adhesive has at least a 5% lower peel strength at a peel rate of 100 mm/min than at 300 mm/min, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the adhesive has at least a 5% lower peel strength at a peel rate of 100 mm/min than at 400 mm/min, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the adhesive has at least a 5% lower peel strength at a peel rate of 100 mm/min than at 500 mm/min, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the adhesive has at least a 5% lower peel strength at a peel rate of 1 mm/s than at 3 mm/s, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the adhesive has at least a 5% lower peel strength at a peel rate of 1 mm/s than at 5 mm/s, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the adhesive has at least a 5% lower peel strength at a peel rate of 1 mm/s than at 7 mm/s, e.g., at least 10% lower, at least 20% lower, at least 30% lower, at least 40% lower, at least 50% lower, at least 60% lower, at least 70% lower, at least 80% lower, at least 90% lower, or at least 100% lower.

In some embodiments, the stimuli-responsive polymer has a peel strength from 1 to 400 N/m at a peel rate of 100 mm/sec, such as, for example, from 1 to 300 N/m, from 1 to 200 N/m, from 1 to 100 N/m, or from 1 to 50 N/m. Peel strength can be determined by a 180° peel test using a human skin substrate analog as described hereinbelow.

In some embodiments, the adhesive has a peel strength from 1 to 400 N/m at a peel rate of 100 mm/sec, such as, for example, from 1 to 300 N/m, from 1 to 200 N/m, from 1 to 100 N/m, or from 1 to 50 N/m. Peel strength can be determined by a 180° peel test using a human skin substrate analog as described hereinbelow.

In some embodiments, the stimuli-responsive polymer has a tack strength at 25 °C of at least 1 N, such as, for example, from 1 N to 5 N, from 1 N to 4 N, from 1 N to 3 N, or from 1 N to 2 N. Tack strength can be measured as described hereinbelow, e.g., compressing an aluminum-tipped rheometer containing the polymer to an aluminum base plate for 60 s, withdrawing the rheometer at 100 micrometers per second, and measuring the axial force in N.

In some embodiments, the adhesive has a tack strength at 25 °C of at least 1 N, such as, for example, from 1 N to 5 N, from 1 N to 4 N, from 1 N to 3 N, or from 1 N to 2 N.

In some embodiments, the stimuli-responsive polymer has an adhesive strength at 25 °C of a least 20 N*s, such as, for example, at least 30 N*s, at least 40 N*s, at least 50 N*s, at least 60 N*s, at least 70 N*s, at least 80 N*s, at least 90 N*s, at least 100 N*s, at least 150 N*s, at least 200 N*s, at least 300 N*s, at least 400 N*s, or at least 500 N*s. Adhesive strength is calculated by integrating the area under the axial force vs time measurements for a sample.

In some embodiments, the adhesive has an adhesive strength at 25 °C of a least 20 N*s, such as, for example, at least 30 N*s, at least 40 N*s, at least 50 N*s, at least 60 N*s, at least 70 N*s, at least 80 N*s, at least 90 N*s, at least 100 N*s, at least 150 N*s, at least 200 N*s, at least 300 N*s, at least 400 N*s, or at least 500 N*s.

In some embodiments, the stimuli-responsive polymer has a storage modulus of from 0.01 MPa to 1 MPa, such as, for example, from 0.1 MPa to 1 MPa, from 0.1 MPa to 0.8 MPa or from 0.1 MPa to 0.5 MPa.

In some embodiments, the adhesive has a storage modulus of from 0.01 to 1 MPa, such as, for example, from 0.1 MPa to 1 MPa, from 0.1 MPa to 0.8 MPa or from 0.1 MPa to 0.5 MPa.

In some embodiments, the stimuli-responsive polymer has a loss modulus of from 0.1 MPa to 1 MPa, such as, for example, from 0.1 MPa to 0.8 MPa or from 0.1 MPa to 0.5 MPa.

In some embodiments, the adhesive has a loss modulus of from 0.1 MPa to 1 MPa, such as, for example, from 0.1 MPa to 0.8 MPa or from 0.1 MPa to 0.5 MPa.

In some embodiments, the stimuli-responsive polymer has a tan(δ) (ratio of storage modulus (G") to loss modulus (G')) at 25 °C and 1 Hz (2*pi radian/s) of at least 0.1, such as, for example, at least 0.2, at least 0.3, at least 0.4 at least 0.5, at least 0.6, at least 0.7, at least 0.8, at least 0.9, at least 1.0, at least 1.1, at least 1.2, at least 1.3, at least 1.4, at least 1.5, at least 1.6, at least 1.7, at least 1.8, at least 1.9, at least 2.0, at least 3.0, at least 4.0, or at least 5.0. In some embodiments, the stimuli-responsive polymer has a tan(δ) from 0.1 to 5, such as, for example, from 0.1 to 4, 0.1 to 3, 0.1 to 2, 0.1 to 1, 0.5 to 5, 0.5 to 4, 0.5 to 3, 0.5 to 3, 0.5 to 2, 0.5 to 1, 1 to 5, 1 to 4, 1 to 3, 1 to 2, 2 to 5, 2 to 4, 2 to 3, 3 to 5, 3 to 4, or 4 to 5. In certain embodiments, the stimuli-responsive polymer has a tan(δ) of 0.2 to 2, e.g., 0.1 to 1.5, 0.1 to 1, 0.1 to 0.5, 0.3 to 1, or 0.5 to 1.

In some embodiments, the adhesive has a tan(δ) (ratio of storage modulus (G") to loss modulus (G')) at 25 °C and 1 Hz (2* pi radian/s) of at least 0.1, such as, for example, at least 0.2, at least 0.3, at least 0.4 at least 0.5, at least 0.6, at least 0.7, at least 0.8, at least 0.9, at least 1.0, at least 1.1, at least 1.2, at least 1.3, at least 1.4, at least 1.5, at least 1.6, at least 1.7, at least 1.8, at least 1.9, at least 2.0, at least 3.0, at least 4.0, or at least 5.0. In some embodiments, the adhesive has a tan(δ) from 0.1 to 5, such as, for example, from 0.1 to 4, 0.1 to 3, 0.1 to 2, 0.1 to 1, 0.5 to 5, 0.5 to 4, 0.5 to 3, 0.5 to 3, 0.5 to 2, 0.5 to 1, 1 to 5, 1 to 4, 1 to 3, 1 to 2, 2 to 5, 2 to 4, 2 to 3, 3 to 5, 3 to 4, or 4 to 5. In certain embodiments, the adhesive has a tan(δ) of 0.2 to 2, e.g., 0.1 to 1.5, 0.1 to 1, 0.1 to 0.5, 0.3 to 1, or 0.5 to 1.

In some embodiments, the stimuli-responsive polymer has less tack when wet compared to when dry. In some embodiments, the adhesive has less tack when wet compared to when dry.

In some embodiments, the stimuli-responsive polymer is a crosslinked polymer is a low-density crosslinked polymer or a polymer with limited, heterogeneous crosslinking.

In some embodiments, the stimuli-responsive polymer has gel fraction consistent with that of a lightly crosslinked polymer network when analyzed by sol-gel analysis. In some embodiments, the stimuli-responsive polymer has a gel fraction from 0.0001 to 0.99, such as, for example, from 0.001 to 0.98, from 0.01 to 0.95, from 0.015 to 0.95, from 0.02 to 0.96, from 0.05 to 0.95, from 0.1 to 0.95, from 0.175 to 0.95, from 0.2 to 0.95, from 0.25 to 0.95, from 0.3 to 0.95, from 0.35 to 0.95, from 0.4 to 0.95, from 0.5 to 0.95, from 0.6 to 0.95, from 0.7 to 0.95, from 0.8 to 0.95, from 0.5 to 0.95, from 0.6 to 0.93, from 0.6 to 0.65 to 0.92, from 0.7 to 0.9, or from 0.7 to 0.89.

In some embodiments, the adhesive has gel fraction consistent with that of a lightly crosslinked polymer network when analyzed by sol-gel analysis. In some embodiments, the adhesive has a gel fraction from 0.0001 to 0.99, such as, for example, from 0.001 to 0.98, from 0.01 to 0.95, from 0.015 to 0.95, from 0.02 to 0.96, from 0.05 to 0.95, from 0.1 to 0.95, from 0.175 to 0.95, from 0.2 to 0.95, from 0.25 to 0.95, from 0.3 to 0.95, from 0.35 to 0.95, from 0.4 to 0.95, from 0.5 to 0.95, from 0.6 to 0.95, from 0.7 to 0.95, from 0.8 to 0.95, from 0.5 to 0.95, from 0.6 to 0.93, from 0.6 to 0.65 to 0.92, from 0.7 to 0.9, or from 0.7 to 0.89.

In some embodiments, following adhesion of the adhesive layer to the penis or vagina, removal of the contraceptive device by light peeling does not cause pain in the subject. In some embodiments, following adhesion of the adhesive layer to the glans of the penis, removal of the condom by light peeling causes minimal or no pain to the subject. Any suitable method can be utilized to determine the existence or absence of pain. There are several validated instruments for the measurement of pain. The instruments can be unidimensional, measuring only the intensity of pain, such as pain Wong-Baker Qualitative Pain Assessment (WBQPA), Numeric Pain Rating Scale (NRPS), visual analogue scale (VAS) and verbal rating scale. Multidimensional instruments measure the intensity, character and impact of pain, for example, the McGill Pain Questionnaire (MPQ) and Brief Pain Inventory (BPI).

In some embodiments, pain is measured using the WBQPA on a scale of 0 (no pain) to 10 (maximum pain). In some embodiments, the pain is less than 4 on the WBQPA scale when the adhesive layer is removed by light peeling, e.g., less than 3, less than 2, less than 1 or 0. In some embodiments, a WBQPA score of 0 is reported upon removal of the contraceptive device by light peeling. As discussed above, in some embodiments, light peeling corresponds to a user peel rate of the contraceptive device (e.g., condom) of 25 mm/s or less, such as, for example, 10 mm/s or less, 5 mm/s or less, 1 mm/s or less, 0.5 mm/s or less, 0.3 mm/s or less, or 0.1 mm/s or less. In some embodiments, light peeling corresponds to a peel rate from 0.01 mm/s to 25 mm/s, such as, for example, from 0.01 mm/s to 10 mm/s, from 0.01 mm/s to 5 mm/s, from 0.01 mm/s to 1 mm/s, from 0.01 mms/ to 0.5 mm/s, from 0.01 mm/s to 0.3 mm/s, from 0.01 mm/s to 0.1 mm/sec, from 0.1 mm/s to 25 mm/s, from 0.1 mm/s to 10 mm/s, from 0.1 mm/s to 5 mm/s, from 0.1 mm/s to 1 mm/s, from 0.1 mms/ to 0.5 mm/s, from 0.1 mm/s to 0.3 mm/s, from 1 mm/s to 25 mm/s, from 1 mm/s to 10 mm/s, or from 1 mm/s to 5 mm/s.

In some embodiments, light peeling corresponds to a user peel rate of the contraceptive device (e.g., condom) of 500 mm/min or less, such as, for example, 400 mm/min or less, 300 mm/min or less, 200 mm/min or less, 100 mm/min or less, or 50 mm/min or less, or 25 mm/min or less, or 10 mm/min or less, or 5 mm/min or less, or 1 mm/min or less, or 0.5 mm/min or less, or 0.3 mm/min or less, or 0.1 mm/min or less. In some embodiments, light peeling corresponds to a peel rate of 50 mm/min to 500 mm/min, such as, for example, from 50 mm/min to 400 mm/min, from 50 mm/min to 300 mm/min, from 50 mm/min to 200 mm/min, from 50 mm/min to 100 mm/min, from 100 mm/min to 500 mm/min, from 100 mm/min to 400 mm/min, from 100 mm/min to 300 mm/min, from 100 mm/min to 200 mm/min, from 200 mm/min to 500 mm/min, from 200 mm/min to 400 mm/min, from 200 mm/min to 300 mm/min, from 300 mm/min to 500 mm/min, from 300 mm/min to 400 mm/min, or from 400 mm/min to 500 mm/min.

In some embodiments, following adhesion of the adhesive layer to the penis or vagina, removal of the contraceptive device by light peeling causes minimal or no pain in the subject when removed in less than 15 seconds, e.g., less than 10 seconds, less than 8 seconds or less than 5 seconds.

In some embodiments, the site of adhesion exhibits limited or no irritation upon removal of the contraceptive device. Various methods are known in research settings for quantitatively assessing skin damage. Certain of these models are based on measurement of baseline skin properties that change when skin is stressed. Measurable parameters such as skin hydration, transepidermal water low (TEWL), and irritation, among others, will provide information differentiating damaged skin from healthy skin. See, e.g., Bernatchez, S. et al, ADVANCES IN WOUND CARE, Vol. 2, No. 4 (2022), which is incorporated herein by reference. Reconstructed human epidermis models are also available that demonstrate reasonable similarities to the native human tissue in terms of morphology, lipid composition and biochemical markers. See, e.g., EpiSkin, SkinEthic and EpiDerm. Animal models of skin injury are also known in the art, including pigs and rodents. See, e.g., Summerfield, A. et al., Molecular Immunology, Vol. 66, Issue 1, July 2015, p. 14-21.

In some embodiments, the stimulus is temperature change. In some embodiments, the adhesive layer adheres to the penis or vagina at a temperature of 37° C and is less adhesive or delaminates from the penis or vagina at a temperature of 25 °C or lower. In some embodiments, the adhesive layer adheres to the glans of the penis at a temperature of 37° C and is less adhesive or delaminates from the glans of the penis at a temperature of 25 °C or lower.

In some embodiments, the adhesive layer may exhibit thermally-responsive stimuli-responsive behavior that enables adhesion to penile skin at body temperature (about 37 °C) and also enables lowered adhesive force or delamination upon cooling below body temperature to about 25 °C, about 20 °C, about 15 °C, about 10 °C, about 5 °C, about 0 °C or lower. Cooling may be achieved, for example, by accelerated heat transfer by rubbing a wet substrate such as a water-impregnated towel or paper towel on the condom or at the interface of the adhered barrier layer while adhered to the penis or by running water from a source such as a shower or a cloth or tissue wipe which contain a reagent which causes cooling (e.g., alcohol evaporation or dissolution of ammonium nitrate) to cool the condom through a thermal transition such that delamination is achievable more easily than if the condom were to be removed at body temperature.

In some embodiments, the stimulus is a physico-chemical change. An exemplary physico-chemical change is dissolution. In some embodiments, the stimulus is a change is dissolution of the adhesive layer when contacted by a solvent. In some embodiments, the composition adheres to the penis or vagina in the absence of the solvent and is less adhesive or delaminates from the penis or vagina when contacted by the solvent. In some embodiments, the composition adheres to the glans of the penis in the absence of the solvent and is less adhesive or delaminates from the glans of the penis when contacted by the solvent.

In some embodiments, the adhesive layer exhibits chemically-responsive behavior and may be water or solvent soluble in nature and may be removed by pulling back edges and flowing water or solvent or rubbing a substrate impregnated with water or solvent until the condom is removed by weakening of adhesive later or dissolution by water or solvent. Chemical delamination may also be achieved in this manner by pH- triggerable delamination by flowing or wiping in this manner with a substrate impregnated with a fluid with a pH suitable for skin contact that also achieves delamination of the condom from skin. Alternatively, chemical delamination may also be achieved by dissolution of the adhesive by flowing or wiping in this manner with a substrate impregnated with a fluid that achieves delamination of the condom from skin.

### 4.2.2.2 Polymer

The adhesive described herein comprises a stimuli-responsive polymer, and optionally, one or more additional polymers.

In some embodiments, the stimuli-responsive polymer has a glass transition temperature (Tg) from 0 °C to 50 °C, such as, for example, from 0 °C to 40 °C, from 0 °C to 30 °C, from 0 °C to 20 °C, from 0 °C to 10 °C, from 5 °C to 50 °C, from 5 °C to 40 °C, from 5 °C to 30 °C, from 5 °C to 20 °C, from 5 °C to 10 °C, from 10 °C to 50 °C, from 10 °C to 40 °C, from 10 °C to 30 °C, from 10 °C to 20 °C, from 20 °C to 50 °C, from 20 °C to 40 °C, from 20 °C to 30 °C, from 30 °C to 50 °C, from 30 °C to 40 °C, or from 40 °C to 50 °C.

In some embodiments, the stimuli-responsive polymer has a glass transition below ambient temperature, such as, for example, a glass transition from -90 °C to 20 °C, from -90 °C to -80 °C, from -80 °C to -70 °C, from -70 °C to -60 °C, from -60 °C to -50 °C, from -50 °C to -40 °C, from -40 °C to -30 °C, from -30 °C to -20 °C, from -20 °C to -10 °C, from -10 °C to 0 °C, from 0 °C to 10 °C, or from 20 °C to 20 °C.

In some embodiments, the stimuli-responsive polymer has a crystalline melt transition below ambient temperature, such as, for example, a crystalline melt transition from -90 °C to 20 °C, from -90 °C to -80 °C, from -80 °C to -70 °C, from -70 °C to -60 °C, from -60 °C to -50 °C, from -50 °C to -40 °C, from -40 °C to -30 °C, from -30 °C to -20 °C, from -20 °C to -10 °C, from -10 °C to 0 °C, from 0 °C to 10 °C, or from 20 °C to 20 °C.

In some embodiments, the stimuli-responsive polymer comprises a single copolymer. In some embodiments, the stimuli-responsive polymer comprises a blend of two or more homopolymers or copolymers, which includes copolymer architecture of block, gradient, and random copolymers of two or more.

In some embodiments, the one or more polymers are selected from, for example, polyacrylates, polymethacrylates, polyurethanes, polyolefins, polyethers, silicones, polyepoxies, synthetic rubbers or other adhesives suitable for use with human skin, including derivatives, copolymers, and mixtures thereof.

In some embodiments, the stimuli-responsive polymer comprises one or more polyacrylate or polymethacrylate polymers.

In some embodiments, the stimuli-responsive polymer comprises one or more polyacrylates or polymethacrylates with 1, 2, 3, 4, 5, 6, 7, 8, 9, 10, 11, 12 or up to 100 carbons in the side chain and 0, 1, 2, 3, 4, 5, 6, 7, 8, 9 10 or up to 100 or more oxygens in the side chain.

Representative, non-limiting polymers include poly(2- ethylhexyl acrylate), poly(butyl acrylate), poly(propyl acrylate), poly(ethyl acrylate), poly(methyl acrylate), poly(octyl acrylate), poly(nonyl acrylate) poly(decyl acrylate), poly(undecyl acrylate), poly(dodecyl acrylate), poly(tridecyl acrylate), poly(C14 acrylate), poly (C15 acrylate), poly(C16 acrylate), poly (C17 acrylate), poly(C18 acrylate), poly(C19 acrylate), poly(C20-C100 or more acrylates, methacrylates and acrylamides thereof), poly(2-hydroxyethyl acrylate), poly(butoxymethyl acrylate), poly(butoxyethyl acrylate), poly(butoxypropyl acrylate), poly(butoxybutyl acrylate), poly(fin(ding)-nemo-acrylate), poly(octadecyl acrylate), poly(octadecyl methacrylate), and poly(acrylic acid).

In some embodiments, the stimuli-responsive polymer comprises polymethacrylate, a polymethacrylate copolymer or blend thereof. The copolymer is derived least one methacrylate monomer and at least one polymerizable comonomer, including any monomer disclosed herein.

In some embodiments, the stimuli-responsive polymer comprises a crosslinked polymethacrylate. The crosslinker can be polyfunctional. In one embodiment, the stimuli-responsive polymer comprises a polymethacrylate crosslinked by an acrylate. In some embodiments, the polymethacrylate is poly(lauryl) methacrylate and the acrylate crosslinker is TMPTA. The weight ratio of the lauryl methacrylate and TMPTA may vary.

In some embodiments, the weight ratio is 98:2, 98.5:1.5, or 99:1, or more particularly, 99.1:0.9, 99.2:0.8, 99.3:0.7, 99.4:0.6, 99.6:0.4, or 99.8:0.2, 99.0:0.1 or any range therein.

In some embodiments, the stimuli-responsive polymer does not comprise polyacrylates or polymethacrylates.

In some embodiments, the stimuli-responsive polymer comprises one or more amorphous or semi-crystalline polyurethanes.

In some embodiments, the stimuli-responsive polymer comprises a semicrystalline polyurethane elastomer with segments that include polyether, polyester, polyurethane, polyurethane urea, poly(isoprene), poly(butadiene) or other crystalline segments.

In some embodiments, the stimuli-responsive polymer comprises semicrystalline polyurethane elastomer either linear or crosslinked with segments that include polyether, polyester, polyurethane, polyurethane urea, poly(isoprene), poly(butadiene) or other crystalline segments.

In some embodiments, the stimuli-responsive polymer does not comprise polyurethane.

In some embodiments, the stimuli-responsive polymer comprises a polyolefin. In some embodiments, the polyolefin is a polyisoprene.

In some embodiments, the stimuli-responsive polymer comprises at least one polyether, for example, poly(ethylene glycol) (PEG) compounds and acrylated or polyurethane-containing PEG compounds.

In some embodiments, the stimuli-responsive polymer comprises at least one polyepoxy. In some embodiments, the polyepoxy comprises one or more epoxy monomers disclosed herein. In some embodiments, the stimuli-responsive polymer does not comprise polyepoxy. In some embodiments, the polyepoxy comprises one or more silicone monomers disclosed herein.

In some embodiments, the stimuli-responsive polymer comprises at least one silicone polymer. In some embodiments, the silicone polymer is a high molecular weight, linear siloxane polymer and a highly condensed, silicate tackifying resin.

In some embodiments, the stimuli-responsive polymer does not comprise a silicone polymer.

In some embodiments, the stimuli-responsive polymer comprises at least one synthetic rubber.

In some embodiments, the stimuli-responsive polymer comprises styrene-butadiene rubber (SBR). Examples of SBRs include those used in the manufacture of pressure sensitive tapes, including synthetic elastomers derived from styrene and butadiene. SBRs, whether solvent borne or waterborne suitable for use in the present disclosure include SBRs with varying percent of bound styrene, average molecular weight and its distribution, and the presence of functional groups introduced during polymerization. Molecular weights of SBRs range from 10 to 1,000,000 g/mol, more specifically 25,000 to 750,000 g/more, more specifically 50,000 to 500,000 g/mol. SBRs generally exhibit low water uptake, less than 1 wt% water, more specifically less than 0.5 wt% water, more specifically 0.1 wt% water, more specifically less than 0.05 wt% water.

In some embodiments, the stimuli-responsive polymer does not comprise synthetic rubber.

In some embodiments, the stimuli-responsive polymer comprises thiol monomers. Exemplary thiol monomers include, but are not limited to, 3-Mercaptopropionic acid; Thioglycolic acid; 3-Mercapto-1-propanol; 2- Mercaptoethanol; 2-(2-Mercaptoethoxy)ethanol; 2-(2-Mercaptopropionylamino)ethanol; 2-(2- Mercaptosuccinyl)ethyl acrylate; 3-(2-Mercaptopropionylamino)propionic acid; 3- (Mercaptopropyl)trimethoxysilane; 2,2'-(Ethylenebis(thio))diethanol; 3-Mercaptopropyltrimethoxysilane; 3-Mercaptopropylmethyldimethoxysilane; 3-(2,2-Dithiobis(ethylthio)propionylamino)propionic acid; 3,6,9- Trioxadecanethiol; 3-Mercapto-1,2-propanediol; 2,2'-Dithiodiethanol; N-Acetyl-L-cysteine; L-Cysteine; 2-(2- Mercaptoethyl)pyridine; 4-(2-Mercaptoethyl)morpholine; 3-Mercapto-1,2,4-triazole; Thiophenol; Pentaerythritol tetrakis(3-mercaptopropionate) (PETMP); Trimethylolpropane tris(3-mercaptopropionate) (TMPMP); Triethanolamine tris(3-mercaptopropionate) (TEAMP); Tris(2-hydroxyethyl) isocyanurate tris(3- mercaptopropionate) (THEICMP); Bis(3-mercaptopropyl) sulfide (BMPS); 1,2-ethanedithiol (EDT); 1,3-propanedithiol; 1,4-butanedithiol; 1,6-hexanedithiol; 1,8-octanedithiol and combinations thereof.

In some embodiments, the stimuli-responsive polymer is a linear or branched crosslinked polymer network wherein the crosslinked polymer network contains 10 or more ester to thiolester linkages which hydrolyze in the presence of an added base or a compound containing a thiol, or a compound containing an amino wherein hydrolysis results in dissolution of the adhesive and delamination from penile skin.

In another embodiment, the stimuli-responsive polymer may be a linear or branched polymer comprising the reaction product by free radical addition polymerization of mono-, di- try-tetra-, penta, and hexafunctional thiol-ene constituents including triallyl isocyanurate pentaerythritol tetrakis (3- mercaptopropionate) or any of the thiol-ene monomeric constituents disclosed herein. In some embodiments, thiol-ene adhesives may exhibit stimuli-responsive adhesive behavior upon cooling below its glass transition. In some embodiments, thiol-ene adhesive layer may exhibit chemically-responsive adhesive behavior such as oxidation of thioether linkages by common oxidizing agents such as hydrogen peroxide to form reversibly clearable disulfide linkages.

In some embodiments, the stimuli-responsive polymer comprises linear or crosslinked polymers that include liquid crystalline polymers with thermal transitions in the range of -10 °C to 50 °C, more particularly 0 °C to 40 °C, more particularly 5 °C to 35 °C, more particularly 5 °C to 20 °C. Liquid crystalline polymer compositions suitable for use include thiol-ene and thiol-acrylate polymers prepared via base catalyzed Michael addition or free radical polymerization processes, including those prepared from thiol building blocks such as 1,6-hexanedithiol, 1,8-octanedithol, 1,10-decanedithiol, 1,12-dodecanedithiol, hexanediol diacrylate, octanediol diacrylate, decanediol diacrylate and diacrylate species containing mesogens such as RM105 - 4-(6- Acryloyloxyhexyloxy)-benzoic acid (4-cyanophenyl ester), RM 23 - 4-Methoxyphenyl 4-((6-(acryloyloxy)hexyl)oxy)benzoate, CB3A - 3-[(3'-Cyanobiphenyl-3-yl)oxy]propylacrylate. Diacrylate mesogens include but are not limited to RM 257 - 4-(3- acryloyoxy-propyloxy) benzoic acid 2-methyl-1,4-phenylene ester, RM 82 - 1,4-Bis[4-(6-acryloyloxyhexyloxy)benzoyloxy]-2-methylbenzene

In some embodiments, free-radical polymerization can be used to crosslink acrylate-functionalized liquid crystal networks. Click chemistry reactions such as Michael addition reactions may also be used to incorporate soft flexible segments in between mesogenic monomers to decrease Tg and enable elastomeric behavior at ambient conditions. In some embodiments, dithiols can be used as flexible spacers and include but are not limited to: ethane dithiol, propane dithiol, or any other dithiol with an all-carbon backbone, 2,2'-(ethylenedioxy)diethanethiol or any other dithiol with a polyethylene glycol backbone, 1,4- benzenedithiol, 4,4'-biphenyldithiol, ethylene bis(thioglycolate), glycol dimercaptopropionate.

In some embodiments, in addition to thiols, amine-functionalized monomers may be used in a similar fashion. For example, n-butylamine can be used as a flexible chain extender or spacer for mesogenic monomers. Using a Michael addition catalyst such as triethyl amine or dipropyl amine, acrylate- functionalized mesogenic oligomers can be created by combining a non-stoichiometric ratio of diacrylate mesogens to dithiol monomers or diacrylate mesogens to difunctional amines. In either case, an excess of acrylate functional groups is optionally preferred. These oligomers can then be photo-crosslinked to form an LCE network.

In some embodiments, LCEs can be synthesized in a one-pot manner by utilizing thiol or amine functionalized crosslinkers with a functionality of 2 or greater. This one-pot approach can be used with both free-radical and Michael-addition polymerization methods. Examples include but are not limited to pentaerythritol tetramercaptoacetate (PETMA), trimethylolpropane tri(3-mercaptopropionate), pentaerythritol tetra(3- mercaptopropionate), di-pentaerythritol tetra(3-mercaptopropionate)

In some embodiments, the stimuli-responsive polymer comprises linear or crosslinked polymers that include silicone polymers, for example, a high molecular weight, linear siloxane polymer and a highly condensed, silicate tackifying resin. Tackifying resins or tackifiers include low-molecular weight compounds with high glass transition temperature used in formulating adhesives to increase the tack, the stickiness of the surface of the adhesive. Tackifiers include resins (e.g., rosins and their derivates, terpenes and modified terpenes, aliphatic, cycloaliphatic and aromatic resins (C5 aliphatic resins, C9 aromatic resins, and C5/C9 aliphatic/aromatic resins), hydrogenated hydrocarbon resins, and their mixtures, terpenephenol resins (TPR, used often with ethylene-vinyl acetate adhesives)), novolacs. Silicone rubber-based pressure-sensitive adhesives suitable for use include special tackifiers based on "MQ" silicate resins, composed of a monofunctional trimethyl silane ("M") reacted with quadrafunctional silicon tetrachloride ("Q").

In some embodiments, the stimuli-responsive polymer exhibits one or more glass transition (Tg), crystallization temperature (Tc), melting temperature (Tm) or other thermal transition ranging from -100 to 100 °C or about - 40 °C, about -30 °C, about -20 °C, about -10 °C, about 10 °C, about 0 °C, about 10 °C, about 15 °C, about 20 °C, about 25 °C, about 30 °C, about 35 °C, about 40 °C, about 45 °C, about 50 °C, about 55 °C, about 60 °C, or about 65 °C or more as measured by differential scanning calorimetry (DSC) Tg, Tc, Tm peak inflection point or dynamic mechanical analysis loss modulus or tan delta peak at 1 Hz.

In some embodiments, the stimuli-responsive polymer may be a linear, brush, star, dendritic, or branched polymer with weight average molecular weight (Mw) of approximately 1 kDa, about 5 kDa, about 10 kDa, about 15 kDa, about 20 kDa, about 30 kDa, about 50kDa, about 75 kDa, about 90 kda, about 100 kDa, about 110 kDa, about 200 kDa, about 300 kDa, about 400 kDa, about 500 kDa, or about 1000 kDa or more.

In some embodiments, the stimuli-responsive polymer is a crosslinked polymer that provides a semi-interpenetrating network or is an interpenetrating network.

In some embodiments, the stimuli-responsive polymer is petroleum-based.

In some embodiments, the stimuli-responsive polymer is bio-based, in whole or in part.

In some embodiments, the stimuli-responsive polymer is a compostable, bio-based or degradable polymer prepared, for example from plasticized polycaprolactone or plasticized poly(lactic acid). In some embodiments, plasticization is carried out to lower glass transition or increase tack.

In some embodiments, the stimuli-responsive polymer is suitable for use in food grade applications such as stickers used for produce or fruit.

The amount of stimuli-responsive polymer present in the adhesive may vary. In some embodiments, the stimuli-responsive polymer comprises at least 70 wt% of the adhesive, such as, for example, at least about 80 wt%, at least 90 wt%, or at least 95 wt%.

In some embodiments, the stimuli-responsive polymer comprises at least 90 wt% of the adhesive, such as, for example, at least 91 wt%, at least 92 wt%, at least 93 wt%, at least 94 wt%, at least 95 wt%, at least 96 wt%, at least 97 wt%, at least 98 wt%, or at least 99 wt%.

In some embodiments, the linear and crosslinked polymers comprise (i) a main chain; (ii) at least one side chain; (iii) crosslinking; and (iv) additives. The main chain (i), side chain (ii) and/or crosslinkers (iii) may comprise one or more monomers.

In some embodiments, the monomers comprising (i), (ii) and (iii) may be the same monomers or different monomers.

The monomers may be any suitable monomers. In one embodiment, the monomers are selected from acrylate monomers, allyl monomers, thiol monomers, epoxy monomers, amine monomers, electron-rich monomers, electron-poor monomers, lactam monomers, lactone monomers, alcohol monomers, carboxylic monomers, isocyanate monomers, Diels Alder monomers, ring opening metathesis monomers or the like.

In some embodiments, the stimuli-responsive polymer does not comprise an acrylic-based adhesive which contains an unreacted polyol plasticizer.

In some embodiments, the stimuli-responsive polymer does not comprise a hydrophobic polyoxyalkylene-based adhesive derived from poly(ethylene glycol) prepared in the presence of a plasticizer.

In some embodiments, the stimuli-responsive polymer does not comprise an acrylic pressure-sensitive adhesive and (i) an elastomer with a tackifying resin or (b) a thermoplastic elastomer. In some embodiments, the adhesive does not comprise an acrylic pressure-sensitive adhesive and (i) an elastomer with a tackifying resin or (b) a thermoplastic elastomer.

The adhesive described herein may be utilized as the adhesive layer in the contraceptive device disclosed herein, e.g., the non-rigid fractional condom described herein, for example, a rigid, non-rolling fractional condom comprising a first adhesive layer (e.g., a stimuli responsive polymer, such as a stimuli-responsive polymer described herein) and a second layer comprising a barrier and reservoir, wherein the condom does not contact the shaft or corona of the penis; wherein the adhesive layer is (a) coextensive with the barrier layer; (b) thicker than the barrier layer and/or (c) the only securing means. The adhesive may be, for example, a stimuli-responsive polymer comprising one or more methacrylate monomers and a tri-functional crosslinker (e.g., TMPTA), providing a low density heterogenous crosslinked polymer. The weight ratio of the polymer to the tri-functional crosslinker may be, for example, about 99.4:06. The non-rigid, fractional condom may exhibit one or more properties disclosed herein, e.g., pain-free removal, low peel strength at low peel rate, high peel strength at high peel rate, loss modulus, etc.

### 4.2.2.2.1 Monomers

In some embodiments, the stimuli-responsive polymer, and optionally, one or more other polymers present in the adhesive, is formed from (i.e., comprises) monomers selected from acrylate monomers, methacrylate monomers, vinyl ether monomer, allyl monomers, thiol monomers, epoxy monomers, amine monomers, electron rich monomers, electron poor monomers, lactam monomers, lactone monomers, alcohol monomers, carboxylic acid monomers, isocyanate monomers, Diels-Alder monomers, ring opening metathesis monomers, or a combination thereof.

In some embodiments, the stimuli-responsive polymer, or optionally, one or more other polymers present in the adhesive, comprises acrylate monomers. In some embodiments, the acrylate monomers are C6-C30 alkyl acrylate monomers, such as, for example, C8-C30 alkyl acrylate monomers, C8-C20 alkyl acrylate monomers, C8-C16 alkyl acrylate monomers, C8-C12 alkyl acrylate monomers, C12-C30 alkyl acrylate monomers, C12-C20 alkyl acrylate monomers, or C12-C16 alkyl acrylate monomers.

Exemplary acrylate monomers include, but are not limited to, octyl acrylate, nonyl acrylate, decyl acrylate, undecyl acrylate, dodecyl acrylate, tridecyl acrylate, tetradecyl acrylate, pentadecyl acrylate, hexadecyl acrylate, heptadecyl acrylate, octadecyl acrylate, nonadecyl acrylate, eicosyl acrylate, heneicosyl acrylate, docosyl acrylate, tricosyl acrylate, tetracosyl acrylate, pentacosyl acrylate, hexacosyl acrylate, heptacosyl acrylate, octacosyl acrylate, nonacosyl acrylate, triacontyl acrylate, methyl acrylate, ethyl acrylate, Butyl acrylate, 2-ethylhexyl acrylate, isobutyl acrylate, Methoxyethyl acrylate, Hydroxyethyl acrylate, Hydroxypropyl acrylate, Ethoxylated hydroxyethyl acrylate, Glycidyl acrylate, Methacrylic acid, Methyl methacrylate, Ethyl methacrylate, Butyl methacrylate, 2-Hydroxyethyl methacrylate, Cyclohexyl methacrylate, Poly(ethylene glycol) methacrylate, Poly(ethylene glycol) diacrylate, Poly(ethylene glycol) dimethacrylate, Trimethylolpropane triacrylate, Triethylene glycol diacrylate, Tetraethylene glycol diacrylate, Neopentyl glycol diacrylate, Diethylene glycol diacrylate, Dipentacrythritol hexaacrylate, Ethoxylated trimethylolpropane triacrylate, Propoxylated glycerol triacrylate, Stearyl acrylate, Lauryl acrylate, Isodecyl acrylate, Acrylic acid, Ethylene glycol diacrylate (EGDA), Triethylene glycol diacrylate (TEGDA), Propylene glycol diacrylate (PGDA), Butanediol diacrylate (BDDA), Neopentyl glycol diacrylate (NPGDA), Pentaerythritol tetraacrylate (PETA), 1,4-Butanediol diacrylate (BDA), Di(trimethylolpropane) tetraacrylate (DTMPTA), Bisphenol A ethoxylate diacrylate (BPAEDA), Ethoxylated bisphenol A diacrylate (EBPA), Decanediol diacrylate, Polyethylene glycol diacrylate (PEGDA), Trimethylolpropane triacrylate (TMPTA), Diethylene glycol diacrylate (DEGDA), and 1,6-Hexanediol diacrylate (HDDA). Trimethylolpropane triacrylate (TMPTA), Tripropylene glycol diacrylate (TPGDA), Pentaerythritol triacrylate (PETA), Dipentaerythritol pentaacrylate (DPEPA), Tris(2-hydroxyethyl) isocyanurate triacrylate (THEIC-TA), Triethylene glycol dimethacrylate (TEGDMA), Triallyl isocyanurate (TAIC), Triethylene glycol diacrylate (TEGDA), Ethoxylated trimethylolpropane triacrylate (ETMPTA), Triallyl cyanurate (TAC), and combinations thereof.

In certain embodiments, the stimuli-responsive polymer, or optionally, one or more other polymers present in the adhesive, comprises acrylate monomers selected from octyl acrylate, nonyl acrylate, decyl acrylate, undecyl acrylate, dodecyl acrylate, tridecyl acrylate, tetradecyl acrylate, pentadecyl acrylate, hexadecyl acrylate, heptadecyl acrylate, octadecyl acrylate, nonadecyl acrylate, eicosyl acrylate, heneicosyl acrylate, docosyl acrylate, tricosyl acrylate, tetracosyl acrylate, pentacosyl acrylate, hexacosyl acrylate, heptacosyl acrylate, octacosyl acrylate, nonacosyl acrylate, triacontyl acrylate, and combinations thereof.

In some embodiments, the acrylate monomer is TMPTA.

In some embodiments, the stimuli-responsive polymer comprises at least 10 wt% acrylate monomers, such as, for example, at least 50 wt%, at least 75 wt%, or at least 90 wt%. In some embodiments, the stimuli-responsive polymer comprises at least 95 wt% acrylate monomers, e.g., at least 96 wt%, at least 97 wt%, at least 98 wt%, or at least 99 wt%.

In some embodiments, the stimuli-responsive polymer, or optionally, one or more other polymers present in the adhesive, comprises methacrylate monomers. In some embodiments, the methacrylate monomers are C6-C30 alkyl methacrylate monomers, such as, for example, C8-C30 alkyl methacrylate monomers, C8-C20 alkyl methacrylate monomers, C8-C16 alkyl methacrylate monomers, C8-C12 alkyl methacrylate monomers, C12-C30 alkyl methacrylate monomers, C12-C20 alkyl methacrylate monomers, or C12-C16 alkyl methacrylate monomers.

Exemplary methacrylate monomers include, but are not limited to, hexyl methacrylate, octyl methacrylate, nonyl methacrylate, decyl methacrylate, undecyl methacrylate, dodecyl methacrylate, tridecyl methacrylate, tetradecyl methacrylate, pentadecyl methacrylate, hexadecyl methacrylate, heptadecyl methacrylate, octadecyl methacrylate, nonadecyl methacrylate, eicosyl methacrylate, heneicosyl methacrylate, docosyl methacrylate, tricosyl methacrylate, tetracosyl methacrylate, pentacosyl methacrylate, hexacosyl methacrylate, heptacosyl methacrylate, octacosyl methacrylate, nonacosyl methacrylate, triacontyl methacrylate, Ethyl methacrylate, Butyl methacrylate, 2-Hydroxyethyl methacrylate, Cyclohexyl methacrylate, Isobornyl methacrylate, Stearyl methacrylate, Lauryl methacrylate, Isodecyl methacrylate, Tetrahydrofurfuryl methacrylate, Glyceryl methacrylate, Trimethylolpropane trimethacrylate, Trimethylolpropane triacrylate, Pentaerythritol triacrylate, Pentaerythritol tetramethacrylate, Poly(ethylene glycol) monomethyl ether methacrylate, Poly(ethylene glycol) monomethyl ether acrylate, Poly(ethylene glycol) diacrylate, Poly(ethylene glycol) dimethacrylate, Poly(ethylene glycol) monoacrylate, Ethoxylated bisphenol A dimethacrylate, Ethoxylated bisphenol A diacrylate, Ethoxylated trimethylolpropane triacrylate, Hydroxypropyl methacrylate, Methacrylic acid, Acryloyloxyethyltrimethylammonium chloride, Diethylaminoethyl methacrylate, Butylaminoethyl methacrylate, N,N-dimethylaminoethyl methacrylate, Methacryloyloxyethyl phthalate, Cyclopropyl methacrylate, and combinations thereof.

In some embodiments, the stimuli-responsive polymer, or optionally, one or more other polymers present in the adhesive, comprises methacrylate monomers selected from hexyl methacrylate, octyl methacrylate, nonyl methacrylate, decyl methacrylate, undecyl methacrylate, dodecyl methacrylate, tridecyl methacrylate, tetradecyl methacrylate, pentadecyl methacrylate, hexadecyl methacrylate, heptadecyl methacrylate, octadecyl methacrylate, nonadecyl methacrylate, eicosyl methacrylate, heneicosyl methacrylate, docosyl methacrylate, tricosyl methacrylate, tetracosyl methacrylate, pentacosyl methacrylate, hexacosyl methacrylate, heptacosyl methacrylate, octacosyl methacrylate, nonacosyl methacrylate, triacontyl methacrylate, and combinations thereof. In some embodiments, the stimuli-responsive polymer comprises undecyl methacrylate monomers, i.e., lauryl methacrylate monomers. In some embodiments, the stimuli-responsive polymer comprises octadecyl methacrylate monomers, i.e., stearyl methacrylate monomers.

In some embodiments, the stimuli-responsive polymer, or optionally, one or more other polymers present in the adhesive, comprises at least 10 wt% methacrylate monomers, such as, for example, at least 50 wt%, at least 75 wt%, or at least 90 wt%. In some embodiments, the stimuli-responsive polymer comprises at least 95 wt% methacrylate monomers, e.g., at least 96 wt%, at least 97 wt%, at least 98 wt%, or at least 99 wt%.

In some embodiments, the stimuli-responsive polymer, or optionally, one or more other polymers present in the adhesive, comprises vinyl ether monomers. Exemplary vinyl ether monomers include, but are not limited to, divinyl ether of ethylene glycol, divinyl ether of diethylene glycol, divinyl ether of triethylene glycol, divinyl ether of polyethylene glycol (DVE-PEG), divinyl ether of polypropylene glycol (DVE-PPG), Divinyl ether of poly(ethylene glycol) methyl ether (DVE-PEGME), divinyl ether of poly(ethylene glycol) butyl ether (DVE), Divinyl ether of poly(ethylene glycol) phenyl ether (DVE-PEGPhE), Divinyl ether of glycerol (DVE-Gly), Divinyl ether of 1,4-cyclohexanedimethanol (DVE-CHDM), Divinyl ether of neopentyl glycol (DVE-NPG), and combinations thereof.

In some embodiments, the stimuli-responsive polymer, or optionally, one or more other polymers present in the adhesive, comprises allyl monomers. Exemplary allyl monomers include, but are not limited to, Diallyl phthalate (DAP), Diallyl maleate (DAM), Diallyl succinate (DAS), Diallyl fumarate (DAF), Diallyl adipate (DAA), Diallyl sebacate (DAS), Diallyl terephthalate (DAT), Diallyl isophthalate (DAI), Diallyl itaconate (DAI), Diallyl carbonate (DAC), Diallyl diglycolate (DADG), Diallyl tris(2- hydroxyethyl) isocyanurate (DATHEIC), Triallyl cyanurate (TAC), Triallyl isocyanurate (TAIC), Triallyl trimellitate (TATM), Triallyl citrate (TAC), Triallyl phosphate (TAP), Triallylamine (TAA), Triallyl cyanide (TACN), Triallyl benzene-1,2,4-tricarboxylate (TABTC), Triallyl trimesate (TATM), Tris(2-hydroxyethyl) isocyanurate triallyl ether (THEIC-TAE), and combinations thereof

In some embodiments, the stimuli-responsive polymer, or optionally, one or more other polymers present in the adhesive, comprises thiol monomers. Exemplary thiol monomers include, but are not limited to, 3-Mercaptopropionic acid; Thioglycolic acid; 3-Mercapto-1-propanol; 2-Mercaptoethanol; 2-(2-Mercaptoethoxy)ethanol; 2-(2-Mercaptopropionylamino)ethanol; 2-(2-Mercaptosuccinyl)ethyl acrylate; 3-(2-Mercaptopropionylamino)propionic acid; 3-(Mercaptopropyl)trimethoxysilane; 2,2'-(Ethylenebis(thio))diethanol; 3-Mercaptopropyltrimethoxysilane; 3-Mercaptopropylmethyldimethoxysilane; 3-(2,2-Dithiobis(ethylthio)propionylamino)propionic acid; 3,6,9- Trioxadecanethiol; 3-Mercapto-1,2-propanediol; 2,2'-Dithiodiethanol; N-Acetyl-L-cysteine; L-Cysteine; 2-(2-Mercaptoethyl)pyridine; 4-(2-Mercaptoethyl)morpholine; 3-Mercapto-1,2,4-triazole; Thiophenol; Pentaerythritol tetrakis(3-mercaptopropionate) (PETMP); Trimethylolpropane tris(3-mercaptopropionate) (TMPMP); Triethanolamine tris(3-mercaptopropionate) (TEAMP); Tris(2-hydroxyethyl) isocyanurate tris(3- mercaptopropionate) (THEICMP); Bis(3-mercaptopropyl) sulfide (BMPS); 1,2-ethanedithiol (EDT); 1,3- propanedithiol; 1,4-butanedithiol; 1,6-hexanedithiol; 1,8-octanedithiol and combinations thereof.

In some embodiments, the stimuli-responsive polymer, or optionally, one or more other polymers present in the adhesive, comprises epoxy monomers. Exemplary epoxy monomers include, but are not limited to, Bisphenol A diglycidyl ether (BADGE), Bisphenol F diglycidyl ether (BFDGE), Novolac diglycidyl ether (NGDE), Phenol novolac diglycidyl ether (PNGDE), Cycloaliphatic epoxy resins, Glycidyl ethers of aliphatic alcohols, Glycidyl ethers of aromatic alcohols, Triglycidyl isocyanurate (TGIC), Diglycidyl ether of 1,4-butanediol (BDDGE), Diglycidyl ether of neopentyl glycol (NPGDGE), Diglycidyl ether of propylene glycol (PGDGE), Epoxidized soybean oil (ESO), Epoxidized linseed oil (ELO), Dicyclopentadiene- based epoxy resins, Tetrafunctional epoxy resins, Epoxy phenolic novolac resins, and combinations thereof.

In some embodiments, the stimuli-responsive polymer, or optionally, one or more other polymers present in the adhesive, comprises amine monomers. Exemplary amine monomers include, but are not limited to, Ethylenediamine; Diethylenetriamine; Triethylenetetramine; Tetraethylenepentamine; Polyethyleneimine; Diaminopropane; Diaminobutane; Diaminopentane; Diethylenetriaminepentaacetic acid (DTPA); Tris(2-aminoethyl)amine; N-(2-Aminoethyl)piperazine; N-(3- Aminopropyl)morpholine; N,N-Dimethylaminopropylamine; N,N-Dimethylethylenediamine; 1,3- Diaminopropane; Isophoronediamine; Jeffamine D-230; Jeffamine T-403; Jeffamine M-207; Jeffamine EDR- 148; and combinations thereof.

In some embodiments, the stimuli-responsive polymer, or optionally, one or more other polymers present in the adhesive, comprises electron rich monomers. Exemplary electron rich monomers include, but are not limited to, Vinyl ethers (e.g. vinyl methyl ether, vinyl ethyl ether), Vinyl acetate, Allyl alcohol, Allyl amine, N-Methylolacrylamide, N-Methylolmethacrylamide, N-Methylolallylamine, N- Methylolvinylacetamide, Acrolein diethyl acetal, Acrolein diethyl ketal, Diacetone acrylamide, 2- Hydroxyethyl acrylate, 2-Hydroxyethyl methacrylate, 2-Hydroxypropyl acrylate, 2-Hydroxypropyl methacrylate, 2,3-Dihydroxypropyl methacrylate, Glycidyl methacrylate, Glycidyl acrylate, Tetrahydrofurfuryl methacrylate, and N-Vinylpyrrolidone, n-vinylformamide, n-vinyl pyridine, styrene, styrene derivatives, and combinations thereof.

In some embodiments, the stimuli-responsive polymer, or optionally, one or more other polymers present in the adhesive, comprises electron poor monomers. Exemplary electron poor monomers include, but are not limited to, Acrylonitrile, Methacrylonitrile, Methyl methacrylate, Acrylic acid, Methacrylic acid, Maleic anhydride, Itaconic acid, Fumaric acid, Acrylamide, Methacrylamide, N- Vinylcarbazole, Vinylidene chloride, Vinyl chloride, Vinyl sulfonic acid, Vinyl acetate, Styrene, Alpha¬methylstyrene, Maleimide, N-Phenylmaleimide, and N-Butylmalcimide, maleic anhydride, and combinations thereof.

In some embodiments, the stimuli-responsive polymer, or optionally, one or more other polymers present in the adhesive, comprises lactam monomers. Exemplary lactam monomers include, but are not limited to, Caprolactam, Valerolactam, Enantholactam, Capryllactam, Laurinlactam, Prolactam, Butyrolactam, Methionyl lactam, Methoxyethyl lactam, Methoxyethyl methionyl lactam, Dimethylaminoethyl lactam, Dimethylaminoethyl methionyl lactam, Dimethylaminoethyl acryloyl lactam, Dimethylaminoethyl methacryloyl lactam, N-vinylpyrrolidone, N-methylpyrrolidone, N- ethylpyrrolidone, and combinations thereof.

In some embodiments, the stimuli-responsive polymer, or optionally, one or more other polymers present in the adhesive, comprises lactone monomers. Exemplary lactone monomers include, but are not limited to, β-propiolactone, γ-butyrolactone, 8-valerolactone, ε-caprolactone, ω- pentadecalactone, β-butyrolactone, 8-decalactone, ε -decalactone, γ-decalactone, 8-dodecalactone, γ-dodecalactone, α-methylene- γ-butyrolactone, β-methyl-y-butyrolactone, β-methyl-γ-valerolactone, γ-hexalactone, and combinations thereof.

In some embodiments, the stimuli-responsive polymer, or optionally, one or more other polymers present in the adhesive, comprises alcohol monomers. Exemplary alcohol monomers include, but are not limited to, Ethylene glycol; Propylene glycol;1,3-Butanediol; 1,4-Butanediol; 1,5- Pentanediol; 1,6-Hexanediol; 1,10-Decanediol; Neopentyl glycol; Diethylene glycol; Triethylene glycol; Tetraethylene glycol; Polyethylene glycol (PEG); Polypropylene glycol (PPG); Polycaprolactone diol; Polymethylolpropane; Hydroxypivalyl hydroxymethylbutyrate (HPHMB); 1,4-Cyclohexanedimethanol; and combinations thereof.

In some embodiments, the stimuli-responsive polymer, or optionally, one or more other polymers present in the adhesive, comprises carboxylic acid monomers. Exemplary carboxylic acid monomers include, but are not limited to, Adipic acid, Succinic acid, Glutaric acid, Sebacic acid, Malonic acid, Phthalic acid, Isophthalic acid, Terephthalic acid, Fumaric acid, Maleic acid, Itaconic acid, Citric acid, 1,4- Cyclohexanedicarboxylic acid, 1,3-Cyclohexanedicarboxylic acid, Dodecanedioic acid, and combinations thereof.

In some embodiments, the stimuli-responsive polymer, or optionally, one or more other polymers present in the adhesive, comprises isocyanate monomers. Exemplary isocyanate monomers include, but are not limited to, Toluene diisocyanate (TDI), Diphenylmethane diisocyanate (MDI), Hexamethylene diisocyanate (HDI), Isophorone diisocyanate (IPDI), 1,6-Hexamethylene diisocyanate (HMDI), 4,4'-Methylenebis(cyclohexyl isocyanate) (H12MDI), Naphthalene diisocyanate (NDI), 2,4-Toluene diisocyanate (2,4-TDI), 2,6-Toluene diisocyanate (2,6-TDI), Polymethylene polyphenyl isocyanate (PAPI), Desmodur N-100, Desmodur L-75, Desmodur HL, Desmodur H, Desmodur VP, Desmodur Z, and combinations thereof.

In some embodiments, the stimuli-responsive polymer, or optionally, one or more other polymers present in the adhesive, comprises Diels-Alder monomers. Exemplary Diels-Alder monomers include, but are not limited to, Maleic anhydride, Furan, Cyclopentadiene, N-phenylmaleimide, Anthracene, N-ethylmaleimide, N-phenylnorbornene, N,N-dimethyl maleimide, 2,5-dimethylfuran, Tetracyanoethylene, Methyl vinyl ketone, and combinations thereof.

In some embodiments, the stimuli-responsive polymer, or optionally, one or more other polymers present in the adhesive, comprises ring opening metathesis monomers. Exemplary ring opening metathesis monomers include, but are not limited to, Norbornene, Dicyclopentadiene (DCPD), Cyclooctene, Tetracyclododecene (TCD), Cyclopentene, Cycloheptene, Cyclohexene, Bicyclo[2.2.1]hept-2-ene, Bicyclo[2.2.2]oct-5-ene, Tricyclo[5.2.1.0(2,6)]dec-8-ene (TCD-Diene), and combinations thereof.

### 4.2.2.2.2 Side Chains

In some embodiments, the stimuli-responsive polymer comprises two or more side chains.

In some embodiments, the stimuli-responsive polymer comprises C6 to C30 side chains or C6 to C30 dangling chain ends. In some embodiments, the C6 to C30 side chains or C6 to C30 dangling chain ends are C6 to C30 alkyl side chains, preferably C12 to C18 alkyl side chains.

In some embodiments, stimuli-responsive polymers comprise at least 80 wt% side chains, such as, for example, at least 85 wt%, at least 90 wt%, or at least 95 wt%. In some embodiments, the side chains are the same. In some embodiments, the side chains are different.

In some embodiments, the side chain comprises linkages made from monomers selected from acrylate, methacrylate, thiol-acrylate Michael Addition, acrylate amine Michael Addition, epoxy thiol, epoxy amine, polyethylenimine (PEI), thiol-ene, alternating copolymers made from C=C electron poor + C=C electron rich monomers, urethane, urea, acrylamide, methacrylamide, polyester, polycarbonate, polyamide, peptoid, peptide, Diels- alder, lactides and lactams, and ring opening metathesis polymerization or olefin metathesis reactions.

In some embodiments, side chain chemistries include C1-C100 side chain linkages achieved by synthetic pathways disclosed herein.

In some embodiments, stimuli-responsive polymers with C6 to C18 side chains or dangling chain ends are prepared from acrylate, methacrylate, alcohol, carboxylic acid, electron rich alkene, electron poor alkene, epoxy, amine, ROMP, Diels-Alder, Lactone, Lactam, Peptide, Peptoid, acrylamide, methacrylamide, thiol, vinyl and allyl monomers.

In some embodiment, the stimuli-responsive polymer comprises a linear or crosslinked polymer with a side chain that optionally undergoes crystallization and/or melting in the region around body and room temperature, in the range of 0 °C to 50 °C, more particularly in the range of 5 °C to 45 °C, more particularly in the range of 10 °C to 40 °C, more particularly in the range of 15 °C to 35 °C. For clarity, side chain crystallization is optional.

In some embodiments, ranges for the linear or crosslinked polymer range from about 40 to about 100%, more particularly, about 50 to 100%. In some embodiments, the range is from about 40 to about 50%, about 50 to about 60%, about 60 to about 70%, about 70 to about 80%, about 80 to about 90% or about 90% or more or any range or value subsumed therein.

In some embodiments, ranges for secondary side chain optionally crystallizable polymer range from about 0 to about 50%, more particularly 1 to about 49%, more particularly about 2 to about 48%, more particularly about 3 to 47%, more particularly about particularly 5 to about 45% or any additional range or value subsumed therein.

### 4.2.2.2.3 Polyfunctional Crosslinker

In the embodiments according to the claims, the stimuli-responsive polymer, is a crosslinked polymer. Crosslinked polymers are prepared using one or more polyfunctional crosslinkers. Crosslinkers may exhibit functionality greater than n=1 reactive site statistically, for example, statistical averages of n = 2, 3, 4, 5, 6, 7, 8, 9, 10 or more reactive sites and may facilitate branching, hyperbranching, interpenetrating networks, semi-interpenetrating networks and partially heterogeneous or generally heterogeneous networks with respect to phase blending or concentration of crosslink density.

In some embodiments, the polyfunctional crosslinker is selected from a difunctional crosslinker, a trifunctional crosslinker, or a tetrafunctional crosslinker. In some embodiments, the polyfunctional crosslinker is a trifunctional crosslinker.

In some embodiments, the polyfunctional crosslinker is a trifunctional crosslinker. In some embodiments, the trifunctional crosslinker is a trifunctional acrylate crosslinker.

In some embodiments, the polyfunctional crosslinker is selected from poly(ethylene glycol) diacrylate, trimethylolpropane triacrylate (TMPTA); ethoxylated trimethyolpropane triacrylate; pentaerythritol tetraacrylate; ethoxylated pentaerythritol tetraacrylate; dipentaerythritol hexaacrylate; ethoxylated dipentaerythritol hexaacrylate; di-, tri-, tetra-, penta-, or hexa- epoxides; polythiols; polyalkenes; tris(2-acryloxyethyl) isocyanurate, ε-caprolactone modified tris(2-acryloxyethyl) isocyanurate, ethoxylated glycerine triacrylate, pentaerythritol triacrylate, and combinations thereof.

In some embodiments, the polyfunctional crosslinker is poly(ethylene glycol) diacrylate with internal repeat units ranging from 1 to 1000 or more, trimethylolpropane triacrylate (TMPTA), ethoxylated trimethyolpropane triacrylate with repeat units ranging from 1 to 1000 or more, pentaerythritol tetraacrylate, ethoxylated pentaerythritol tetraacrylate with repeat units ranging from 1 to 1000 or more, penta and hexafunctional acrylates and ethoxylated versions as described above including dipentaerythritol hexaacrylate and ethoxylated dipentaerythritol hexaacrylate with repeat units ranging from 1 to 1000 or more, di, tri, tetra, penta, hexa more expoxide monomers, polythiols, polyalkenes cured by UV light, visible light, gamma or e-beam irradiation, heat or hydrosilation.

In some embodiments, the polyfunctional crosslinker is selected from trimethylolpropane triacrylate (TMPTA), ethoxylated trimethylolpropane triacrylate, polyethylene glycol diacrylate, pentaerythritol tetraacrylate, ethoxylated pentaerythritol tetraacrylate, hexanediol diacrylate, and combinations thereof. In some embodiments, the polyfunctional crosslinker is trimethylolpropane triacrylate (TMPTA).

In the embodiments covered by the claims, the stimuli-responsive polymer is a crosslinked polymer having a heterogeneous crosslink network. For example, favorable adhesive failure or delamination from skin, including residual adhesive removal after peeling away of adhesive, may be achieved by heterogeneous crosslinking distribution to concentrate failure sites within network or a "swollen heterogeneous network" formed by polymerization of a crosslinker and a monomer with different reactivity ratios.

In some embodiments, the stimuli-responsive polymer is characterized by low-density crosslinking.

In some embodiments, the crosslinker is aggregated within the stimuli-responsive polymer network. This embodiment creates high stress concentration network sites and/or drives rheological behavior and enables energy dissipation (high tan delta and high loss modulus in comparison with those in a more uniform network) to facilitate adhesive failure when desirable.

The amount of the crosslinker in the stimuli-responsive polymer, may vary. In some embodiments, the polymer comprises from 0 to 30 wt % of the crosslinker, such as, for example, from 0.001 to 29 wt%, more particularly 0.005 to 28 wt%, more particularly 0.0075 to 28 wt%, more particularly 0.01 to 27 wt%, more particularly 0.02 to 26 wt%, more particularly 0.05 to 26 wt%, more particularly 0.1 to 25 wt%, more particularly 0.15 to 24 wt%.

In some embodiments, the stimuli-responsive polymer comprises a crosslinker in an amount selected from about 0.1 wt%, about 0.2 wt%, about 0.3 wt%, about 0.4 wt%, about 0.5 wt%, about 0.6 wt%, about 0.7 wt%, about 7.5 wt%, about 0.8 wt%, about 0.9 wt%, about 9.5 wt% or about 1.0 wt% or more, in each case with respect to the weight of the polymer.

In a particular embodiment, the stimuli-responsive polymer comprises a crosslinker in an amount from about 0.1 wt% and about 1 wt%, from about 0.2 wt% to about 0.8 wt%, from about 0.3 wt% to about 0.7 wt%, or from about 0.4 wt% to about 0.6 wt%, in each case with respect to the weight of the polymer.

In some embodiments, the stimuli-responsive polymer comprises from 0.1 wt% to 2.5 wt% polyfunctional crosslinker, such as, for example, from 0.1 wt% to 2 wt%, from 0.1 to 1.5 wt%, from 0.1 wt% to 1 wt%, from 0.1 wt% to 0.9 wt, from 0.1 wt% to 0.8 wt%, from 0.1 wt% to 0.7 wt%, from 0.1 wt% to 0.6 wt%, from 0.1 wt% to 0.5 wt%, from 0.1 wt% to 0.4 wt%, from 0.1 wt% to 0.3 wt% from 0.1 wt% to 0.2 wt%, from 0.2 wt% to 1.5 wt%, from 0.2 wt% to 1 wt%, from 0.3 wt% to 1.5 wt%, from 0.3 wt% to 1 wt%, from 0.4 wt% to 1.5 wt%, from 0.4 wt% to 1 wt%, from 0.5 wt% to 1.5 wt%, from 0.5 wt% to 1 wt%, from 0.6 wt% to 1.5 wt%, from 0.6 wt% to 1 wt%, from 0.7 wt% to 1.5 wt%, from 0.7 wt% to 1 wt%, from 0.8 wt% to 1.5 wt%, from 0.8 wt% to 1 wt%, from 0.9 wt% to 1.5 wt% or from 0.9 wt% to 1 wt%.

In some embodiments, the stimuli-responsive polymer comprises from 0.4 wt% to 0.8 wt% polyfunctional crosslinker, such as, for example, from 0.4 wt% to 0.7 wt%, from 0.4 wt% to 0.6 wt%, from 0.4 wt% to 0.5 wt%, from 0.5 wt% to 0.8 wt%, from 0.5 wt% to 0.7 wt%, from 0.5 wt% to 0.6 wt%, from 0.6 wt% to 0.8 wt%, from 0.6 wt% to 0.7 wt%, or from 0.7 wt% to 0.8 wt%. In some embodiments, the polyfunctional crosslinker is trimethylolpropane triacrylate (TMPTA).

In some embodiment, the weight ratio of the one or more monomers to the one or more polyfunctional crosslinkers is 99:1, 98: 2, 97:3, 96:4, 95.3, 94:6, 93.7: 92:8, 91:9, 90:10, 89:11, 88:12, 87:13, 86:14; 85: 15, 84:16, 83:17, 82:18, 81:19, 80:20, 79:21, 78:22, 77:23, 76:24, 75:25, 74:26, 73:27, 72:28, 71: 29, 70:30, 69:31, 68:32, 67:33, 66:34, 65:35, 64:36, 63:37, 62:38, 61:39, 60:40, 59:41, 58:42, 57:43, 56:44, 55:45, 54:46, 53:47, 52:48, 51:49 or 50:50.

In some embodiments, the weight ratio of the one or more monomers to the one or more polyfunctional crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, from 99.7:0.3 to 99.9:0.1, or from 99.8:0.2 to 99.9:0.1.

### 4.2.2.2.4 Embodiments

In some embodiments, the stimuli-responsive polymer comprises one or more methacrylate monomer and one or more acrylate monomer crosslinker. In some embodiments, the weight ratio of the one or more methacrylate monomer to the one or more acrylate monomer crosslinker may vary. In some embodiments, the weight ratio is 99:1, 98:2, 97:3, 96:4, 95.3, 94:6, 93.7: 92:8, 91:9, 90:10, 89:11, 88:12m 87:13, 86:14; 85: 15, 84:16, 83:17, 82:18, 81:19, 80:20, 79:21, 78:22, 77:23, 76:24, 75:25, 74:26, 73:27, 72:28, 71: 29, 70:30, 69:31, 68:32, 67:33, 66:34, 65:35, 64:36, 63:37, 62:38, 61:39, 60:40, 59:41, 58:42, 57:43, 56:44, 55:45, 54:46, 53:47, 52:48, 51:49 or 50:50.

In some embodiments, the stimuli-responsive polymer comprises one or more C6-C30 alkyl methacrylate monomers and one or more C6-C30 alkyl acrylate monomers. The weight ratio of the one or more C6-C30 alkyl methacrylate monomers to the one or more C6-C30 alkyl acrylate monomers may vary. In some embodiments, the weight ratio is 99:1, 98: 2, 97:3, 96:4, 95.3, 94:6, 93.7: 92:8, 91:9, 90:10, 89:11, 88:12m 87:13, 86:14; 85: 15, 84:16, 83:17, 82:18, 81:19, 80:20, 79:21, 78:22, 77:23, 76:24, 75:25, 74:26, 73:27, 72:28, 71: 29, 70:30, 69:31, 68:32, 67:33, 66:34, 65:35, 64:36, 63:37, 62:38, 61:39, 60:40, 59:41, 58:42, 57:43, 56:44, 55:45, 54:46, 53:47, 52:48, 51:49 or 50:50.

In some embodiments, the stimuli-responsive polymer comprises both C6-C30 alkyl acrylate monomers and C6-C30 alkyl methacrylate monomers. The molar ratio of the components according to this embodiment may vary consistent with stoichiometric calculations from mass ratio equivalents.

In some embodiments, the stimuli-responsive polymer comprises poly(lauryl methacrylate), i.e., poly(dodecyl methacrylate) crosslinked with one or more polyfunctional crosslinkers. In some embodiments, the weight ratio of the lauryl methacrylate to the one or more polyfunctional crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, or from 99.7:0.3 to 99.9:0.1, from 99.8:0.2 to 99.9:0.1.

In some embodiments, the stimuli-responsive polymer comprises poly(lauryl methacrylate) crosslinked with one or more trifunctional crosslinkers. In some embodiments, the weight ratio of the lauryl methacrylate to the one or more trifunctional crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, or from 99.7:0.3 to 99.9:0.1, or from 99.8:0.2 to 99.9:0.1.

In some embodiments, the stimuli-responsive polymer comprises poly(lauryl methacrylate) crosslinked with an acrylate crosslinker. In some embodiments, the weight ratio of the lauryl methacrylate to the one or more acrylate crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, or from 99.7:0.3 to 99.9:0.1, or from 99.8:0.2 to 99.9:0.1.

In some embodiments, the stimuli-responsive polymer comprises poly(lauryl methacrylate) crosslinked with a trifunctional acrylate crosslinker. In some embodiments, the weight ratio of the lauryl methacrylate to the one or more trifunctional acrylate crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, or from 99.7:0.3 to 99.9:0.1, or from 99.8:0.2 to 99.9:0.1.

In some embodiments, the stimuli-responsive polymer comprises poly(lauryl methacrylate) crosslinked with one or more polyfunctional crosslinkers selected from poly(ethylene glycol) diacrylate, trimethylolpropane triacrylate; ethoxylated trimethyolpropane triacrylate; pentaerythritol tetraacrylate; ethoxylated pentaerythritol tetraacrylate; dipentaerythritol hexaacrylate; ethoxylated dipentaerythritol hexaacrylate; di-, tri-, tetra-, penta-, or hexa- epoxides; polythiols; polyalkenes; tris(2-acryloxyethyl) isocyanulate, ε-caprolactone modified tris(2-acryloxyethyl) isocyanurate, ethoxylated glycerine triacrylate, ethoxylated glycerine triacrylate, and pentaerythritol triacrylate. In some embodiments, the weight ratio of the lauryl methacrylate to the one or more polyfunctional crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, or from 99.7:0.3 to 99.9:0.1, or from 99.8:0.2 to 99.9:0.1.

In some embodiments, the stimuli-responsive polymer comprises poly(lauryl methacrylate) crosslinked with one or more polyfunctional crosslinkers selected from trimethylolpropane triacrylate, ethoxylated trimethylolpropane triacrylate, polyethylene glycol diacrylate, pentaerythritol tetraacrylate, ethoxylated pentaerythritol tetraacrylate, hexanediol diacrylate, and combinations thereof. In some embodiments, the weight ratio of the lauryl methacrylate to the one or more polyfunctional crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, or from 99.7:0.3 to 99.9:0.1, or from 99.8:0.2 to 99.9:0.1.

In some embodiments, the stimuli-responsive polymer comprises poly (lauryl methacrylate) crosslinked with trimethylolpropane triacrylate. In some embodiments, the weight ratio of the lauryl methacrylate to the trimethylolpropane triacrylate is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, or from 99.7:0.3 to 99.9:0.1, or from 99.8:0.2 to 99.9:0.1.

In some embodiments, the adhesive comprises poly(lauryl methacrylate) and TMPTA, wherein the TMPTA crosslinks the lauryl methacrylate. The weight ratio of the components according to this embodiment may vary. In some embodiments, the weight ratio is 99:1, or more particularly, about 99.4: 0.6, about 99.6: 0.4 or about 99.8: 0.2.

Any of the above stimuli-responsive polymers may form the adhesive layer, optionally with other additive or components, of the condom described herein and more particularly, the non-rigid, non-rolled, fractional condom described herein wherein comprising a first adhesive layer (e.g., a stimuli-responsive polymer, such as a stimulus-responsive polymer described herein) and a second layer comprising a barrier and reservoir, wherein the condom does not contact the shaft or corona of the penis; wherein the adhesive layer is (a) coextensive with the barrier layer; (b) thicker than the barrier layer and/or (c) the only securing means. Optionally, the second layer is continuous, i.e., the barrier and the reservoir are formed together, i.e., in one piece. Optionally, the reservoir is self-forming. Optionally, the fractional condom consists of only three layers, inclusive of a backing layer. Optionally, the condom comprises one or more additional means of securing the condom to the penis. The condom may exhibit one or more properties disclosed herein, such as pain-free removal, low peel strength at low peel rate, high peel strength at high peel rate, loss modulus, etc.

### 4.2.3. Additives

In some embodiments, the adhesive further comprises one or more additives. Exemplary additives include tackifiers, plasticizers, pigments, fillers, fluorescents, flow agents, wetting agents, surfactants, anti-foaming agents, rheology modifiers, colorants, permeation enhancers, stabilizers, antioxidants, and combinations thereof. In some embodiments, adhesion may be enhanced or decreased through the addition of an additive.

Exemplary plasticizers include, are not limited to, Glyceryl triacetate (triacetin), Glyceryl monooleate (GMO), Glyceryl monostearate (GMS), Glyceryl tristearate (tristearin), Glyceryl tributyrate (tributyrin), Glyceryl tripropionate (triproprionin), Glyceryl trioleate (triolein), Glyceryl dilaurate (GDL), Glyceryl dimyristate (GDM), Glyceryl distearate (GDS), Diethylhexyl phthalate (DEHP), Diisononyl phthalate (DINP), Dibutyl phthalate (DBP), Diisodecyl phthalate (DIDP), Butyl benzyl phthalate (BBP), Dimethyl phthalate (DMP), Di-n-octyl phthalate (DnOP), Diisobutyl phthalate (DIBP), Diethyl phthalate (DEP), Dicyclohexyl phthalate (DCHP), Methyl decanoate, Ethyl decanoate, Propyl decanoate, Isopropyl decanoate, Butyl decanoate, Isobutyl decanoate, Pentyl decanoate, Hexyl decanoate, Heptyl decanoate, Octyl decanoate, Decyl decanoate, and C1-C20 anoates on either side of ester.

In some embodiments, a plasticizer may be added to lower glass transition of a polymer to tune adhesive regime. Plasticizers suitable for use include glycerol, 1,-butanol 1-octanol, stearic acid, n-butyl stearate, poly(ethylene glycol), Mw varying from 100 to 200 to 400 to 1000 to 2000 to 4000 to 10000 daltons or more, water, various organic solvents, 1-decanoate, and 1-octanoate.

In some embodiments, the additives are stimuli-responsive. For example, an additive such as poly(ethylene glycol) Mw 400 daltons or glycerol could be used in blending ratios from 1 wt% 90 wt% with an adhesive layer constituent such as poly(n-dimethyl acylamide), such that the additive plasticizes an adhesive layer above the crystallization temperature of the additive and no longer plasticizes the adhesive layer below the crystallization temperature of the additive.

In some embodiments, stimuli-responsive additives may exhibit crystallization, glass transition, or other thermal transition in the range of 0 °C to 50 °C, more particularly 5 °C to 40 °C, more particularly 10 °C to 30 °C, and further particularly 12 °C to 25 °C.

Exemplary tackifiers include, but are not limited to, Rosin esters, Hydrocarbon resins, Terpene resins, Styrene resins, Polyterpene resins, Coumarone-indene resins, Phenolic resins, Tall oil rosin, Aliphatic resins, and Aromatic resins.

Exemplary tackifying resins or tackifiers include low-molecular weight compounds with high glass transition temperature used in formulating adhesives to increase the tack, the stickiness of the surface of the adhesive. In some embodiments, tackifiers include resins (e.g., rosins and their derivates, terpenes and modified terpenes, aliphatic, cycloaliphatic and aromatic resins (C5 aliphatic resins, C9 aromatic resins, and C5/C9 aliphatic/aromatic resins), hydrogenated hydrocarbon resins, and their mixtures, terpene-phenol resins (TPR, used often with ethylene-vinyl acetate adhesives)), novolacs. Silicone rubber-based pressure-sensitive adhesives suitable for use in the present disclosure include special tackifiers based on "MQ" silicate resins, composed of a monofunctional trimethyl silane ("M") reacted with quadrafunctional silicon tetrachloride ("Q").

In some embodiments, the tackifier or plasticizer is selected from glyceryl, phthalates, polyethylene glycol derivatives with molecular weights ranging from 1 to 1000 or more repeat units, and C10-C40 linear or branched wax or modified wax constituents, such as n-butyl stearate or ethyl decanoate.

Exemplary fillers include, but are not limited to, Calcium carbonate, Talc, Silica, Glass fibers, Carbon black, Barium sulfate, Kaolin, Mica, Wollastonite, Alumina, Titanium dioxide, Cellulose, Wood flour, Fly ash, and Graphite.

Exemplary water or solvent chemically swellable particulate additives include, but are not limited to, poly(sodium acrylate) molecular weight ranging from 100 to 5 million daltons, more particularly 100 to 1 million daltons, more particularly 1000 to 1 million daltons, with particle sizes ranging from 1 micron to 1000 microns, more particularly 20 microns to 800 microns, more particularly 20 to 300 microns, in concentrations ranging from 0.01 to 95 wt,%, more particularly 0.1 to 75 wt%, more particularly 1 to 60 wt %, more particularly 2 to 55 wt%.

In some embodiments, particulate additives may decrease diffusion of water or other solvents into adhesive layer and may be hydrophobic constituents such as steric acid, hydrophobic fumed silica or polyethylene waxes molecular weight ranging from 100 to 5 million daltons, more particularly 100 to 1 million daltons, more particularly 200 to 1 million daltons, with particle sizes ranging from 1 micron to 1000 microns, more particularly 20 microns to 800 microns, more particularly 20 to 300 microns, in concentrations ranging from 0.01 to 95 wt,%, more particularly 0.1 to 75 wt%, more particularly 1 to 60 wt %, more particularly 2 to 55 wt%.

In some embodiments, particulate additives may create physical sites for increased or reduced adhesion to skin and may be stimuli-responsive in nature. Constituents that create physical sites for enhanced or reduced adhesion to skin may be ceramic additives such as fumed silica, zinc oxide or titanium dioxide or may be polymers that have molecular weight ranging from 100 to 5 million daltons, more particularly 100 to 1 million daltons, more particularly 200 to 1 million daltons, with particle sizes ranging from 1 micron to 1000 microns, more particularly 20 microns to 800 microns, more particularly 20 to 300 microns, in concentrations ranging from 0.01 to 95 wt,%, more particularly 0.1 to 75 wt%, more particularly 1 to 60 wt %, more particularly 2 to 55 wt%.

Additives used herein may include nucleating agents for stimuli- responsive adhesives undergo change in adhesive behavior upon crystallization by cooling. The addition of nucleating agents such as nano-scale fumed silica and polyethylene waxes could be used to tune crystallization temperature of adhesives that exhibit crystalline transitions such as poly(octadecyl methacrylate). Nucleation-inducing additives include additives ranging in particle size from 1 nm to 1000 microns, more particularly 10 nm to 500 microns, more particularly 10 nm to 250 microns. Nucleation-inducing additives may be blended with adhesive in solution blending, high-shear mixing or other blending technique or may be generated in situ during adhesive preparation or diaphragm formation through techniques that include precipitation or phase separation. For example, stearic acid could be mixed with adhesive solutions under high shear conditions to form nanophases that remain dispersed in adhesive blends such as poly(stearyl) methacrylate.

In some embodiments, additives may serve as crack propagating agents to facilitate adhesive failure upon removal via mechanical peeling.

In some embodiments, additives are present in the adhesive in an amount from 0 to 60 wt%, more particularly from 0.1 wt% to 50 wt%, from 0.1 wt% to 40 wt%, from 0.1 wt% to 30 wt%, from 0.1 wt% to 20 wt%, from 0.1 wt% to 10 wt%, from 0.1 wt% to 5 wt%, from 0.1 wt% to 2.5 wt%, from 0.1 wt% to 1 wt%.

In some embodiments, the one or more additives is present in an amount of about 1, about 2, about 3, about 4, about 5, about 6, about 7 about 8, about 9, about 10, about 11, about 12, about 13, about 14, or about 15 wt.% or more of the adhesive.

In some embodiments, the one or more additives are present in an amount of about 20 wt%, about 25 wt%, about 30 wt%, about 35 wt%, about 40 wt%, about 45 wt%, about 50 wt%, about 55 wt%, or about 60 wt% of the adhesive.

In some embodiments, the one or more additives is present in an amount of about 0.1 wt%, about .2 wt%, about 0.3 wt%, about 0.4 wt%, about 0.5 wt%, about 0.6 wt%, about 0.7 wt%, about 0.8 wt%, or about 0.9 wt% or more of the adhesive.

In some embodiments, the one or more additives is present in an amount of about 0.01 wt%, about .02 wt%, about 0.03 wt%, about 0.04 wt%, about 0.05 wt%, about 0.06 wt%, about 0.07 wt%, about 0.08 wt%, or about 0.09 wt% or more of the adhesive.

### 4.2.4. Method of making the stimuli-responsive polymer

In some embodiments, the stimuli-responsive polymer is prepared by curing one or more monomers and a first amount of one or more polyfunctional crosslinkers to form a prepolymer; and post-curing the prepolymer; thereby providing the stimuli-responsive polymer.

In some embodiments, the weight ratio of the one or more monomers to the one or more polyfunctional crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, from 99.7:0.3 to 99.9:0.1, from 99.8:0.2 to 99.9:0.1

In some embodiments, the curing is a photopolymerization utilizing photoinitiators. Exemplary photoinitiators include, but are not limited to, includude 2,2-dimethoxy-2-phenylacetophenone (DMPA), Eosin y, diphenyl(2,4,6- trimethylbenzoyl)phosphine oxide (TPO), lithium phenyl-2,4,6-trimethylbenzoylphosphinate (LAP), and biocompatible photoinitiators.

In some embodiments, curing is done neat, i.e., in the absence of solvent.

In some embodiments, the post-curing comprises subjecting the prepolymer to an elevated temperature relative to the curing, e.g., at least 50°C, at least 100°C, at least 150 °C, or at least 200 °C.

In some embodiments, the method further comprises pausing curing prior to completion, adding a second amount of one or more polyfunctional crosslinkers, and restarting curing.

In some embodiments, polymers with C6 to C18 side chains or dangling chain ends are prepared from acrylate, methacrylate, alcohol, carboxylic acid, electron rich alkene, electron poor alkene, epoxy, amine, ROMP, Diels-Alder, Lactone, Lactam, Peptide, Peptoid, acrylamide, methacrylamide, thiol, vinyl and allyl monomers. In some embodiments, light crosslinking such as the netpoint concentration afforded by a range including but not limited to 0.4wt%, 0.6 wt%, 0.75 wt%, 0.95 wt% trimethylolpropane triacrylate in a poly(lauryl) or poly(stearyl methacrlate polymer (including but not limited to 99.6 wt%, 99.4 wt%, 99.25 wt%, 99,05 wt%) lauryl or stearyl methacrylate) affords a shear rate responsive polymer that is tacky to human skin and that subsequently can be removed from human skin with minimal pain, and residual adhesive that remains on human skin can be removed by light rubbing with minimal pain.

In some embodiments, these long side chain/low crosslink density polymers can be prepared by any monomers, crosslinkers or other constituents described herein by any reaction process described herein. For example, linear or branched poly(ethyeneimine, PEI) can be modified on side chain and chain ends using lauryl or octadecyl acrylate under base catalyzed conditions via Michael addition or lauryl or octadecyl isocyanate using isocyanate/amine reaction. In another embodiment, off or on stoichiometric thiolenes can be prepared using combinations of mono, di, tri and tetrafunctional thiol and alkene monomers with monofunctional constituent such as lauryl mercaptopropionate or dodecyl vinyl ether such that monofunctional constituent comprises 0.1, 0.2, 0.3, 0.4 or 0.5 or more mole % of overall thiolene constituency. In another representative embodiment, an alternating copolymer comprising maleimide or n-butyl maleimide and dodecyl vinyl ether is prepared using radical alternating polymerization and can be lightly crosslinked by dodecyl vinyl ether with less than wt% or a polymethacrylate or acrylate to form heterogeneous crosslinked networks with low crosslink density and high C12 side chain wt % (60, 70, 80, 90 or more wt% alkyl side chain C6 or greater with C12-C18 preferred). In another embodiment, octadecyl amine is polymerize red with a Michael addition co-monomer such as ethylene glycol diacrylate or hexanediol diacrylate under base catalyzed conditions. In one embodiment, the resulting poly(beta aminoester) is prepared with 1:25 : 1.0 C=C : NH2 (double NH2 reaction with acrylate) reaction such that acrylate end capped groups result, and acrylate endcapped poly(betamino ester) reaction products can be photocured using UV light, and crosslink density can be reduce by the addition of monofunctional acrylates or methacrylates such as stearyl or lauryl methacrylate or acrylate or by adding thiol chain transfer or capping agents such as PETMP or IOMP or EGBMP or 1,10- decanediothiol or PETMP. In another embodiment, a similar Michael addition synthetic process can be used for thiol/acrylate Michael addition reaction products, with excess acrylate preferred.

In another embodiment, the adhesive is a liner or branched crosslinked polymer network wherein the crosslinked polymer network contains 10 or more ester to thiolester linkages which hydrolyze in the presence of an added base or a compound containing a thiol, or a compound containing an amino wherein hydrolysis results in dissolution of the adhesive and delamination from penile skin.

In another embodiment, adhesive layer may be a liner or branched polymer comprising the reaction product by free radical addition polymerization of mono-, di- try- tetra-, penta, and hexafunctional thiol-ene constituents including triallyl isocyanurate pentaerythritol tetrakis (3-mercaptopropionate) or any of the thiol-ene monomeric constituents disclosed herein. One embodiment, thiol-ene adhesive may exhibit stimuli-responsive adhesive behavior upon cooling below its glass transition. In another embodiment, thiol-ene adhesive layer may exhibit chemically-responsive adhesive behavior such as oxidation of thioether linkages by common oxidizing agents such as hydrogen peroxide to form reversibly clearable disulfide linkages.

### 4.3. Package

In one aspect, the present disclosure provides packages comprising a contraceptive device described herein.

In some embodiments, a package comprises a condom described herein. In some embodiments, a package comprises a diaphragm described herein.

In some embodiments, a package may be rigid, semi-rigid, flexible, or combination thereof in which the contraceptive device is placed in until used, wherein the package may or may not have additional uses beyond storage as described by herein. In some embodiments, the package may be 0.005 mm or thinner, 0.005 mm or greater, 0.01 mm or greater, 0.02 mm or greater, 0.03 mm or greater, 0.04 mm or greater, or 0.05 mm or greater. The package may be approximately rectangular, circular, elliptical polygonal, or curvilinear polygonal in shape.

In some embodiments, the package comprises a flexible wrapper comprising foil, plastic, plastic-lined paper, foil-lined paper, or a combination thereof.

In some embodiments, package is a blister-pack design. In some embodiments, the blister-pack design package comprises a semi-rigid plastic or paper or cardboard well covered by a thin metallic, paper or plastic film, which may be punctured by pressing on the bottom of the rigid well. Semi-rigid is defined to be a material and thickness that maintains its designed geometry, but may be deformed by exerting a force via of between 1 and 5 N (Newtons) or between 5 and 10 N or between 10 and 20 N, or between 20 and 100 N, or between 100 and 150 N, or between 150 and 200 N, or between 200 and 300 N. The deformation may be an elastic bending deformation, or a buckling deformation or a creasing deformation. The well may be cylindrical, conical, spherical, a body of revolution, polyhedral, irregular or non-symmetric in shape. The well may have a circular, rectangular, elliptical, polygonal or curvilinear polygonal cross section.

In some embodiments, the package is a well design. In some embodiments, the well design package comprises rigid plastic, paper, or cardboard construction. In some embodiments, a face or faces of the container are sealed via a removable plastic film or metal foil film. In some embodiments, a tab may protrude from the film, enabling it to be peeled away by finger-strength or another means of gripping the tab. In some embodiments, the sealing film may be punctured to expose the contraceptive device within.

In some embodiments, the package is used to contain and discard the contraceptive device after use. In some embodiments, the package contains any ejaculate that may have been deposited within or on the condom.

In some embodiments, the package further comprises a delaminating composition suitable to induce delamination of the adhesive layer from penis or vagina when the adhesive layer has been adhered to the penis or vagina. In some embodiments, the package further comprises a delaminating composition suitable to induce delamination of the adhesive layer from the glans of the penis when the adhesive layer has been adhered to the glans of the penis.

In some embodiments, the delaminating composition is a wipe. In some embodiments, the wipe comprises a solvent that dissolves, denatures, or swells the stimuli-responsive polymer, thereby inducing delamination of the adhesive layer from the penis or vagina when the adhesive layer has been adhered to the penis or vagina and the wipe subsequently applied to the contraceptive device. In some embodiments, the wipe comprises a solvent that dissolves, denatures, or swells the stimuli-responsive polymer, thereby inducing delamination of the adhesive layer from the glans of the penis when the adhesive layer has been adhered to the glans of the penis and the wipe subsequently applied to the condom.

In some embodiments, the wipe comprises a volatile additive that cools the wipe upon evaporation, thereby inducing delamination of the adhesive layer from the penis or vagina when the adhesive layer has been adhered to the penis or vagina and the wipe subsequently applied to the contraceptive device. In some embodiments, the wipe comprises a volatile additive that cools the wipe upon evaporation, thereby inducing delamination of the adhesive layer from the glans of the penis when the adhesive layer has been adhered to the glans of the penis and the wipe subsequently applied to the condom.

In some embodiments, the package may further comprise a lubricant, spermicide, or both.

### 4.4. Kits

In one aspect, the present disclosure provides kits comprising a contraceptive device described herein and instructions for use.

In some embodiments, a kit comprises a condom described herein and instructions for use. In some embodiments, a kit comprises a packaged condom described herein and instructions for use.

In some embodiments, a kit comprises a fractional condom described herein and instructions for use. In some embodiments, a kit comprises a packaged fractional condom described herein and instructions for use.

In some embodiments, a kit comprises a diaphragm described herein and instructions for use. In some embodiments, a kit comprises a packaged diaphragm described herein and instructions for use.

In some embodiments, the kit may comprise a lubricant, spermicide or both.

### 4.5. Methods of Use

In one aspect, the present disclosure provides methods of applying a condom described herein to the penis of a human subject comprising contacting the adhesive layer of a condom described herein to the penis and applying pressure to the condom sufficient to adhere the condom to penis. In some embodiments, pressure is applied with one or more fingers or the hand of the subject.

In some embodiments, a method of applying a fractional condom described herein to the penis of a human subject comprises contacting the adhesive layer of the condom to the glans of the penis and applying pressure to the condom sufficient to adhere the condom to the glans of the penis. In some embodiments, pressure is applied with one or more fingers or the hand of the subject.

In some embodiments, the condom or fractional condom disclosed herein is applied prior to sexual arousal or prior to full sexual arousal.

In some embodiments, a method of applying a diaphragm described herein to the vagina of a human subject comprises contacting the adhesive layer of the diaphragm to the vagina and applying pressure to the diaphragm sufficient to adhere the diaphragm to the vagina. In some embodiments, pressure is applied with one or more fingers or the hand of the subject.

In some embodiments, adhesion to skin may be achieved through a hot melt process through oral fusion or through other external heat such as that provided by a hair dryer or by compression in a human hand for about 0 to about 20 seconds in which the adhesive exhibits increased adhesive force upon heating to about 30 °C, about 35 °C, about 37 °C, about 40 °C, about 45 °C, about 55 °C, about 60 °C or about 65 °C or greater.

In some embodiments, application of the fractional condom disclosed herein to the penis of a human subject is easier than application of a fractional condom known in the art, including, but not limited to, the GALATIC CAP^{™}.

In some embodiments, application of the fractional condom disclosed herein is easier than application of a fractional condom known in the art, e.g., the GALATIC CAP^{™}.

In some embodiments, application of the fractional condom disclosed herein is easier than application of a fractional condom known in the art (e.g., GALATIC CAP^{™}) by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% or more, as measured by any suitable method (e.g., user-self reporting in a coital log) in a non-clinical or clinical setting.

In certain embodiments, fewer than 30% of users find the condom difficult or very difficult to apply and more particularly, fewer than 25%, fewer than 20%, fewer than 15%, fewer than 10% or fewer than 5%,

In some embodiments, the fractional condom disclosed herein remains adhered to the penis of the human subject in a manner superior to other fractional condoms known in the art, including, but not limited to, the condoms disclosed in WO2014178661A and US 11,234,858. Adherence can be measured by any suitable method (e.g., user-self reporting in a coital log) in a non-clinical or clinical setting.

In some embodiments, use of the condom disclosed herein enhances adherence in comparison to other fractional condoms known in the art by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% or more.

In some embodiments, the use of the condom disclosed herein enhances sexual pleasure of the user and/or the partner by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% or more, as measured by any suitable method (e.g., user self-reporting in a coital log by user, partner or both) in a non-clinical or clinical setting. The comparison may be with respect to a conventional condom or a fractional condom.

In some embodiments, the condom disclosed herein enhances sexual pleasure of the user and/or the partner by about 2X, about 3X, about 4X, about 5X, about 6X, about 7X, about 8X, about 9X or about 10X or more, as measured by self-reporting of an individual(s) (e.g., using a coital log) or more formal studies, including comparative population studies.

Sexual pleasure may be measured by any suitable method. See e.g., Siegler AJ, et al. Arch Sex Behav. 2018 Aug;47(6):1745-1754, which is specifically incorporated herein. In one embodiment, sexual pleasure is measured by an event-level, male sexual pleasure scale such as EMSEXpleasure. In another embodiment, sexual pleasure is measured using the Quality of Sexual Experience (QSE) scale. S. Sanders, et al., J Sex Med. 2013 Oct;10(10):2409-17), incorporated herein by reference. Both are reliable event-level measure of the quality of a sexual experience, the latter for both men and women.

In other embodiments, sexual pleasure may be assessed by the Sexual Pleasure Scale (SPS) ( Patricia M Pascoal et al. (2016) The Journal of Sexual Medicine, 13(9), 1408-1413), the Body, Emotions, Sensations, Touch/Trust (B.E.S.T.) Scale (Beckmeyer et al. (2021) Journal of American College Health, 1-12) or the Pleasuremeter (Castellanos-Usigli and Braeken-van Schaik (2019). Sexual and reproductive health matters, 27(1), 313-315).

In some embodiments, the use of the condom disclosed herein enhances sexual sensation of the user and/or the partner by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% or more, as measured by any suitable method in a non-clinical or clinical setting. In one embodiment, the enhanced sexual pleasure is measured by any suitable method in a non-clinical setting, e.g., by individual self-reporting (e.g., via a coital log) or a clinical setting. The comparison may be with respect to a conventional condom or a fractional condom.

In some embodiments, the condom disclosed herein enhances sexual sensation of the user and/or the partner by about 2X, about 3X, about 4X, about 5X, about 6X, about 7X, about 8X, about 9X or about 10X or more, as measured by any suitable method (e.g., user self-reporting in a coital log) in a non-clinical setting, e.g., by individual self-reporting (e.g., via a coital log) or a clinical setting. The comparison may be with respect to a conventional condom or a fractional condom.

In some embodiments, use of the condom disclosed herein is preferred over a conventional condom by at least a majority population of users (either actual users and/or partners) to a conventional condom, wherein the majority constitutes about 80%, about 85%, about 90% or about 95% or more of the population. The users may be a non-clinical or clinical population of users.

In some embodiments, the use of the condom disclosed herein is preferred over a conventional condom by about 85% to 100% of users.

In embodiment, at least 175 out of 200 users in a population prefer the condom disclosed herein to a conventional condom.

In some embodiments, use of the condom disclosed herein results in an increase in condom usage among a population of users in a non-clinical or clinical setting.

In one embodiment, the population is a group of human subjects in a clinical trial.

In another aspect, the present disclosure provides methods of removing a condom described herein from the penis of a human subject comprising applying a stimulus to the condom whose adhesive layer is adhered to the penis and removing the condom from the penis.

In some embodiments, a method of removing a fractional condom described herein from the glans of the penis of a human subject comprises applying a stimulus to the condom whose adhesive layer is adhered to the glans of the penis and removing the condom from the glans of the penis.

In some embodiments, a method of removing a diaphragm described herein from the vagina of a human subject comprises applying a stimulus to the diaphragm, whose adhesive layer is adhered to the vagina and removing the diaphragm from the vagina.

In some embodiments, the condom disclosed herein is easier to remove than a fractional condom known in the art.

In some embodiments, the ease of removal is improved by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% or more, as measured in non-clinical or clinical settings by any suitable method (e.g., individual self-reporting in a coital log),

In some embodiments, fewer than 30% of users find the condom difficult or very difficult to remove and more particularly, fewer than 25%, fewer than 20%, fewer than 15%, fewer than 10% or fewer than 5%,

In some embodiments, the removal of the contraceptive device is injury-free.

In some embodiments, removal of the contraceptive device causes minimal or no pain in the subject as measured by the WBQPA, e.g., a WBQPA score of less than 4, less than 3, less than 2, less than 1, or 0.

In some embodiments, removal of the contraceptive devices causes no significant irritation, inflammation, or redness in the user.

In some embodiments, when the contraceptive device is removed from the penis or vagina, 50 wt% or less of the adhesive remains on the penis or vagina, such as, for example, 40 wt% or less, 30 wt% or less, 20 wt% or less, 10 wt% or less, or 5 wt% or less. In some embodiments, even if adhesive remains on the penis or vagina after removal, the properties of the adhesive are such that it is easily removed by light rubbing or rolling.

In some embodiments, the stimulus is mechanical action or force. In some embodiments, the mechanical action is shear rate. In some embodiments, the shear rate is induced by peeling, pulling, or rubbing at varying rates.

In some embodiments, the peeling is light peeling as described hereinabove. In some embodiments, light peeling corresponds to a peel rate of 25 mm/s or less, such as, for example, 10 mm/s or less, 5 mm/s or less, 1 mm/s or less. 0.5 mm/s or less, 0.3 mm/s or less, or 0.1 mm/s or less. In some embodiments, light peeling corresponds to a peel rate from 0.01 mm/s to 25 mm/s, such as, for example, from 0.01 mm/s to 10 mm/s, from 0.01 mm/s to 5 mm/s, from 0.01 mm/s to 1 mm/s, from 0.01 mms/ to 0.5 mm/s, from 0.01 mm/s to 0.3 mm/s, from 0.01 mm/s to 0.1 mm/sec, from 0.1 mm/s to 25 mm/s, from 0.1 mm/s to 10 mm/s, from 0.1 mm/s to 5 mm/s, from 0.1 mm/s to 1 mm/s, from 0.1 mms/ to 0.5 mm/s, from 0.1 mm/s to 0.3 mm/s, from 1 mm/s to 25 mm/s, from 1 mm/s to 10 mm/s, or from 1 mm/s to 5 mm/s. In some embodiments, light peeling corresponds to a peel rate of 500 mm/min or less, such as, for example, 400 mm/min or less, 300 mm/min or less, 200 mm/min or less, 100 mm/min or less, or 50 mm/min or less. In some embodiments, light peeling corresponds to a peel rate of 50 mm/min to 500 mm/min, such as, for example, from 50 mm/min to 400 mm/min, from 50 mm/min to 300 mm/min, from 50 mm/min to 200 mm/min, from 50 mm/min to 100 mm/min, from 100 mm/min to 500 mm/min, from 100 mm/min to 400 mm/min, from 100 mm/min to 300 mm/min, from 100 mm/min to 200 mm/min, from 200 mm/min to 500 mm/min, from 200 mm/min to 400 mm/min, from 200 mm/min to 300 mm/min, from 300 mm/min to 500 mm/min, from 300 mm/min to 400 mm/min, or from 400 mm/min to 500 mm/min.

In some embodiments, the stimulus is temperature change, and application of a stimulus comprises cooling the temperature of the contraceptive device to 25 °C or lower. In some embodiments, the stimulus is temperature change, and application of a stimulus comprises cooling the temperature of the condom to 25 °C or lower.

In some embodiments, the stimulus is physico-chemical change, and application of a stimulus comprises applying a wipe to the contraceptive device, wherein the wipe comprises a solvent that dissolves, denatures, or swells the stimuli-responsive polymer, thereby inducing delamination of the adhesive layer from the penis or vagina. In some embodiments, the stimulus is physico-chemical change, and application of a stimulus comprises applying a wipe to the condom, wherein the wipe comprises a solvent that dissolves, denatures, or swells the stimuli-responsive polymer, thereby inducing delamination of the adhesive layer from the glans of the penis.

In some embodiments, the condom disclosed herein is removed without application of a liquid, such as baby oil.

In some embodiments herein, the condom is removed without leaving the site of sexual intercourse.

In some embodiments, the condom is removed without the user having to urinate.

### 4.6. Methods of Manufacture

In one aspect, the present disclosure provides methods of preparing the contraceptive devices described herein comprising adhering an adhesive layer to a barrier layer, wherein the adhesive layer comprises an adhesive that comprises a stimuli-responsive polymer formed from one or more monomers and optionally one or more polyfunctional crosslinkers.

In some embodiments, a method of preparing a condom comprises adhering an adhesive layer to a barrier layer, wherein the adhesive layer comprises an adhesive that comprises a stimuli-responsive polymer formed from one or more monomers and optionally one or more polyfunctional crosslinkers.

In some embodiments, a method of preparing a diaphragm comprises adhering an adhesive layer to a barrier layer, wherein the adhesive layer comprises an adhesive that comprises a stimuli-responsive polymer formed from one or more monomers and optionally one or more polyfunctional crosslinkers.

In some embodiments, a method of manufacturing a contraceptive device described herein is provided comprising (i) providing an adhesive composition described herein and (ii) applying the adhesive to a substrate using one of the following methods: screen printing, pad printing, roll to roll coating, dip coating, thermoforming, extrusion, injection molding or other scaled methods of film manufacturing, thereby providing the contraceptive device disclosed herein. In some embodiments, the substrate is a release liner. In some embodiments, the substrate is a barrier layer.

In some embodiments, the contraceptive device comprises an adhesive layer that is cured using photopolymerization. In some embodiments, the adhesive is cured using UV or visible light after application as a coating onto a barrier substrate. In some embodiments, the adhesive is fully or partially UV or visible light cured and then applied to a substrate.

In some embodiments, the adhesive is crosslinked after processing onto a release liner or barrier layer by latent reaction using the aforementioned crosslinking chemistries or others. For example, residual epoxide and alcohol or amine chemistries could be used to achieve crosslinking after dip coating an adhesive onto a release liner or barrier layer or applying via spray or in a roll-to-roll coating method.

In some embodiments, the adhesive is self-healing, in that it can be prepared separately from a barrier layer, applied to a barrier layer in an additional step and optionally be made to undergo regenerative adhesive capability after removal from skin or other application surface. An example of a transfer process to apply a separately prepared adhesive layer to a barrier layer is pad printing. Another example is the adhesive may be prepared separately from a barrier layer and extruded through an orifice or nozzle and transferred to a barrier layer. Another example is the adhesive may be prepared on a form or mould separately from the barrier, and the barrier transferred to the adhesive a solid layer or a liquid deposited via dip coating, spray, brush, painting or rolling transfer.

In some embodiments, the contraceptive device is subjected to one or more quality testing steps. In some embodiments, the contraceptive device is subjected to a water-leakage test or electric test (e.g., a dry electric test).

In some embodiments, the contraceptive device is subjected to one or more tests of its physical properties, including, but not limited to, tensile strength, tensile or overlap shear strength, peel adhesion strength, and/or impact strength, and provides satisfactory results.

In some embodiments, the contraceptive device is subjected to one or more tests selected from: ASTM F2255-05 Standard Test Method for Strength Properties of Tissue Adhesives in Lap-Shear by Tension Loading; ASTM F2256-05 Standard Test Method for Strength Properties of Tissue Adhesives in T- Peel by Tension Loading; and ASTM F2258-05 Standard Test Method for Strength Properties of Tissue Adhesives in Tension and provides satisfy factory results.

### 4.7. Examples (not according to the claims)

Below are examples of specific embodiments for carrying out the present disclosure. The examples are offered for illustrative purposes only, and are not intended to limit the scope of the present disclosure in any way. Efforts have been made to ensure accuracy with respect to numbers used (e.g., amounts, temperatures, etc.), but some experimental error and deviation should, of course, be allowed for.

### 4.7.1. Example 1

Poly(ethyl acrylate), average Mw ~95 kDa by GPC, solids 18-22 wt% toluene solution was purchased from Sigma Aldrich and cast onto a polyethylene plastic substrate approximately 0.1 mm in thickness as measured by calipers. Toluene solvent was allowed to evaporate in a fume hood for approximately 72 hours. After solvent evaporation, a polymer film approximately 50 to 500 microns in thickness remained on the polyethylene substrate, with sections of the polymer film measured by calipers to be approximately 200 and 400 microns in thickness, respectively. This polymer film was sandwiched with a second polyethylene 0.3 mm layer and stored for 6 months at approximately 20 C in approximately 45%- 60% relative humidity. After 6 months, the poly(ethyl acrylate) film easily remained adhered to the 0.3 mm thick polyethylene film on which it was cast. To demonstrate thermally responsive delamination from human skin, the polyethylene/poly(ethyl acrylate) substrate was pressed onto a bare human arm, and adhesive behavior was demonstrated and recorded via video and shown to be strong enough to substantially limit facile removal of polyethylene/poly(ethyl acrylate) substrate from the human arm.

The adhered polyethylene/poly(ethyl acrylate) substrate attached to a human arm was run under room temperature water (approximately 20 °C) from a kitchen sink for approximately 5-10 seconds while also being subjected to light pulling/peeling hand forces, and delamination was achieved without pain or substantial applied hand force after 5-10 seconds with no observable adhesive residue on arm.

### 4.7.2. Example 2

Poly(ethyl acrylate), average Mw ~95 kDa by GPC, solids 18-22 wt% toluene solution was purchased from Sigma Aldrich and cast onto commercially available 6" by 6" approximately 0.2 mm thick dental dam latex and polyurethane substrates. The dental dam was measured by calipers to be approximately 0.20 mm. Toluene solvent was allowed to evaporate in ambient air for 72 hours in approximately 45%- 60% relative humidity. After solvent evaporation, a polymer film approximately 50 to 500 microns in thickness remained on the dental dam substrates, with sections of the polymer film measured by calipers to be approximately 200 and 400 microns in thickness, respectively.

Unlike the poly(ethyl acrylate) film that was stored for 6 months reported in Example 1, the poly(ethyl acrylate) film in Example 2 exhibited limited tack at room temperature (approximately 19 °C) when assessed for qualitative skin adhesion 72 h after casting onto latex and polyurethane dental dams.

To demonstrate thermally responsive delamination from human skin, a latex dental dam/poly(ethyl acrylate) substrate with a circular adhesive layer approximately 50 to 500 microns thick (with sections measured by calipers being approximately 200 and 400 microns thick, respectively) and approximately 4 cm in diameter as measured by calipers was pressed onto an erect human penis, and was heated using a hair dryer on a warm heat setting for approximately 5 seconds while pressing onto the penis head, and adhesive behavior was observed immediately after 5 second hair dryer exposure (oral fusion is an alternative technique to a hairdryer to heat a partially tacky adhesive in a hot melt process into a more ideal thermomechanical regime to achieve adhesion in one embodiment of the present disclosure). A rubber band was placed around the base of the tip of the penis to secure the non-adhesive section of the dental dam substrate and prevent any shear removal/stress concentrations from forming at non- adhered sites, and the erect penis was subjected to forces consistent with those observed during sexual intercourse using masturbatory techniques. Upon ejaculation, no semen was observed to leave the condom that was adhered to the head of the penis even after removal of the rubber band placed below the head of the penis. The adhered latex dental dam was then subjected to cool water (approximately 10-20 °C) in a common shower and lightly removed by peeling along the edges. Delamination with minimal or no pain in penile skin was observed while running cool water along the adhered dental dam/flowing water interface in approximately 10-20 seconds, and no adhesive residue was observed on penile skin or inside the urethra. Urination was easily achievable immediately after the removal of the adhered dental dam.

Example 2 was repeated and similar results were observed. When the methods of Example 2 were repeated, a paper towel was run across the base of the head of the penis, and no semen was observed on the paper towel. In example 2, a flaccid penis with adhered condom was dipped in ice water, and the condom was easily removed with no observable adhesive residue on the penis.

### 4.7.3. Example 3

Approximately 10 mL of Poly(ethyl acrylate), average Mw ~95 kDa by GPC, solids 18-22 wt% toluene solution was added to a stainless steel mixing container. Approximately 1 mL of poly(butyl acrylate), average Mw 99 kDa by GPC, solids 25-30 wt%, was added to the same stainless steel mixing container and stirred using a polyethylene pipette for approximately 20 seconds. This Poly(ethyl acrylate)/poly(butyl acrylate mixture appeared homogeneous and optically transparent after stirring for approximately 20 seconds no visible signs of phase separation or precipitation. Both poly(ethyl acrylate) and poly(butyl acrylate) solutions were purchased from Sigma Aldrich and used as received. After mixing, approximately 3 mL of blended poly(ethyl acrylate)/poly(butyl acrylate) solutions were drawn into a 3 mL polyethylene pipette and dropped onto 6" x 6" latex dental dams in triplicate. Dental dams were approximately 200 microns in thickness as measured by calipers. Dental dam/adhesive solutions were allowed to evaporate for approximately 72 hours in ambient air conditions with approximately 40%-60% relative humidity at temperatures ranging from 15 C to 25 C. After solvent evaporation, a polymer blend film approximately 50 to 500 microns in thickness remained on the latex dental dam substrate, and sections of the adhesive/latex substrate were measuring using calipers such that adhesive the layer was measured to be approximately 200 and 400 microns thick, respectively. To demonstrate thermally responsive delamination from human skin, the latex/poly(ethyl acrylate)/poly(butyl acrylate substrate was pressed onto a bare human arm, and adhesive behavior was demonstrated to be strong enough to substantially limit facile removal of polyethylene/poly(ethyl acrylate)/poly(butyl acrylate substrate from the human arm. The adhered polyethylene/poly(ethyl acrylate)/poly(butyl acrylate) substrate attached to a human arm was run under cold water (approximately 5-10 C) from a refrigerator-chilled bottle while also being subjected to light pulling/peeling hand forces, and delamination was achieved without pain or substantial applied hand force after 2-5 seconds with no observable adhesive residue on arm and no observed pain in removal.

### 4.7.4. Example 4

A circular/ovular substrate was cut using common scissors from one of the triplicate latex dental dam/poly(ethyl acrylate)/poly(butyl acrylate) samples prepared using the methods of Example 3 such that an adhesive elastomeric substrate resulted for which covered approximately 100% of the latex surface after cutting. The resulting circular/ovular substrate had an approximate major diameter of 5.5 cm and a minor diameter of approximately 5 cm as measured by calipers. The resulting adhesive/latex substrate was then placed roughly symmetrically on the head of an erect human penis such that the urethra was centered on the adhesive substrate ("adhesive condom") and the edges of the adhesive condom extended to approximately 50% to 75% surface area of the erect penile head, noting that the urethra was fully covered and that the base of the frenulum was exposed. When the adhesive condom was placed on the head of the erect penis, natural folding resulted that served as semen reservoirs.

After the adhesive condom was applied to the erect penis, the condom appeared securely fixed to the head of the penis. The erect penis with its head partially covered by the adhesive condom as described above was inserted into a human vagina after a light volume of common sexual lubricant was applied to the vagina, and sexual intercourse proceeded. Approximately every 15 seconds for several minutes the erect penis with applied adhesive condom was removed from the vagina and inspected, and the adhesive condom appeared to remain in place without delamination occurring from the head of the erect penis. After several minutes of sexual intercourse, ejaculation occurred, and the ejaculation began while the adhesive condom covered erect penis was in the vagina and concluded outside the vagina so that the ability of the adhesive condom to prevent external flow of semen during and ejaculation could be evaluated. No semen was observed by during or immediately following ejaculation. To remove the semen-containing adhesive condom, the surface and edges of the condom was exposed to cold water approximately 10 °C to 15 °C that was dispensed from a common shower head, and the condom was removed with minimal or no pain slowly, section by section, by hand by pulling back the edges of the condom in the presence of cold water for approximately 15-45 seconds. To the person with the penis in Example 4, ejaculation was easily achieved, and the sexual experience was notably different from that with a traditional condom that covers the head, frenulum and shaft of the penis, and for comparison, sexual intercourse was repeated with a lubricated latex condom and ejaculation was not achievable in the same sexual position as that in which the adhesive condom was used until the traditional latex condom was removed.

### 4.7.5. Example 5

Poly(n-dimethyl acrylamide) ("pDMAA"), Mw approximately 150,000 Da, was purchased from Scientific Polymer Products, Inc. Glycerol (>99%) was purchased from Sigma Aldrich, Inc. 50/50 pDMAA/glycerol blends by weight were prepared by dissolving 4.17 g of pDMAA, and 4.17 g glycerol in a solution mixture of (4.17 g acetone purchased from a local hardware store, technical grade and 2.50 g 190 proof ethanol). Homogeneous, generally clear solutions were observed upon mixing and heating pDMAA/glycerol/acetone/ethanol solutions to approximately 50 °C in sealed glass vials for 10 min and vortexing mixtures repeatedly (heat, vortex, repeat) until clear solutions were observed. pDMAA/glycerol solutions were then cast onto 6" x 6" latex dental dams and allowed to evaporate for approximately 48 to 96 hours at ambient temperatures in a chemical fume hood. After evaporation of acetone, ethanol and whatever water impurities were present in acetone/ethanol blends, the resulting pDMAA/glycerol films were approximately 400 microns thick and generally tacky to the touch at 20 °C and significantly more tacky/adhesive at 37 °C. A 5" x 5" 50/50 pDMAA/glycerol adhesive dental dam was applied to a human forearm arm and shown to exhibit significant adhesive force on video. This adhesive could be painlessly peeled from a human forearm while running tap water at room temperature over the human arm and was observed to be completely soluble in tap water.

### 4.7.6. Example 6

Poly(octadecyl methacrylate) ("pODMA"), Mw approximately 96,000 Da, was purchased from Scientific Polymer Products, Inc. in solution (approximately 20 wt%) in toluene. pODMA solutions were cast onto 6" x 6" latex dental dams and allowed to evaporate for approximately 48 to 96 hours at ambient temperatures in a chemical fume hood. After evaporation of toluene, the resulting p(ODMA) films were approximately 300 microns thick and not tacky to the touch at 20 °C (waxy in nature) and significantly tackier/adhesive at 37 °C. A 5" x 5" p(ODMA). The adhesive dental dam was applied to a human forearm arm and shown to exhibit significant adhesive force on video. This ODMA-containing substrate could be removed from human arm using ice water approximately 0 °C, and any residual ODMA remaining on human arm could be removed using olive oil or molten candle wax.

### 4.7.7. Example 7

The methods of example 7 were repeated to prepare a polymer blend on a latex dental dam comprising 1.0 parts poly(n-butyl acrylate), 6.50 parts poly(n-ethyl acrylate) and 3.0 parts poly(octadecyl methacrylate). Poly(n-butyl acrylate), Mw ~99,000, and poly(ethyl acrylate), Mw ~95,000 solutions in toluene were purchased from Sigma Aldrich and pODMA was purchased from Scientific Polymer Products as described in Example 6. In comparison to the 100% pODMA coated adhesive dental dams reported in Example 6, the three-component dental dams of Example 7 exhibited significantly less transfer of residual poly(ODMA) to human skin during delamination at 0 °C.

### 4.7.8. Example 8

Poly(ethylene) glycol (Mw 400 da) (PEG-400) and Poly(n-dimethyl acrylamide) ("pDMAA"), Mw approximately 150,000 Da, was purchased from Scientific Polymer Products, Inc. A 50/50 wt blend of PEG-400 and pDMAA was prepared in a 50 overall wt% solution of acetone and ethanol (solution was prepared using 4 g PEG-400, 4 g pDMAA, 4 g acetone (crude, from hardware store, likely water present) and 4 g 190 proof ethanol. A latex dental dam substrate taped down to a polypropylene Tupperware dish lid was placed in a vacuum oven at ambient pressure and at 65 °C with a constant air flow into a fume hood and allowed to equilibrate to 65 °C. The PEG- 400/pDMAA 50/50 wt% solution was cast onto this 65 °C substrate for 24 h in vacuum oven under constant air flow conditions and ambient pressure. A resulting film was observed that exhibited strong adhesive behavior in the range of 45-65 °C (above the crystallization temperature of PEG- 400)_and no tack/adhesion at ambient temperature (below the crystallization temperature of PEG-400).

### 4.7.9. Example 9

A series of adhesives and barrier substrates was prepared by polymerization of monomeric species. Monomers including n-butyl acrylate, n-ethyl acrylate, n-octyl acrylate, n-hexyl acrylate, 2- hydroxyethyl methacrylate, isobornyl methacrylate, cetyl methacrylate, octadecyl methacrylate, lauryl methacrylate, acrylamide, n-isopropyl acrylamide, poly(ethylene glycol) diacrylate, Mn ~ 750, trimethylolpropane triacrylate, ethoxylated trimethyololpropane triacrylate, pentaerythritol tetraacrylate and diurethane dimethacrylate, triethylene glycol divinyl ether, n-vinyl dodecyl ether (dodecyl vinyl ether), isooctyl 3-mercaptopropionate, triallyl isocyanurate, were purchased from Sigma Aldrich and/or Scientific Polymer.

Monomers were massed out using an analytical balance and added with 1.0 wt% DMPA photoinitiator until homogeneous solutions formed. These solutions were then injected between Rain-X coated 1.0mm thick glass slides separated by 1.0 mm thick spacers and UV cured at 365 nm for 15 min in a UVP CL-1000 crosslinker, after which samples were post-cured at 90 °C under ambient conditions for approximately 10 hours. Samples, optically clear after post-curing at 90 °C, were then cooled to room temperature where they appeared optically clear to the naked eye and then placed in a freezer at -20 °C for 5 min, after which samples appeared to cloud or become opaque to varying extents. Samples could be removed from glass slides and handled. Initially room temperature samples with clouding behaved as nonstick waxy solids. Upon heating to body temperature samples became very adhesive to skin, more adhesive to polyurethane dental dams and less adhesive to latex dental dams. Samples that appeared optically clear and did not cloud after cooling behaved as gels with limited tack or adhesiveness to the polyurethane dental dams or latex dental dams.

In certain cases, samples with varying degrees of clouding exhibited the following properties: (A) waxy solids at room temperature, (B) adhesive to human skin after melting upon application to human skin and good simultaneous adhesion to latex and polyurethane condoms or dental dams, (C), frequency-responsive and/or shear responsive adhesive behavior such that melted wax adhesives maintained polyurethane and latex condom and dental dam barrier adhesion to human skin while barriers were "pulled" or "jerked" at higher forces or frequencies or shear rates such as those that would be expected to remove an adhesive barrier from human skin, while also exhibiting the ability to be removed from human skin with minimal or no pain by low frequency or low force or very soft or slow peeling. (D) For select samples, residual adhesive that remained on human skin after removal was melted and very difficult to remove, while in other cases adhesive could be very easily rubbed away with minimal pain or no pain in a very natural human manner. Videos were taken of observed behavior. Optically opaque samples that exhibited melting to human skin, including human forearm, bicep, shoulder and penis head skin and varying degrees of frequency, shear, or shear rate near-painless removal include UV cured poly(stearyl methacrylate samples) with varying crosslinkers that included poly(ethylene glycol) diacrylate, Mn 750, trimethylolpropane triacrylate, pentaerithrital tetraacrylate and ethoxylated trimethylolpropane triacrylate in crosslinker ranges that included 0 wt%, 0.2 wt%, 0. 4 wt%, 0.6 wt%, 0.75 wt%, 0.85 wt%, 0.95 wt%, 1.0 wt%, 2.0 wt%, 3.0 wt%, 5.0 wt%, 7.5 wt%, 10.0 wt%, 15.0 wt%, 20 wt%, 25 wt% and 30 wt% crosslinker prepared using 1.0 wt% DMPA photoinitiator.

Optically clear samples that exhibited strong adhesion to human skin, including human forearm, bicep, shoulder and penis head skin and varying degrees of frequency, shear, or shear rate near¬painless removal include UV cured poly(stearyl methacrylate samples) with varying crosslinkers that included poly(ethylene glycol) diacrylate, Mn 750, trimethylolpropane triacrylate, pentaerithrital tetraacrylate and ethoxylated trimethylolpropane triacrylate in crosslinker ranges that included 0 wt%, 0.2 wt%, 0. 4 wt%, 0.6 wt%, 0.75 wt%, 0.85 wt%, 0.95 wt%, 1.0 wt%, 2.0 wt%, 3.0 wt%, 5.0 wt%, 7.5 wt%, 10.0 wt%, 15.0 wt%, 20 wt%, 25 wt% and 30 wt% crosslinker prepared using 1.0 wt% DMPA photoinitiator.

Compositions that did not exhibit strong adhesion to human skin, including human forearm, bicep, shoulder and penis head skin, included poly(butyl acrylate) and poly(hexyl acrylate) crosslinked with 0.2, 0.4, 0.6, 1.0 and 2.0 or more wt% crosslinker that included poly(ethylene glycol) diacrylate, Mn 750, trimethylolpropane triacrylate, pentaerithrital tetraacrylate and ethoxylated trimethylolpropane triacrylate.

Compositions that did exhibit strong adhesion to human skin, including human forearm, bicep, shoulder and penis head skin included poly(lauryl methacrylate) and poly(octadecyl methacrylate) crosslinked with 0.4, 0.5, 0.6, 0.7 wt% trimethyolpropane triacrylate crosslinker and 1.0 wt% DMPA that were photopolymerized and post-cured at 90 C for 10 h or more. Higher concentrations of crosslinker such as 1.0 and 2.0 wt% TMPTA afforded skin-adhesive samples with less adhesive force.

### 4.7.10. Example 10

Adhesive samples were prepared by photopolymerizing a monomeric solution of 98.43 wt% lauryl methacrylate (LMA) and 0.59 wt% trimethylolpropane triacrylate (TMPTA), with 0.98 wt% 2,2-dimethoxy-2-phenylacetophenone (DMPA) photoinitiator. The monomers were massed out using an analytical balance and mixed until a homogenous solution was formed using a FlackTek SpeedMixer (DAC 330-100 PRO). Approximately 3 mL of solution was pipetted onto 25 mm disposable aluminum rheometer base plates (TA Instruments) and cured under a 365 nm UV lamp (UVP CL-1000 UV crosslinker) for 1 hour in an inert nitrogen atmosphere. The samples were then post-cured in at 120 °C for 1 hour at atmospheric pressure. The samples were then allowed to cool to ambient temperature.

A set of comparison samples were prepared using n-butyl acrylate (BA), TMPTA and DMPA. The comparison monomeric solutions had the following compositions: (a) 98.37 wt% BA, 0.55 wt% TMPTA, 1.08 wt% DMPA; (b) 98.27 wt% BA, 0.75 wt% TMPTA, 0.98 wt% DMPA; (c) 97.87 wt% BA, 1.16 wt% TMPTA, 0.98 wt% DMPA. Solutions (a-c) were injected between two Rain-X coated 1.0 mm thick glass slides which were separated by 1.0 mm of glass spacers. The samples were cured under a 365 nm UV light source (UVP CL-1000 UV crosslinker) for 15 minutes, and post-cured at 90 °C and atmospheric pressure for 12 hours, before cooling to ambient temperature. The samples were removed from the glass slides and placed on the surface of the 25 mm rheometer base plates.

Upon manual inspection, the adhesive samples and comparison samples were observed to be optically clear to the naked eye. All samples exhibited viscoelastic behavior without flow (a soft solid). It was observed that the adhesive sample exhibited strong tack to human skin (fingertip and inside of wrist), nitrile and glass, while the comparison samples exhibited elastomeric properties with little to no tack to human skin (fingertip or inside of wrist) and nitrile.

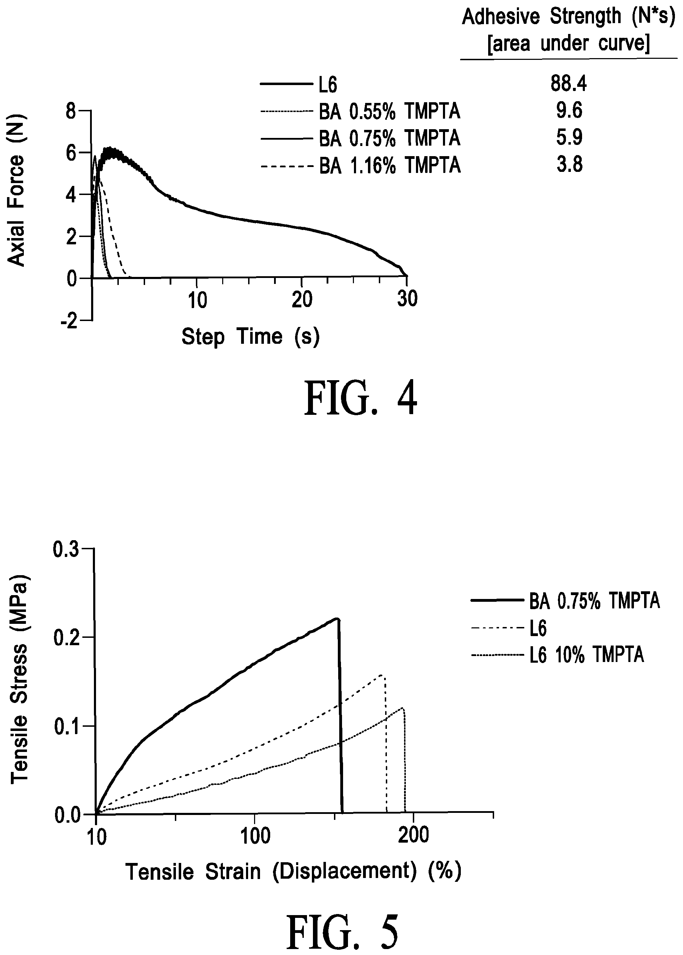

Tack strength was quantitatively measured using a TA Instruments Discovery HR-2 rheometer with a 8.00 mm aluminum tip and Peltier-cooled aluminum base plate, with measurements taken at a sample temperature of 25 °C. The sample was contacted and compressed for 60 seconds, after which the rheometer tip was withdrawn at a rate of 100 micrometers per second, and the axial force was measured. The maximum axial tack force measured was 6 N for the adhesive sample. The same process was repeated for the comparison samples, yielding peak tack forces of 5 N, 6 N, and 4 N for samples (a), (b) and (c) to aluminum, respectively. The adhesive strength, measured in Newton-seconds (Ns), is calculated by integrating the area under the force vs time measurements for each sample, yielding 88.4 Ns for the adhesive, and 9.6 Ns, 5.9 Ns and 3.8 Ns, for comparison samples (a), (b) and (c) (FIG. 4). The tack energy of the adhesive was also computed by multiplying the adhesive strength integral multiplied by the constant withdraw rate of 100 micrometers/sec, yielding a tack energy of 8.84x10-3 Joules (J) for the adhesive, and 0.96x10-3 J, 0.59x10-3 J and 0.38x10-3 J for the comparison samples (a), (b) and (c), respectively.

### 4.7.11. Example 11

A series of adhesive samples were prepared by photopolymerizing of monomeric solutions with the following composition: 98.43 wt% LMA and 0.59 wt% TMPTA with 0.98 wt% 2DMPA photoinitiator. The monomers were massed out using an analytical balance and mixed until a homogenous solution was formed using a FlackTek SpeedMixer (DAC 330-100 PRO).

A prepolymer solutions were formed by partially curing 90 g of the monomeric solution under a 365 nm UV light source (UVP CL-1000 UV crosslinker) for 14 minutes under a continuous-flow inert nitrogen atmosphere in a "pre-cure" step. This was repeated four individual times. Following the pre-cure, an additional mass of TMPTA was added to each of the four samples: (1) 0.00 wt% TMPTA ("L6"), (2) 0.06 wt% TMPTA ("L6 10% TMPTA"), (3) 0.12 wt% TMPTA, and (4) 0.18 wt% TMPTA. The solutions were again mixed unitl homogenous using a SpeedMixer.

Subsequently, 5 mL volumes of the prepolymer solutions were then pipetted onto silicone-impregnated paper affixed to a 1.0 mm thick glass slide and cured for a further 41 minutes under UV light in an inert nitrogen atmosphere. Upon removal from the crosslinker, the samples were each individually sandwiched between another piece of silicone-impregnated paper affixed to a 1.0 mm thick glass slide and compressed to a thickness of between 600-700 micrometers using spring clamps, and post-cured for 1 hour at 120 °C and atmospheric pressure, before cooling to ambient temperature. The samples were observed to be optically clear to the naked eye.

A tensile strain capacity assessment was performed using a universal testing machine (Instron 5944). Samples were measured with micrometers to be 600 microns thick, and were cut into 2 cm wide rectangular strips and clamed in the Instron fixtures, with a starting fixture separation of 5 cm. The sample was strained in tension at a rate of 300 mm/min until failure (FIG. 5) Sample (1) failed at 0.15 MPa stress at 182% strain. Sample (2) failed at 0.12 MPa stress and 217% strain. Sample (4) failed at 0.067 MPa stress and 120% strain.

An additional non-adhesive comparison sample was prepared from a monomeric solution comprising 98.27 wt% BA, 0.75 wt% TMPTA, 0.98 wt% DMPA; which was injected between two Rain-X coated 1.0 mm thick glass slides which were separated by 1.0 mm of glass spacers. The samples were cured under a 365 nm UV light source (UVP CL-1000 UV crosslinker) for 15 minutes, and post-cured at 90 °C and atmospheric pressure for 12 hours, before cooling to ambient temperature. This sample failed at 0.22 MPa stress and 152% strain.

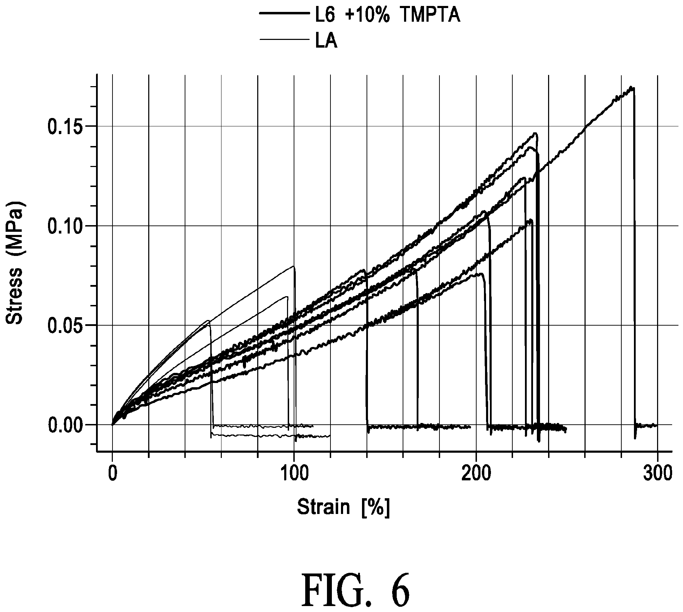

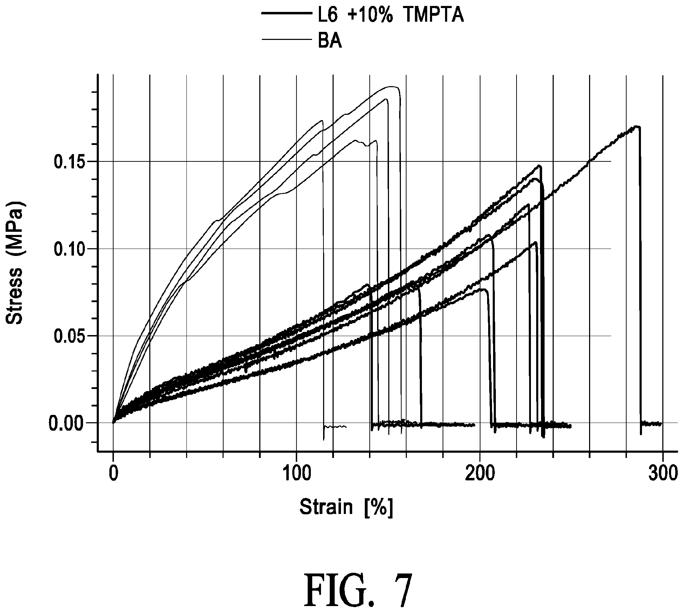

Additional strain capacity measurements were performed on L6 10% TMPTA samples, BA samples and LA samples. The samples thicknesses were measured, cut into 1.6 cm wide rectangular strips and clamed in the Instron fixtures, with a starting fixture separation of 3 cm. The sample was strained in tension at a rate of 300 mm/min until failure (FIG. 6 and FIG. 7). L6 10% TMPTA had a significantly higher strain capacity than the LA elastomer. The Young's modulus was computed from the slope of the stress-strain curve between 0% and 5% strain and found to be 1.33 ± 0.47 kPa for L6 10% TPMTA, 1.33 ± 0.40 kPA for LA and 3.15 ± 1.13 kPA for BA.

Alien tape, a commercially available thick elastomeric nano-texture tape, was also tested in the same manner. It did not fail before the maximum extents of the Instron were reached.

### 4.7.12. Example 12

Adhesive samples were prepared by photopolymerizing a monomeric solution of 98.43 wt% lauryl methacrylate (LMA) and 0.59 wt% trimethylolpropane triacrylate (TMPTA), with 0.98 wt% 2,2-dimethoxy-2-phenylacetophenone (DMPA) photoinitiator. The monomers were massed out using an analytical balance and mixed until a homogenous solution was formed using a FlackTek SpeedMixer (DAC 330-100 PRO). Approximately 1.5 mL of solution was pipetted onto 75 by 50 mm glass slides, 1.0 mm in thickness, and cured under a 365 nm UV lamp (UVP CL-1000 UV crosslinker) for 1 hour in an inert nitrogen atmosphere. The samples were removed from the crosslinker and sandwiched between another piece of silicone paper affixed to a 1.0 mm thick glass slide, and compressed using spring clips, yielding a gap thickness of approximately 100 microns. The samples were then post-cured in at 120 °C for 1 hour at atmospheric pressure, and then left to cool to ambient temperature.

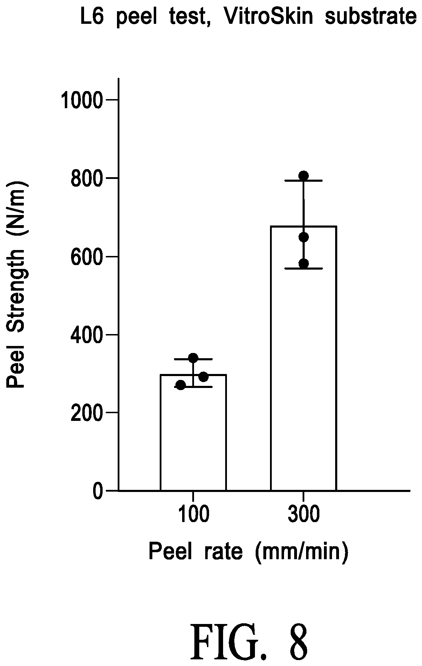

180-degree Peel tests were conducted using a universal testing machine (Instron 5944) and Vitro-Skin (Florida Suncare Testing Inc, IMS Division) as a human-skin substrate analog (**FIG. 8**). The A 5 cm wide rectangular strip of Vitro-Skin was pressed onto the adhesive with moderate finger pressure and left to settle for 1 minute prior to testing. The glass slide was affixed in a stationary position to the lower Instron fixture. The Vitro-Skin was affixed to the upper fixture in a standard 180-degree peel test configuration. The Vitro-Skin was peeled from the adhesive at 100 mm/min and repeated for three samples. The average peel strength (force per unit width) was measured to be approximately 300 N/m. The procedure was repeated for a peel rate of 300 mm/min yielding an average of approximately 700 N/m illustrating a shear-rate responsive behavior of the adhesive, that at very low peel rates, the peeling force is low, and at high peel rates, the adhesion force remains high.

Comparative analyses were carried for 3M Tegaderm, a commercially available medical adhesive. A 26 mm wide rectangular strip of Tegaderm was pressed onto a clean glass slide with a 500-gram mass for 1 minute prior to testing. The glass slide was affixed in a stationary position to the lower Instron fixture. The Tegaderm was affixed to the upper fixture in a standard 180-degree peel test configuration. The adhesive was again peeled from the glass slide at 100 mm/min and 300 mm/min, repeated 10 times for each peel rate. Average peel forces were approximately 98 N/m and 94 N/m, respectively, indicating no significant shear-rate dependence of the adhesive.

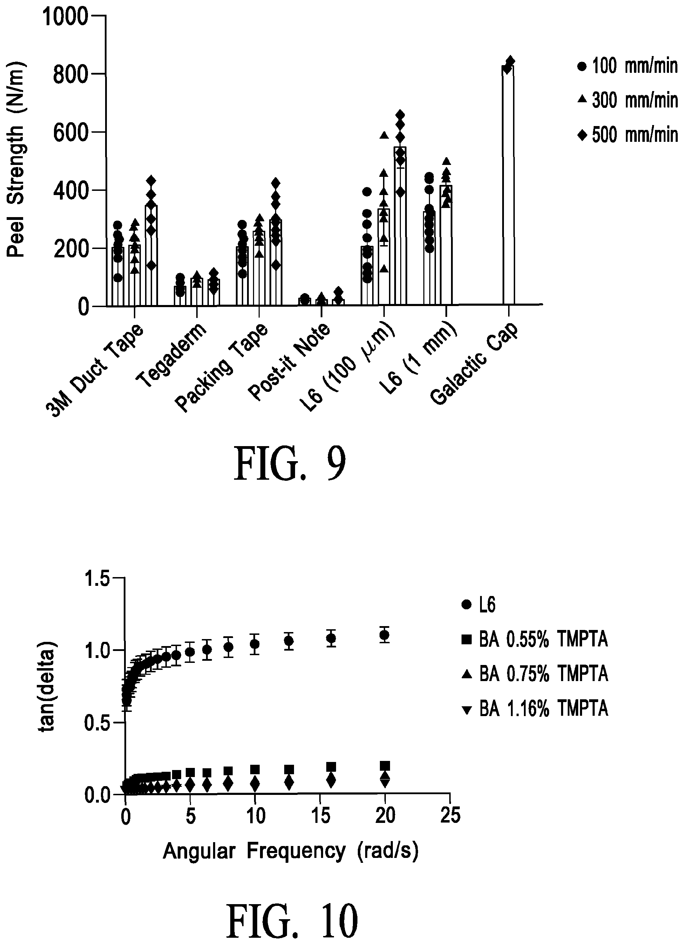

Additional comparisons were carried out on several other commercially available adhesives (**FIG. 9**). For a conventional pressure-sensitive adhesives, variations observed in the peel force required for removal are predominantly influenced by the pressure used to apply the adhesive to the target surface, and optionally time in contact with the target surface, in comparison to the speed or peel rate at which the adhesive is removed, which have no influence or a weak influence on the delamination behavior (e.g., peel strength).

In contrast, the stimuli-responsive adhesives described herein exhibit a different behavior (peel strength or perceived pain) during delamination at different peel rates that is predominantly influenced by the peel rate or shear rate of the adhesive as it is being removed (or another stimulus applied to the adhesive immediately before delamination). The delamination behavior is independent or weakly dependent on the pressure used to apply the adhesive to the target surface, and optionally the time in contact with the target surface. (These statements assume target surface, environmental variables, temperature, humidity, are all controlled to be equivalent).

### 4.7.13. Example 13

Adhesive samples were prepared by photopolymerizing a monomeric solution of 98.43 wt% lauryl methacrylate and 0.59 wt% trimethylolpropane triacrylate (TMPTA), with 0.98 wt% 2,2-dimethoxy-2-phenylacetophenone (DMPA) photoinitiator. The monomers were massed out using an analytical balance and mixed until a homogenous solution was formed using a FlackTek SpeedMixer (DAC 330-100 PRO). Approximately 3 mL of solution was pipetted onto 25 mm disposable aluminum rheometer base plates (TA Instruments) and cured under a 365 nm UV lamp (UVP CL-1000 UV crosslinker) for 1 hour in an inert nitrogen atmosphere. The samples were then post-cured in at 120 °C for 1 hour at atmospheric pressure. The samples were then allowed to cool to ambient temperature.

A set of comparison samples were prepared using n-butyl acrylate (BA), TMPTA and DMPA. The comparison monomeric solutions had the following compositions: (a) 98.37 wt% BA, 0.55 wt% TMPTA, 1.08 wt% DMPA; (b) 98.27 wt% BA, 0.75 wt% TMPTA, 0.98 wt% DMPA; (c) 97.87 wt% BA, 1.16 wt% TMPTA, 0.98 wt% DMPA. Solutions (a-c) were injected between two Rain-X coated 1.0 mm thick glass slides which were separated by 1.0 mm of glass spacers. The samples were cured under a 365 nm UV light source (UVP CL-1000 UV crosslinker) for 15 minutes, and post-cured at 90 °C and atmospheric pressure for 12 hours, before cooling to ambient temperature. The samples were removed from the glass slides and placed on the surface of the 25 mm rheometer base plates.

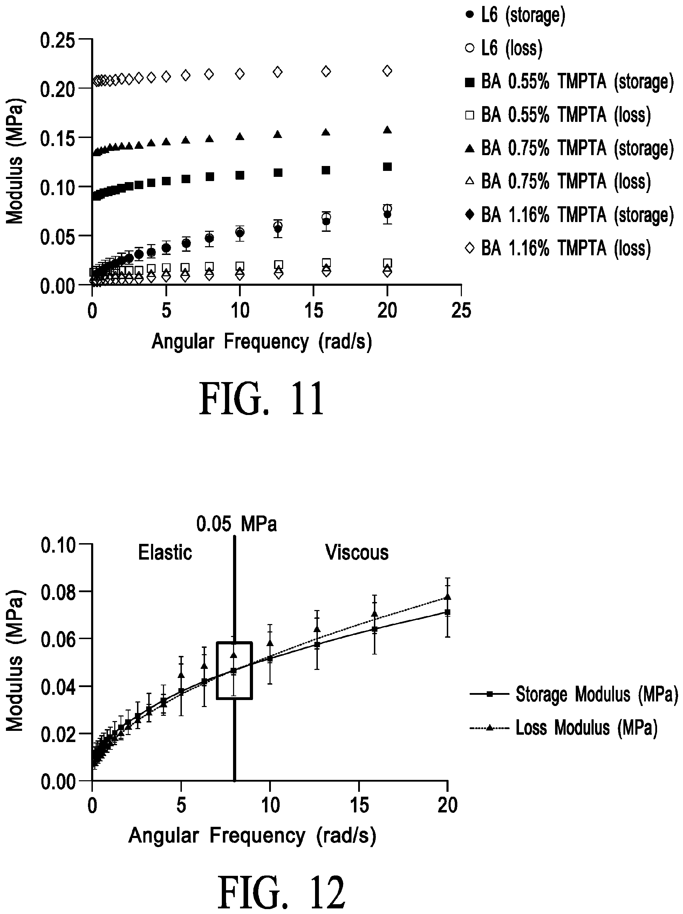

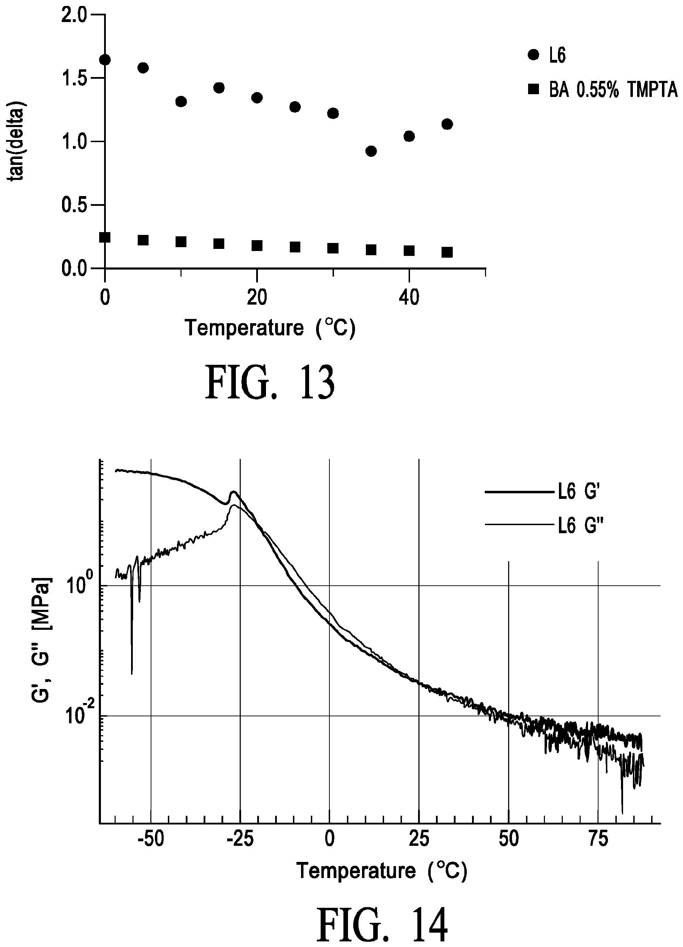

Oscillating temperature sweeps were performed on a TA Instruments Discovery HR-2 rheometer. A strain oscillation amplitude sweep was performed on the adhesive sample at 25 °C to determine the nominal oscillating strain amplitude. The storage and loss moduli curves were found to be constant, and a 1% strain was selected (**FIG. 11** and **FIG. 12**). A logarithmic sweep in angular frequency from 1.0 to 100.0 rad/sec was performed with 5 points per decade, and over a temperature sweep of from 0 °C to 50 °C in 5 °C steps (**FIG. 10** and **FIG. 13**). A high loss modulus for L6 was observed by a high tan(delta) value of between 0.5 at low frequencies to 0.9 at high frequencies. In contrast, the comparison samples (denoted by "butyl acrylate (2 drop TMPTA)" (b), "butyl acrylate (3 drop TMPTA)" (c) and "butyl acrylate (4 drop TMPTA) (d)) had a significantly lower loss modulus, near approximately 0.1 throughout the frequency range.

### 4.7.14. Example 14

Adhesive samples were prepared by photopolymerizing of monomeric solutions with the following composition: 98.43 wt% LMA and 0.59 wt% TMPTA with 0.98 wt% 2DMPA photoinitiator. The monomers were massed out using an analytical balance and mixed until a homogenous solution was formed using a FlackTek SpeedMixer (DAC 330-100 PRO).

A prepolymer solution was formed by partially curing 90 g of the monomeric solution under a 365 nm UV light source (UVP CL-1000 UV crosslinker) for 14 minutes under a continuous-flow inert nitrogen atmosphere in a "pre-cure" step. Subsequently, 5 mL of the prepolymer solution was pipetted onto silicone-impregnated paper affixed to a 1.0 mm thick glass slide and cured for a further 41 minutes under UV light in an inert nitrogen atmosphere. Upon removal from the crosslinker, the samples were sandwiched between another piece of silicone-impregnated paper affixed to a 1.0 mm thick glass slide and compressed to a thickness of between 600-700 micrometers using spring clamps, and post-cured for 1 hour at 120 °C and atmospheric pressure, before cooling to ambient temperature. The samples were observed to be optically clear to the naked eye.

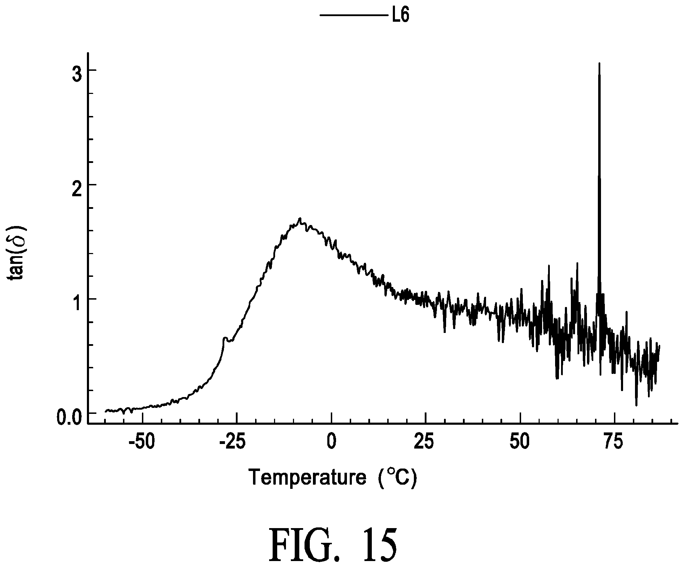

Dynamic Mechanical Analysis (DMA) was performed using a Mettler Toledo DMA1-Star instrument. The samples were performed by carefully cutting them into discs measuring approximately 0.80 mm in thickness and 4.75 mm in diameter. The shear deformation mode was selected, with a displacement limit of 1.0 micrometers. A temperature sweep was carried out from -50 °C to 100 °C, with a gradual heating rate of 3 °C per minute. Additionally, the frequency of deformation was set at 2 Hz. The storage and loss moduli were measured (**FIG. 14**), as well as tan(δ) (**FIG. 15**).

### 4.7.15. Example 15

LMA, TMPTA and 1.0 wt% DMPA photoinitiator were mixed until a homogeneous solution formed and were then UV cured at 365 nm in polypropylene boxes (approximately 0.1-1.0 mm thick samples) under nitrogen for 45 min in a UVP CL-1000 crosslinker), after which samples were post-cured at 120 °C under ambient conditions for approximately 1 hour. Samples, optically clear after post-curing at 120 °C, were then cooled to room temperature where they appeared optically clear to the naked eye. Samples exhibited excellent tack to human skin and could be removed with minimal or no pain.

Samples were also prepared separately using a photoreactor. A magnetic stir plate was placed under a UVP CL-1000L crosslinker, and approximately 100 mL of homogeneous LMA, TMPTA and 1.0 wt% DMPA photoinitiator was stirred at approximately 180 RPM using a magnetic stir bar while being irradiated at 365 nm for approximately 15-20 minutes, after which viscosity increased to all more uniform adhesive film coatings to be prepared. Optionally additional TMPA (10%, 20% or 30% increase from original TMPTA composition) was added after initial 15 to 20 minute irradiation and mixed for 15 minutes using a FlackTek SpeedMixer (DAC 330-100 PRO) at 800 rpm to assure homogeneous mixing. Additional TMPTA was added to account for converted TMPTA used to build molecular weight and increase viscosity that might not be incorporated into network. Adhesive coatings ranging in thickness from 0.025 mm to 1.75 mm made from increased viscosity prepolymers were prepared from increased viscosity LMA/TMPTA/DMPA mixtures on top of silicone release liner paper under nitrogen and irradiation in UVP CL-1000L crosslinker at 365 nm for 45 min and thermal post cure at 120 °C for 1 hour. Release liner/adhesive coating layers could be sandwiched between an additional silicone release liner paper and stored for further use and process demonstrated is consistent with that suitable for use in roll-to-roll UV coating of adhesives on release liners.

The roll or sheets of adhesives may be fabricated into a condom device through a suitable transfer process to mate the adhesive to a latex, elastomeric or other polymeric barrier layer. Additionally, multiple layers of adhesive may be laminated together to form a composite adhesive layer with a greater thickness if desired using a suitable roll-to-roll or sheet laminating fabrication process.

### 4.7.16. Example 16

Samples were prepared of monomeric species and DMPA photoinitiator to form linear polymers. Three compositions were prepared: (a) 99.0 wt% lauryl methacrelate (LMA) and 1.0 wt% DMPA, (b) 99.0 wt% lauryl acrylate (LA) and 1.0 wt% DMPA, and (c) 99.0 wt% butyl acrylate (BA) and 1.0 wt% DMPA using analytic balances. Each solution was mixed using a FlackTek SpeedMixer (DAC 330-100 PRO) at 400 rpm until a homogenous solution was achieved. Approximately 6 mL of solution was pipetted into 1 inch by 3 inch compartments in a polypropylene tray, for a total of 12 compartments per solution. The solutions were UV cured (UVP CL-1000 crosslinker) under a nitrogen inert atmosphere for 1 hour, after which they were thermally post-cured for 1 hour at 120 C. After post-curing the samples cooled to ambient temperature and were observed to be optically clear.

Samples of 2 mg/mL solution of each cured polymer were prepared in HPLC-grade THF. The solutions were filtered through 0.22 micron PTFE filters and 0.5 microliters were injected on GPC with THF as eluent and polystyrene standards. A 1 mL/min flow rate and a light scattering detector were used. It was found that the BA sample had an average molecular weight of 1381218 g/mol and a polydispersity index (PDI) of 1.2. The LMA sample was found to have an average molecular weight of 27040 g/mol and a PDI of 2.4. The LA sample was found to have an average molecular weight of 759767 g/mol and a PDI of 3.2.

### 4.7.17. Example 17

Crosslinked adhesive samples were prepared by photopolymerizing a monomeric solution of 98.43 wt% lauryl methacrylate and 0.59 wt% trimethylolpropane triacrylate (TMPTA), with 0.98 wt% 2,2-dimethoxy-2-phenylacetophenone (DMPA) photoinitiator (L6). The monomers were massed out using an analytical balance and mixed until a homogenous solution was formed using a FlackTek SpeedMixer (DAC 330-100 PRO). Approximately 3 mL of solution was pipetted onto 25 mm disposable aluminum rheometer base plates (TA Instruments) and cured under a 365 nm UV lamp (UVP CL-1000 UV crosslinker) for 1 hour in an inert nitrogen atmosphere. The samples were then post-cured in at 120 °C for 1 hour at atmospheric pressure. The samples were then allowed to cool to ambient temperature.

Comparison samples comprised of linear polymers were prepared by photopolymerizing monomeric solutions of (a) 99.0 wt% lauryl methacrelate (LMA) and 1.0 wt% DMPA, and (b) 99.0 wt% butyl acrylate (BA) and 1.0 wt% DMPA using analytic balances. Each solution was mixed using a FlackTek SpeedMixer (DAC 330-100 PRO) at 400 rpm until a homogenous solution was achieved. Approximately 6 mL of solution was pipetted into 1 inch by 3 inch compartments in a polypropylene tray, for a total of 12 compartments per solution. The solutions were UV cured (UVP CL-1000 crosslinker) under a nitrogen inert atmosphere for 1 hour, after which they were thermally post-cured for 1 hour at 120 °C. After post-curing, the samples cooled to ambient temperature and were observed to be optically clear. Approximately 0.5 mL of the polymer samples were scooped onto 25 mm disposable aluminum rheometer base plates and allowed to settle for 30 minutes.

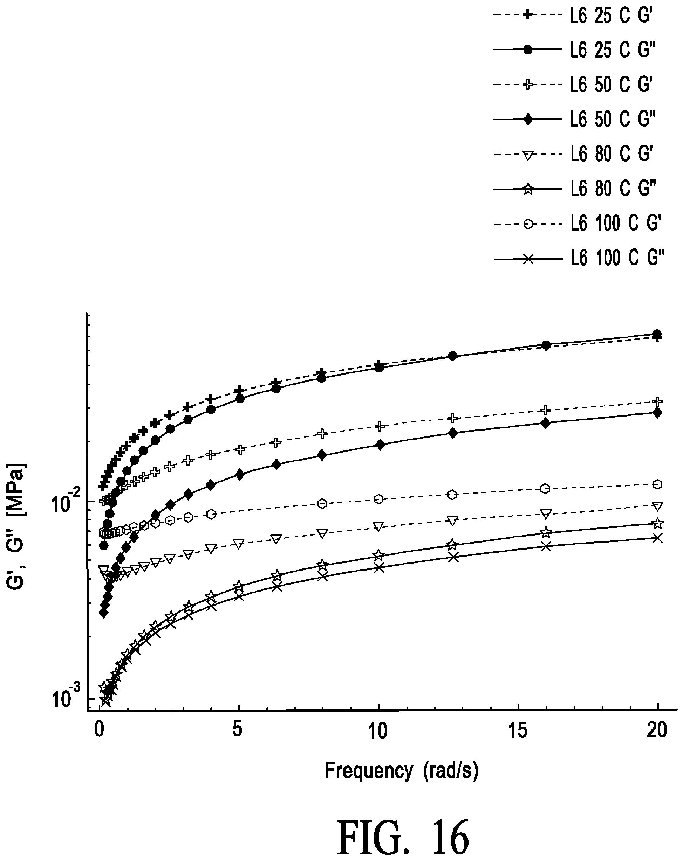

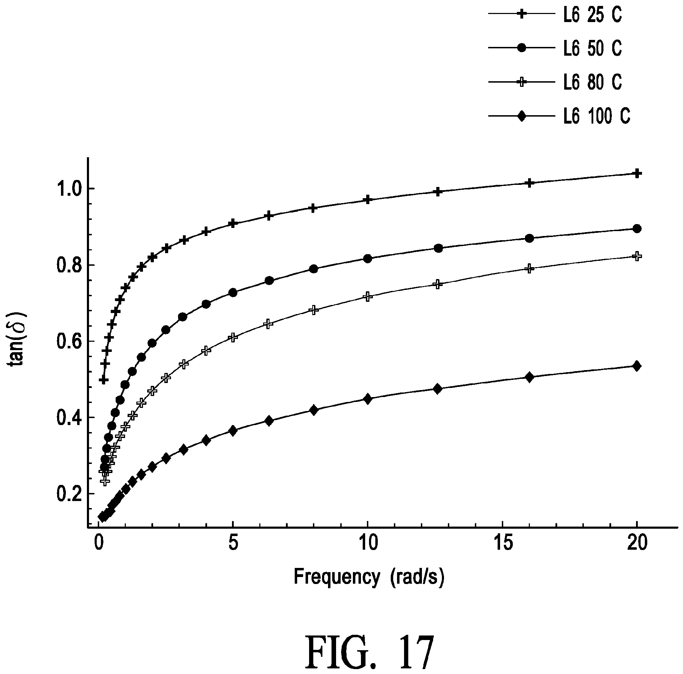

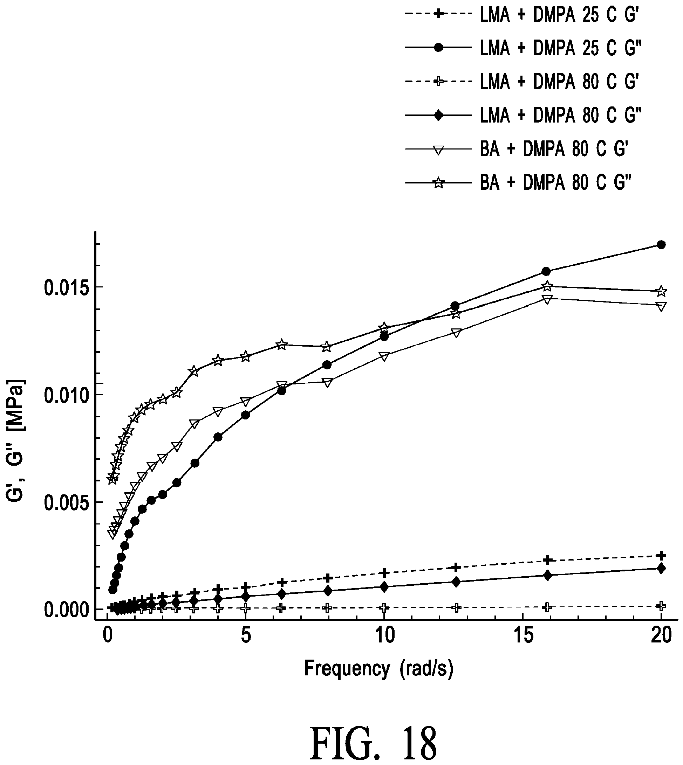

Frequency sweeps were performed on a TA Instruments Discovery HR-2 rheometer to measure the loss and storage moduli. A strain oscillation amplitude sweep was performed on the adhesive sample at 25 °C to determine the nominal oscillating strain amplitude. The storage and loss moduli curves were found to be constant, and a 1% strain was selected. A logarithmic sweep in angular frequency from 0.2 to 20.0 rad/sec was performed at 25 °C, 50 °C, 80 °C and 100 °C. The ratio of the loss modulus over the storage modulus, tan(delta) is reported, showing a high loss behavior (FIGs. 16-19).

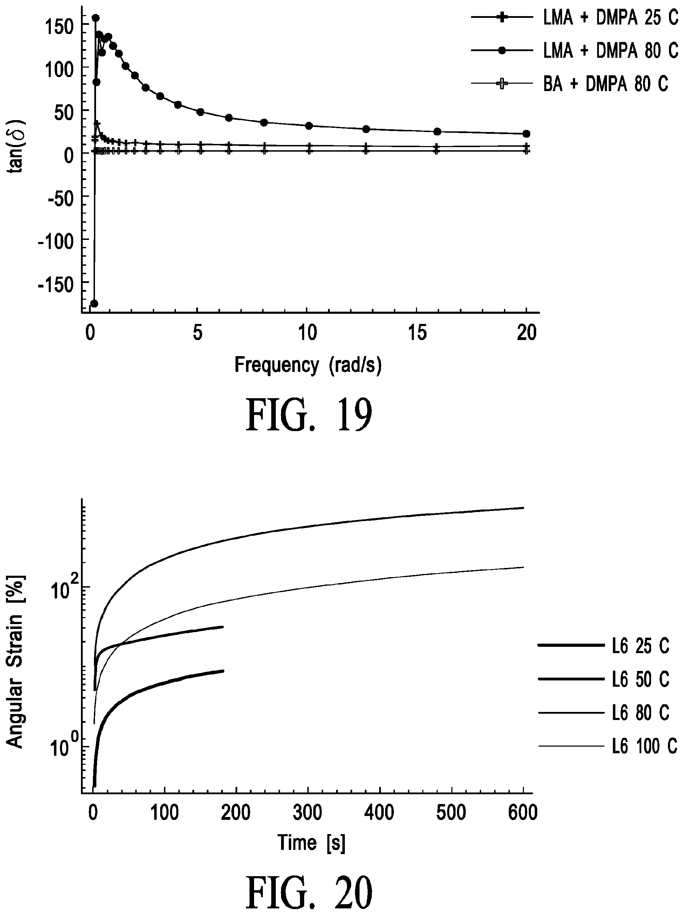

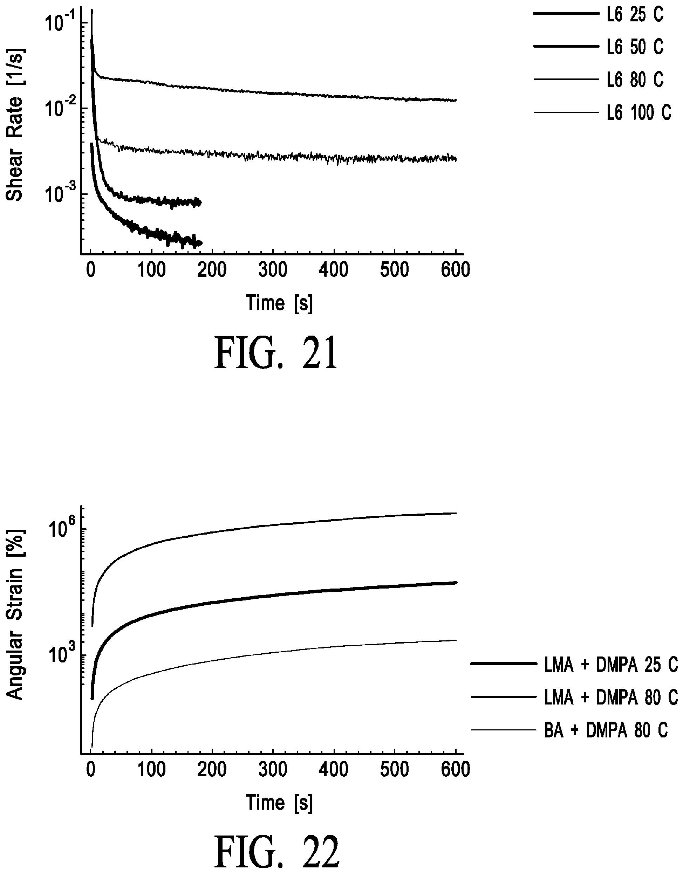

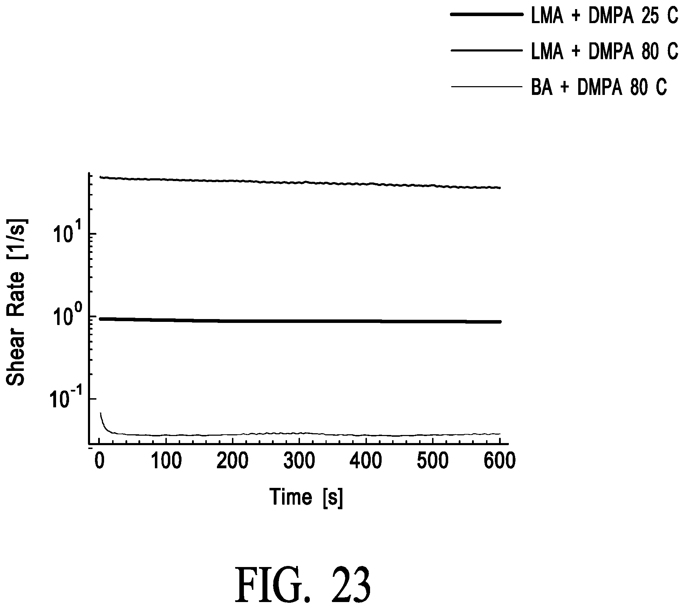

A constant torque of 500 micro-Newton-meters was then applied to samples at 25 °C, 50 °C, 80 °C, and 100 °C, and the resulting shear rate and strain were measured over time, for 180 seconds at 25 °C and 50 °C, and for 600 seconds at 80 °C and 100 °C (FIGs. 20-23).

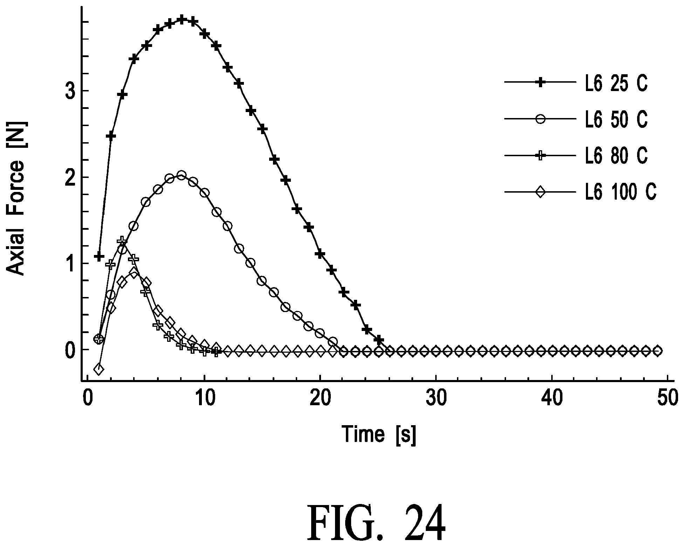

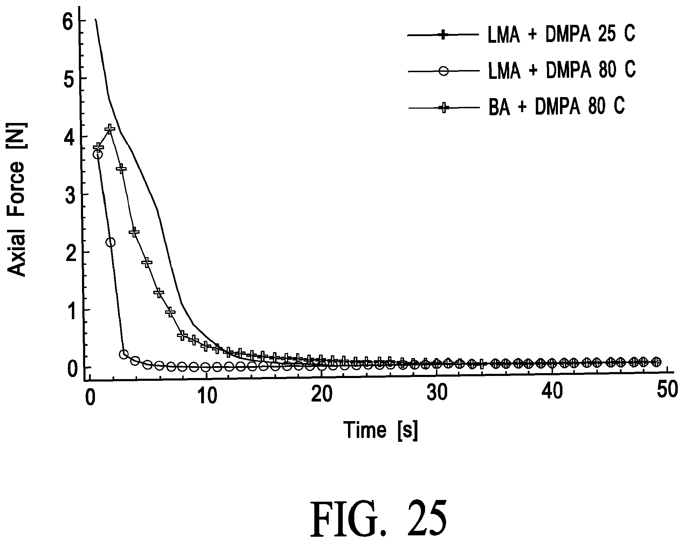

Finally tack strength was measured at 25 °C, 50 °C, 80 °C and 100 °C **(****FIG. 24** and **FIG.** 25). The sample was contacted and compressed for 60 seconds, after which the rheometer tip was withdrawn at a rate of 100 micrometers per second, and the axial force was measured. The peak tack force for L6 was found to be 3.8 N at 25 °C, 2.0 N at 50 °C, 1.3 N at 80 °C and 0.9 N at 100 °C. The LMA comparison sample was found to have a peak tack force of 6.0 N at 25 °C and 3.7 N at 80 °C. The BA comparison sample was found to have a peak tack force of 4.1 N at 80 °C.

The same process was repeated for the comparison samples, yielding peak tack forces of 5 N, 6 N, and 4 N for samples (a), (b) and (c) to aluminum, respectively. The adhesive strength, measured in Newton-seconds (Ns), is calculated by integrating the area under the force vs time measurements for each sample, yielding 88.4 Ns for the adhesive, and 9.6 Ns, 5.9 Ns and 3.8 Ns, for comparison samples (a), (b) and (c). The tack energy of the adhesive was also computed by multiplying the adhesive strength integral multiplied by the constant withdraw rate of 100 micrometers/sec, yielding a tack energy of 8.84x10⁻³ Joules (J) for the adhesive, and 0.96x10⁻³ J, 0.59x10⁻³ J and 0.38x10⁻³ J for the comparison samples (a), (b) and (c), respectively.

### 4.7.18. Example 18

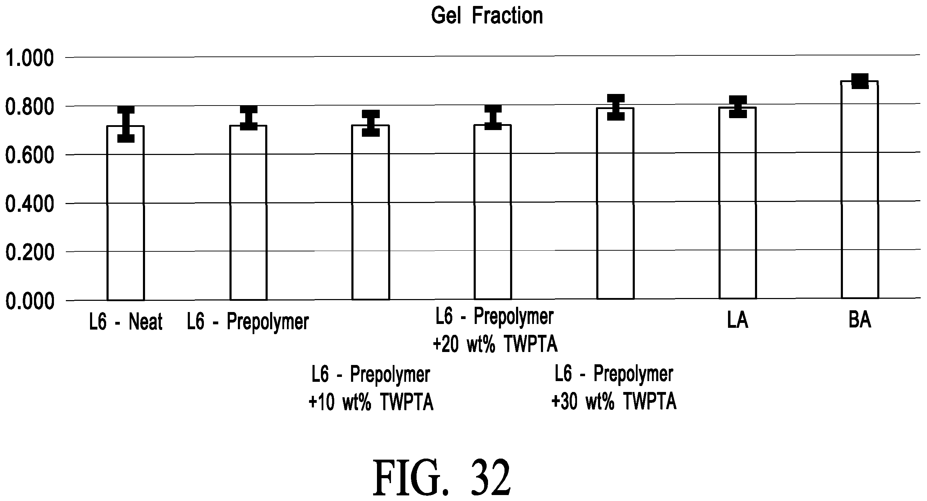

Sol-gel analysis was carried out according to known methods in dichloromethane (DCM) on UV cured samples. Cured polymer samples of masses ranging from approximately 0.2 g to 0.6 g were massed in tared glass vials and subjected to approximately 40 mL of DCM in sealed glass vials. The vials were mixed in a RapidVap vortexer at 35 RPM for 24 hours at approximately 25 °C, after which the DCM was decanted, resulting consistent gels, highly swollen in DCM, and were allowed to dry for 24 hours at ambient temperature and pressure in a commercial chemical fume hood and then further dried for 3 hours at 60 °C and subsequently 2 hrs at 120 °C. Final masses of remaining samples/vials were taken, and gel fractions were calculated as final polymer mass divided by initial polymer mass. Representative data are shown in Table 1 and **FIG. 32****.**

**Table 1.**

| **Sample** | **Weight Ratio TMPTA** | **Mole Ratio TMPTA** | **Gel Fraction** | **Gel Fraction StDev** |
|---|---|---|---|---|
| **L6- Neat** | 0.00600 | 0.006988 | 0.726176221 | 0.062066072 |
| **L6- Prepolymer** | 0.00600 | 0.006988 | 0.753822567 | 0.034776617 |
| **L6- Prepolymer + 10 wt% TMPTA** | 0.00660 | 0.007687 | 0.72430944 | 0.03909231 |
| **L6- Prepolymer + 20 wt% TMPTA** | 0.00720 | 0.008386 | 0.751012672 | 0.039157418 |
| **L6- Prepolymer + 30 wt% TMPTA** | 0.00780 | 0.009085 | 0.788462401 | 0.039040367 |
| **LA** | 0.00600 | 0.007396 | 0.794309658 | 0.027018842 |
| **BA** | 0.00600 | 0.013872 | 0.901328696 | 0.009819267 |

Despite highly weight ratios and mole ratios (within 2x crosslink density), the adhesives exhibited similar gel fraction in the range of 0.70 to 0.90.

### 4.7.19. Example 19

A series of condoms were fabricated using L6, L6 + 10 wt% increased TMPTA, L6 + 20 wt% increased TMPTA, L6 + 30 wt% increased TMPTA, lauryl acrylate (LA) with 0.6 wt% TMPTA and a Styrenic adhesive. Condoms approximately 1 inch by 2 inches rectangular in shape or 1 inch in diameter circular in shape were prepared by transferring approximately 3 inches by 3 inches by squares of adhesive to latex dental dams approximately 0.20 mm in diameter. Adhesive thicknesses ranged from 200 microns to 800 microns thick.

The fabricated condoms were placed on a combination of erect and flaccid penises of several individuals. In some cases, vaginal penetrative sexual intercourse was then carried out, in other cases manual stimulation was carried out. Subsequently, the adhesives were removed from the penis, and the pain self-reported using a standard Wong-Baker type pictogram-numeric pain scale ranging from 0 (no pain) to 10 (worst imaginable pain).

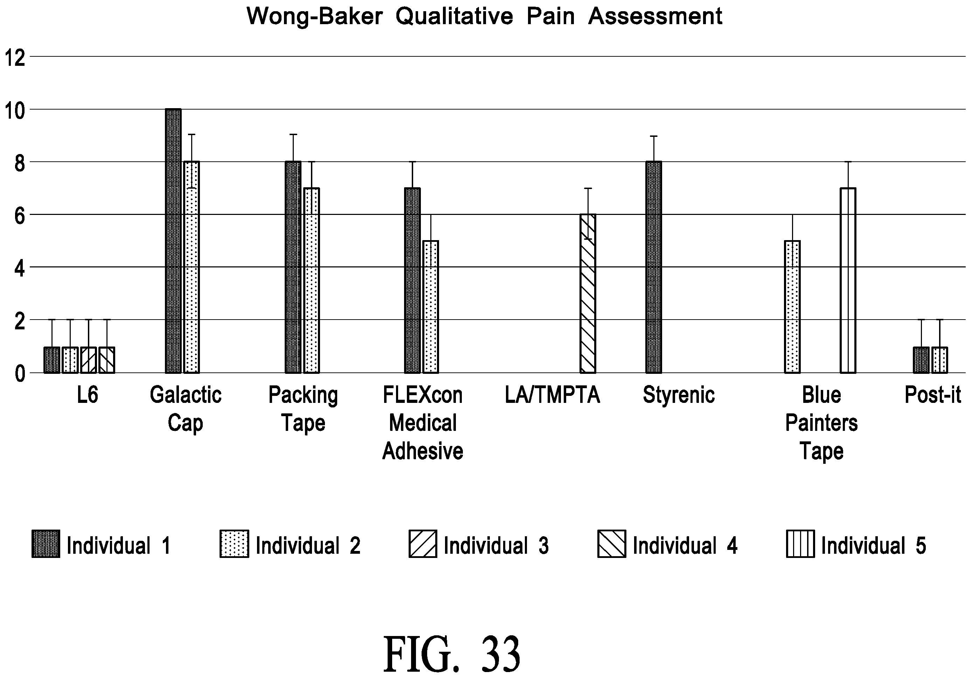

Additionally, commercially available adhesive tapes were tested, which included the Galactic Cap, FLEXcon medical adhesive, 3M Scotch-brand clear packing tape, 3M Scotch-brand blue painters tape and 3M post-it notes. Results are shown in **FIG. 33****.,** and the number of samples of each type of adhesive per individual is reported in Table 2.

**Table 2**

| **Adhesive Type** | **Individual 1** | **Individual 2** | **Individual 3** | **Individual 4** | **Individual 5** |
|---|---|---|---|---|---|
| **L6** | >100 | >25 | >5 | 2 | |
| **Galactic Cap** | 8 | 2 | | | |
| **Packing Tape** | | 6 | | | |
| **FLEXcon Medical Adhesive** | 5 | 4 | | | |
| **LA/TMPTA** | | | | 1 | |
| **Styrenic** | 1 | | | | |
| **Blue Painters Tape** | | 8 | | | 2 |
| **Post-It** | 10 | 10 | | | |

It was observed that L6 and L6 variant samples typically took between 3 and 15 seconds to remove, averaging between 5 and 8 seconds. Galactic Cap samples took more than 5 minutes to remove per instance and required the use of coconut oil or hydrating lotion. Packing tape, FLEXcon medical adhesive and blue painters' tape all typically took between 10 and 30 seconds to remove, depending on surface area of the sample. The post-it note while relatively pain free and fast to remove (between 1 and 5 seconds), had a relatively low adhesion force.

The following publications are cited in the present disclosure:
U.S. Provisional Patent Application Nos. 63/381,071 (filed October 26, 2022); 63/381,653 (filed October 31, 2022); 63/493,761 (April 2, 2023); 63/501,237 (filed May 10, 2023); 63/493,762 (filed April 2, 2023); and 63/501,238 (filed May 10, 2023). Additionally, the following PCT patent application, concurrently filed with the present application, is also cited:
- the application titled "STIMULUS-RESPONSIVE, REVERSIBLE ADHESIVE MEDICAL COMPOSITIONS, ARTICLES AND METHODS" filed October 26, 2023 under attorney docket no. 41822-57382 (002WO).

## Claims

1. A condom comprising:
a barrier layer suitable for prohibiting passage of semen and comprising an inner surface and outer surface, and
an adhesive layer adhered to at least a portion of the inner surface of the barrier layer; wherein:
the condom is configured for adhesion of the adhesive layer to the glans of the penis of a human subject,
the condom is configured to provide fractional coverage of the penis, and
the adhesive layer comprises an adhesive that comprises a stimuli-responsive polymer formed from one or more monomers and one or more polyfunctional crosslinkers, wherein the stimuli-responsive polymer is a crosslinked polymer having a heterogeneous crosslink network, and wherein
when the condom is applied to the penis, the adhesive layer adheres to the glans of the penis, and
the adhesive layer can be removed from the glans of the penis following application of a stimulus to the condom.

2. The condom of claim 1, wherein the condom is sized and shaped such that, when applied to the penis, the condom does not contact the corona and/or shaft of the penis.

3. The condom of claim 1 or 2, wherein the barrier layer: comprises a membrane or film; and/or
comprises natural latex rubber, synthetic rubber, amorphous polyurethane, semi-crystalline polyurethanes (including various thermoplastic polyurethanes, polyethylene, polypropylene, polydimethylsiloxane and other silicone rubbers), polyethylene terephthalate, poly(vinyl chloride), polyisoprene, vulcanized polyisoprene or other vulcanized or crosslinked rubbers, ethylene vinyl acetate, poly(vinyl acetate), elastomeric or flexible materials, or a combination thereof, preferably
natural latex rubber, synthetic rubber, or polyurethanes; and/or
has a thickness from 0.001 mm to 2 mm, e.g., from 0.001 mm to 1.5 mm, from 0.001 mm to 1 mm, from 0.001 mm to 0.5 mm, from 0.001 mm to 0.1 mm, from 0.001 mm to 0.01 mm, from 0.025 mm to 0.25 mm, such as, for example, from 0.025 mm to 0.2 mm, from 0.025 mm to 0.15 mm, from 0.025 mm to 0.1 mm, or from 0.025 mm to 0.05 mm.

4. The condom of any one of claims 1-3, wherein the barrier layer further comprises a reservoir sized and shaped suitably for collecting semen ejaculated from the penis, preferably
wherein the reservoir is configured distal to the urethral opening of the penis.

5. The condom of claim 4, wherein the reservoir is spherical or cylindrical, and/or
wherein the reservoir self-forms when subjected to pressure from ejaculation by the penis, and/or
wherein the reservoir comprises a polymer coating that swells or gels when contacted with semen, preferably
wherein the polymer coating comprises chitosan, alginate, polyacrylic acid, crosslinked polyacrylic acid, sodium polyacrylate, crosslinked sodium polyacrylate, or a combination thereof.

6. The condom of any one of claims 1-5, wherein the condom or barrier layer has a planar or curved geometry selected from a square, circle, oval, hemisphere, rectangle, polygon, or curvilinear polygon, preferably:
wherein the barrier layer has a circle geometry, preferably
wherein the radius of the circle is at least about 0.5 cm, e.g., about 1.0 cm, about 2.0 cm, about 3.0 cm, or about 5.0 cm; or
wherein the barrier layer has a rectangle geometry, preferably
wherein the rectangle has a length from 0.5 cm to 5 cm and a width from 0.5 cm to 5 cm, and/or
wherein the condom or barrier layer is planar.

7. The condom of any one of claims 1-6, further comprising: a lubricant on the outer surface of the barrier layer, preferably
wherein the lubricant is selected from a water-based lubricant, silicon-based lubricant, polysaccharide-based lubricant, natural lubricant, and oil-based lubricant; and/or
a spermicide on the outer surface of the barrier layer in the form of a solution or a gel, preferably
wherein the spermicide is selected from Nonoxynol-9, octoxynol-9, benzalkonium chloride, lactic acid, menfegol, and combinations thereof.

8. The condom of any one of claims 1-7, further comprising an elastomeric ring affixed to the outer portion of the inner surface of the barrier layer or the edge of the barrier layer, preferably
further comprising one or more protruding arms connected to the elastomeric ring.

9. The condom of any one of claims 1-8, wherein the adhesive layer is: adhered to only a portion of the inner surface of the barrier layer, preferably
wherein the adhesive layer is adhered to the outer portion of the inner surface of the barrier layer; or
coextensive with the inner surface of the barrier layer.

10. The condom of any one of claims 1-9, wherein the adhesive layer is in a patterned configuration, and/or
wherein the adhesive layer has a thickness from 0.1 microns to 3,000 microns, e.g., from 1 micron to 2,000 microns, from 25 microns to 1,000 microns, from 25 microns to 750 microns, or from 25 microns to 500 microns.

11. The condom of any one of claims 1-10, wherein the stimuli-responsive polymer has a glass transition temperature (Tg) from -100 to 100 °C, preferably from 0 °C to 50 °C, preferably from 0 °C to 40 °C, more preferably from 5 °C to 40 °C.

12. The condom of any one or more of claims 1-11, wherein the crosslinked polymer comprises one or more C6 to C30 side chains or one or more C6 to C30 dangling chain ends, preferably wherein the C6 to C30 side chains or C6 to C30 dangling chain ends are C6 to C30 alkyl side chains, preferably C12 to C18 alkyl side chains.

13. The condom of any one of claims 1-12, wherein the crosslinked polymer is a semi-interpenetrating network or the crosslinked copolymer is an interpenetrating network.

14. The condom of any one of claims 1-13, wherein the stimuli-responsive polymer comprises monomers selected from acrylate monomers, methacrylate monomers, vinyl ether monomer, allyl monomers, thiol monomers, epoxy monomers, amine monomers, electron rich monomers, electron poor monomers, lactam monomers, lactone monomers, alcohol monomers, carboxylic acid monomers, isocyanate monomers, Diels-Alder monomers, ring opening metathesis monomers, or a combination thereof, preferably:
wherein the stimuli-responsive polymer comprises (i) acrylate monomers, preferably:
wherein the acrylate monomers are C6-C30 alkyl acrylate monomers, preferably C8-C20 alkyl acrylate monomers, preferably C8-C20 alkyl acrylate monomers, more preferably C8-C16 alkyl acrylate monomers, preferably
wherein the C8-C30 alkyl acrylate monomers are selected from octyl acrylate, nonyl acrylate, decyl acrylate, undecyl acrylate, dodecyl acrylate, tridecyl acrylate, tetradecyl acrylate, pentadecyl acrylate, hexadecyl acrylate, heptadecyl acrylate, octadecyl acrylate, nonadecyl acrylate, eicosyl acrylate, heneicosyl acrylate, docosyl acrylate, tricosyl acrylate, tetracosyl acrylate, pentacosyl acrylate, hexacosyl acrylate, heptacosyl acrylate, octacosyl acrylate, nonacosyl acrylate, triacontyl acrylate, and combinations thereof; or
wherein the acrylate monomers are selected from methyl acrylate, ethyl acrylate, Butyl acrylate, 2-ethylhexyl acrylate, isobutyl acrylate, Methoxyethyl acrylate, Hydroxyethyl acrylate, Hydroxypropyl acrylate, Ethoxylated (2) hydroxyethyl acrylate, Glycidyl acrylate, Methacrylic acid, Methyl methacrylate, Ethyl methacrylate, Butyl methacrylate, 2-Hydroxyethyl methacrylate, Cyclohexyl methacrylate, Poly(ethylene glycol) methacrylate, Poly(ethylene glycol) diacrylate, Poly(ethylene glycol) dimethacrylate, Trimethylolpropane triacrylate, Triethylene glycol diacrylate, Tetraethylene glycol diacrylate, Neopentyl glycol diacrylate, Diethylene glycol diacrylate, Dipentaerythritol hexaacrylate, Ethoxylated trimethylolpropane triacrylate, Propoxylated glycerol triacrylate, Stearyl acrylate, Lauryl acrylate, Isodecyl acrylate, Acrylic acid, Ethylene glycol diacrylate (EGDA), Triethylene glycol diacrylate (TEGDA), Propylene glycol diacrylate (PGDA), Butanediol diacrylate (BDDA), Neopentyl glycol diacrylate (NPGDA), Pentaerythritol tetraacrylate (PETA), 1,4-Butanediol diacrylate (BDA), Di(trimethylolpropane) tetraacrylate (DTMPTA), Bisphenol A ethoxylate diacrylate (BPAEDA), Ethoxylated bisphenol A diacrylate (EBPA), Decanediol diacrylate, Polyethylene glycol diacrylate (PEGDA), Trimethylolpropane triacrylate (TMPTA), Diethylene glycol diacrylate (DEGDA), and 1,6-Hexanediol diacrylate (HDDA), Trimethylolpropane triacrylate (TMPTA), Tripropylene glycol diacrylate (TPGDA), Pentaerythritol triacrylate (PETA), Dipentaerythritol pentaacrylate (DPEPA), Tris(2-hydroxyethyl) isocyanurate triacrylate (THEIC-TA), Triethylene glycol dimethacrylate (TEGDMA), Triallyl isocyanurate (TAIC), Triethylene glycol diacrylate (TEGDA), Ethoxylated trimethylolpropane triacrylate (ETMPTA), Triallyl cyanurate (TAC), and combinations thereof, and/or
wherein the stimuli-responsive polymer comprises at least 10 wt% acrylate monomers, e.g., at least 50 wt%, at least 65 wt%, at least 95 wt%, at least 96 wt%, at least 97 wt%, at least 98 wt%, or at least 99 wt%;
(ii) methacrylate monomers, preferably:
wherein the methacrylate monomers are C6-C30 alkyl methacrylate monomers, preferably C8-C30 alkyl methacrylate monomers, more preferably C8-C20 alkyl methacrylate monomers, more preferably C8-C16 alkyl methacrylate monomers, preferably
wherein the C8-C30 alkyl methacrylate monomers are selected from octyl methacrylate, nonyl methacrylate, decyl methacrylate, undecyl methacrylate, dodecyl methacrylate, tridecyl methacrylate, tetradecyl methacrylate, pentadecyl methacrylate, hexadecyl methacrylate, heptadecyl methacrylate, octadecyl methacrylate, nonadecyl methacrylate, eicosyl methacrylate, heneicosyl methacrylate, docosyl methacrylate, tricosyl methacrylate, tetracosyl methacrylate, pentacosyl methacrylate, hexacosyl methacrylate, heptacosyl methacrylate, octacosyl methacrylate, nonacosyl methacrylate, triacontyl methacrylate, and combinations thereof; or
wherein the methacrylate monomers are selected from Ethyl methacrylate, Butyl methacrylate, 2-Hydroxyethyl methacrylate, Cyclohexyl methacrylate, Isobornyl methacrylate, Stearyl methacrylate, Lauryl methacrylate, Isodecyl methacrylate, Tetrahydrofurfuryl methacrylate, Glyceryl methacrylate, Trimethylolpropane trimethacrylate, Trimethylolpropane triacrylate, Pentaerythritol triacrylate, Pentaerythritol tetramethacrylate, Poly(ethylene glycol) monomethyl ether methacrylate, Poly(ethylene glycol) monomethyl ether acrylate, Poly(ethylene glycol) diacrylate, Poly(ethylene glycol) dimethacrylate, Poly(ethylene glycol) monoacrylate, Ethoxylated bisphenol A dimethacrylate, Ethoxylated bisphenol A diacrylate, Ethoxylated trimethylolpropane triacrylate, Hydroxypropyl methacrylate, Methacrylic acid, Acryloyloxyethyltrimethylammonium chloride, Diethylaminoethyl methacrylate, Butylaminoethyl methacrylate, N,N-dimethylaminoethyl methacrylate, Methacryloyloxyethyl phthalate, Cyclopropyl methacrylate, and combinations thereof, and/or
wherein the stimuli-responsive polymer comprises poly (lauryl methacrylate), or
wherein the stimuli-responsive polymer comprises poly (lauryl methacrylate) and one or more other polymethacrylates, or
wherein the stimuli-responsive polymer adhesive comprises poly (lauryl methacrylate) and one or more polyacrylates, and/or
wherein the stimuli-responsive polymer comprises at least 10 wt% methacrylate monomers, e.g., at least 50 wt%, at least 65 wt%, at least 95 wt%, at least 96 wt%, at least 97 wt%, at least 98 wt%, or at least 99 wt%;
(iii) vinyl ether monomers selected from divinyl ether of ethylene glycol, divinyl ether of diethylene glycol, divinyl ether of triethylene glycol, divinyl ether of polyethylene glycol (DVE-PEG), divinyl ether of polypropylene glycol (DVE-PPG), Divinyl ether of poly(ethylene glycol) methyl ether (DVE-PEGME), divinyl ether of poly(ethylene glycol) butyl ether (DVE), Divinyl ether of poly(ethylene glycol) phenyl ether (DVE-PEGPhE), Divinyl ether of glycerol (DVE-Gly), Divinyl ether of 1,4-cyclohexanedimethanol (DVE-CHDM), Divinyl ether of neopentyl glycol (DVE-NPG), and combinations thereof, preferably
wherein the stimuli-responsive polymer further comprises acrylate monomers, methacrylate monomers, allyl monomers, thiol monomers, epoxy monomers, amine monomers, electron rich monomers, electron poor monomers, lactam monomers, lactone monomers, alcohol monomers, carboxylic acid monomers, isocyanate monomers, Diels-Alder monomers, ring opening metathesis monomers, or a combination thereof;
(iv) allyl monomers selected from Diallyl phthalate (DAP), Diallyl maleate (DAM), Diallyl succinate (DAS), Diallyl fumarate (DAF), Diallyl adipate (DAA), Diallyl sebacate (DAS), Diallyl terephthalate (DAT), Diallyl isophthalate (DAI), Diallyl itaconate (DAI), Diallyl carbonate (DAC), Diallyl diglycolate (DADG), Diallyl tris(2- hydroxyethyl) isocyanurate (DATHEIC), Triallyl cyanurate (TAC), Triallyl isocyanurate (TAIC), Triallyl trimellitate (TATM), Triallyl citrate (TAC), Triallyl phosphate (TAP), Triallylamine (TAA), Triallyl cyanide (TACN), Triallyl benzene-1,2,4-tricarboxylate (TABTC), Triallyl trimesate (TATM), Tris(2-hydroxyethyl) isocyanurate triallyl ether (THEIC-TAE), and combinations thereof, preferably
wherein the stimuli-responsive polymer further comprises acrylate monomers, methacrylate monomers, vinyl ether monomer, thiol monomers, epoxy monomers, amine monomers, electron rich monomers, electron poor monomers, lactam monomers, lactone monomers, alcohol monomers, carboxylic acid monomers, isocyanate monomers, Diels-Alder monomers, ring opening metathesis monomers, or a combination thereof;
(v) thiol monomers selected from 3-Mercaptopropionic acid; Thioglycolic acid; 3-Mercapto-1-propanol; 2- Mercaptoethanol; 2-(2-Mercaptoethoxy)ethanol; 2-(2-Mercaptopropionylamino)ethanol; 2-(2- Mercaptosuccinyl)ethyl acrylate; 3-(2-Mercaptopropionylamino)propionic acid; 3- (Mercaptopropyl)trimethoxysilane; 2,2'-(Ethylenebis(thio))diethanol; 3-Mercaptopropyltrimethoxysilane; 3-Mercaptopropylmethyldimethoxysilane; 3-(2,2-Dithiobis(ethylthio)propionylamino)propionic acid; 3,6,9- Trioxadecanethiol; 3-Mercapto-1,2-propanediol; 2,2'-Dithiodiethanol; N-Acetyl-L-cysteine; L-Cysteine; 2-(2- Mercaptoethyl)pyridine; 4-(2-Mercaptoethyl)morpholine; 3-Mercapto-1,2,4-triazole; Thiophenol; Pentaerythritol tetrakis(3-mercaptopropionate) (PETMP); Trimethylolpropane tris(3-mercaptopropionate) (TMPMP); Triethanolamine tris(3-mercaptopropionate) (TEAMP); Tris(2-hydroxyethyl) isocyanurate tris(3- mercaptopropionate) (THEICMP); Bis(3-mercaptopropyl) sulfide (BMPS); 1,2-ethanedithiol (EDT); 1,3-propanedithiol; 1,4-butanedithiol; 1,6-hexanedithiol; 1,8-octanedithiol and combinations thereof, preferably
wherein the stimuli-responsive polymer further comprises acrylate monomers, methacrylate monomers, vinyl ether monomer, allyl monomers, epoxy monomers, amine monomers, electron rich monomers, electron poor monomers, lactam monomers, lactone monomers, alcohol monomers, carboxylic acid monomers, isocyanate monomers, Diels-Alder monomers, ring opening metathesis monomers, or a combination thereof;
(vi) epoxy monomers selected from Bisphenol A diglycidyl ether (BADGE), Bisphenol F diglycidyl ether (BFDGE), Novolac diglycidyl ether (NGDE), Phenol novolac diglycidyl ether (PNGDE), Cycloaliphatic epoxy resins, Glycidyl ethers of aliphatic alcohols, Glycidyl ethers of aromatic alcohols, Triglycidyl isocyanurate (TGIC), Diglycidyl ether of 1,4-butanediol (BDDGE), Diglycidyl ether of neopentyl glycol (NPGDGE), Diglycidyl ether of propylene glycol (PGDGE), Epoxidized soybean oil (ESO), Epoxidized linseed oil (ELO), Dicyclopentadiene- based epoxy resins, Tetrafunctional epoxy resins, Epoxy phenolic novolac resins, and combinations thereof, preferably
further comprising acrylate monomers, methacrylate monomers, vinyl ether monomer, allyl monomers, thiol monomers, amine monomers, electron rich monomers, electron poor monomers, lactam monomers, lactone monomers, alcohol monomers, carboxylic acid monomers, isocyanate monomers, Diels-Alder monomers, ring opening metathesis monomers, or a combination thereof;
(vii) amine monomers selected from Ethylenediamine; Diethylenetriamine; Triethylenetetramine; Tetraethylenepentamine; Polyethyleneimine; Diaminopropane; Diaminobutane; Diaminopentane; Diethylenetriaminepentaacetic acid (DTPA); Tris(2-aminoethyl)amine; N-(2-Aminoethyl)piperazine; N-(3- Aminopropyl)morpholine; N,N-Dimethylaminopropylamine; N,N-Dimethylethylenediamine; 1,3- Diaminopropane; Isophoronediamine; Jeffamine D-230; Jeffamine T-403; Jeffamine M-207; Jeffamine EDR- 148; and combinations thereof, preferably
wherein the stimuli-responsive polymer further comprises acrylate monomers, methacrylate monomers, vinyl ether monomer, allyl monomers, thiol monomers, epoxy monomers, electron rich monomers, electron poor monomers, lactam monomers, lactone monomers, alcohol monomers, carboxylic acid monomers, isocyanate monomers, Diels-Alder monomers, ring opening metathesis monomers, or a combination thereof;
(viii) electron rich monomers selected from Vinyl ethers (e.g. vinyl methyl ether, vinyl ethyl ether), Vinyl acetate, Allyl alcohol, Allyl amine, N-Methylolacrylamide, N-Methylolmethacrylamide, N-Methylolallylamine, N- Methylolvinylacetamide, Acrolein diethyl acetal, Acrolein diethyl ketal, Diacetone acrylamide, 2- Hydroxyethyl acrylate, 2-Hydroxyethyl methacrylate, 2-Hydroxypropyl acrylate, 2-Hydroxypropyl methacrylate, 2,3-Dihydroxypropyl methacrylate, Glycidyl methacrylate, Glycidyl acrylate, Tetrahydrofurfuryl methacrylate, and N-Vinylpyrrolidone, n-vinylformamide, n-vinyl pyridine, styrene, styrene derivatives, and combinations thereof, preferably
wherein the stimuli-responsive polymer further comprises acrylate monomers, methacrylate monomers, vinyl ether monomer, allyl monomers, thiol monomers, epoxy monomers, amine monomers, electron poor monomers, lactam monomers, lactone monomers, alcohol monomers, carboxylic acid monomers, isocyanate monomers, Diels-Alder monomers, ring opening metathesis monomers, or a combination thereof;
(ix) electron poor monomers selected from Acrylonitrile, Methacrylonitrile, Methyl methacrylate, Acrylic acid, Methacrylic acid, Maleic anhydride, Itaconic acid, Fumaric acid, Acrylamide, Methacrylamide, N- Vinylcarbazole, Vinylidene chloride, Vinyl chloride, Vinyl sulfonic acid, Vinyl acetate, Styrene, Alpha-methylstyrene, Maleimide, N-Phenylmaleimide, and N-Butylmaleimide, maleic anhydride, and combinations thereof, preferably
wherein the stimuli-responsive polymer further comprises acrylate monomers, methacrylate monomers, vinyl ether monomer, allyl monomers, thiol monomers, epoxy monomers, amine monomers, electron rich monomers, lactam monomers, lactone monomers, alcohol monomers, carboxylic acid monomers, isocyanate monomers, Diels-Alder monomers, ring opening metathesis monomers, or a combination thereof;
(x) lactam monomers selected from Caprolactam, Valerolactam, Enantholactam, Capryllactam, Laurinlactam, Prolactam, Butyrolactam, Methionyl lactam, Methoxyethyl lactam, Methoxyethyl methionyl lactam, Dimethylaminoethyl lactam, Dimethylaminoethyl methionyl lactam, Dimethylaminoethyl acryloyl lactam, Dimethylaminoethyl methacryloyl lactam, N-vinylpyrrolidone, N-methylpyrrolidone, N- ethylpyrrolidone, and combinations thereof, preferably
wherein the stimuli-responsive polymer comprises acrylate monomers, methacrylate monomers, vinyl ether monomer, allyl monomers, thiol monomers, epoxy monomers, amine monomers, electron rich monomers, electron poor monomers, lactone monomers, alcohol monomers, carboxylic acid monomers, isocyanate monomers, Diels-Alder monomers, ring opening metathesis monomers, or a combination thereof;
(xi) lactone monomers selected from β-propiolactone, γ-butyrolactone, 8-valerolactone, ε-caprolactone, ω- pentadecalactone, β-butyrolactone, 8-decalactone, ε -decalactone, γ-decalactone, 8-dodecalactone, γ-dodecalactone, α-methylene- γ-butyrolactone, β-methyl-y-butyrolactone, β-methyl-γ-valerolactone, γ-hexalactone, and combinations thereof, preferably
wherein the stimuli-responsive polymer further comprises acrylate monomers, methacrylate monomers, vinyl ether monomer, allyl monomers, thiol monomers, epoxy monomers, amine monomers, electron rich monomers, electron poor monomers, lactam monomers, alcohol monomers, carboxylic acid monomers, isocyanate monomers, Diels-Alder monomers, ring opening metathesis monomers, or a combination thereof;
(xii) alcohol monomers selected from Ethylene glycol; Propylene glycol;1,3-Butanediol; 1,4-Butanediol; 1,5- Pentanediol; 1,6-Hexanediol; 1,10-Decanediol; Neopentyl glycol; Diethylene glycol; Triethylene glycol; Tetraethylene glycol; Polyethylene glycol (PEG); Polypropylene glycol (PPG); Polycaprolactone diol; Polymethylolpropane; Hydroxypivalyl hydroxymethylbutyrate (HPHMB); 1,4-Cyclohexanedimethanol; and combinations thereof, preferably
wherein the stimuli-responsive polymer further comprises acrylate monomers, methacrylate monomers, vinyl ether monomer, allyl monomers, thiol monomers, epoxy monomers, amine monomers, electron rich monomers, electron poor monomers, lactam monomers, lactone monomers, carboxylic acid monomers, isocyanate monomers, Diels-Alder monomers, ring opening metathesis monomers, or a combination thereof;
(xiii) carboxylic acid monomers selected from Adipic acid, Succinic acid, Glutaric acid, Sebacic acid, Malonic acid, Phthalic acid, Isophthalic acid, Terephthalic acid, Fumaric acid, Maleic acid, Itaconic acid, Citric acid, 1,4- Cyclohexanedicarboxylic acid, 1,3-Cyclohexanedicarboxylic acid, Dodecanedioic acid, and combinations thereof, preferably
wherein the stimuli-responsive polymer further comprises acrylate monomers, methacrylate monomers, vinyl ether monomer, allyl monomers, thiol monomers, epoxy monomers, amine monomers, electron rich monomers, electron poor monomers, lactam monomers, lactone monomers, alcohol monomers, isocyanate monomers, Diels-Alder monomers, ring opening metathesis monomers, or a combination thereof;
(xiv) isocyanate monomers selected from Toluene diisocyanate (TDI), Diphenylmethane diisocyanate (MDI), Hexamethylene diisocyanate (HDI), Isophorone diisocyanate (IPDI), 1,6-Hexamethylene diisocyanate (HMDI), 4,4'-Methylenebis(cyclohexyl isocyanate) (H12MDI), Naphthalene diisocyanate (NDI), 2,4-Toluene diisocyanate (2,4-TDI), 2,6-Toluene diisocyanate (2,6-TDI), Polymethylene polyphenyl isocyanate (PAPI), Desmodur N-100, Desmodur L-75, Desmodur HL, Desmodur H, Desmodur VP, Desmodur Z, and combinations thereof, preferably
wherein the stimuli-responsive polymer further comprises acrylate monomers, methacrylate monomers, vinyl ether monomer, allyl monomers, thiol monomers, epoxy monomers, amine monomers, electron rich monomers, electron poor monomers, lactam monomers, lactone monomers, alcohol monomers, carboxylic acid monomers, Diels-Alder monomers, ring opening metathesis monomers, or a combination thereof;
(xv) Diels-Alder monomers selected from Maleic anhydride, Furan, Cyclopentadiene, N-phenylmaleimide, Anthracene, N-ethylmaleimide, N-phenylnorbornene, N,N-dimethyl maleimide, 2,5-dimethylfuran, Tetracyanoethylene, Methyl vinyl ketone, and combinations thereof, preferably
wherein the stimuli-responsive polymer further comprises acrylate monomers, methacrylate monomers, vinyl ether monomer, allyl monomers, thiol monomers, epoxy monomers, amine monomers, electron rich monomers, electron poor monomers, lactam monomers, lactone monomers, alcohol monomers, carboxylic acid monomers, isocyanate monomers, ring opening metathesis monomers, or a combination thereof; or
(xvi) ring opening metathesis monomers selected from Norbornene, Dicyclopentadiene (DCPD), Cyclooctene, Tetracyclododecene (TCD), Cyclopentene, Cycloheptene, Cyclohexene, Bicyclo[2.2.1]hept-2-ene, Bicyclo[2.2.2]oct-5-ene, Tricyclo[5.2.1.0(2,6)]dec-8-ene (TCD-Diene), and combinations thereof, preferably
wherein the stimuli-responsive polymer further comprises acrylate monomers, methacrylate monomers, vinyl ether monomer, allyl monomers, thiol monomers, epoxy monomers, amine monomers, electron rich monomers, electron poor monomers, lactam monomers, lactone monomers, alcohol monomers, carboxylic acid monomers, isocyanate monomers, Diels-Alder monomers, or a combination thereof.

15. The condom of any one of claims 1-14, wherein the polyfunctional crosslinker is selected from: a difunctional crosslinker, a trifunctional crosslinker, or a tetrafunctional crosslinker, preferably
wherein the polyfunctional crosslinker is a trifunctional crosslinker, preferably
wherein the trifunctional crosslinker is an acrylate crosslinker;
poly(ethylene glycol) diacrylate, trimethylolpropane triacrylate; ethoxylated trimethyolpropane triacrylate; pentaerythritol tetraacrylate; ethoxylated pentaerythritol tetraacrylate; dipentaerithrotol hexaacrylate; ethoxylated dipentaerithrotol hexaacrylate; di-, tri-, tetra-, penta-, or hexa- epoxides; polythiols; polyalkenes; tris(2-acryloxyethyl) isocyanulate, ε-caprolactone modified tris(2-acryloxyethyl) isocyanurate, ethoxylated glycerine triacrylate, ethoxylated glycerine triacrylate, pentaerythritol triacrylate, and combinations thereof, and/or
wherein the stimuli-responsive polymer comprises from 0.001 to 29 wt%, preferably from 0.1 wt% to 1.5 wt% polyfunctional crosslinker, preferably from 0.2 wt% to 1 wt%, more preferably from 0.4 wt% to 0.8 wt%, and/or
wherein the weight ratio of the one or more monomers to the one or more polyfunctional crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, from 99.7:0.3 to 99.9:0.1, or from 99.8:0.2 to 99.9:0.1.

16. The condom of claim 1, wherein the stimuli-responsive polymer comprises a poly(lauryl methacrylate) polymer crosslinked with one or more polyfunctional crosslinkers, preferably:
wherein the polyfunctional crosslinker is selected from a difunctional crosslinker, a trifunctional crosslinker, or a tetrafunctional crosslinker, preferably
wherein the polyfunctional crosslinker is a trifunctional crosslinker, preferably
wherein the trifunctional crosslinker is an acrylate crosslinker; or
wherein the polyfunctional crosslinker is selected from poly(ethylene glycol) diacrylate, trimethylolpropane triacrylate; ethoxylated trimethyolpropane triacrylate; pentaerythritol tetraacrylate; ethoxylated pentaerythritol tetraacrylate; dipentaerithrotol hexaacrylate; ethoxylated dipentaerithrotol hexaacrylate; di-, tri-, tetra-, penta-, or hexa- epoxides; polythiols; polyalkenes; tris(2-acryloxyethyl) isocyanulate, ε-caprolactone modified tris(2-acryloxyethyl) isocyanurate, ethoxylated glycerine triacrylate, ethoxylated glycerine triacrylate, pentaerythritol triacrylate, and combinations thereof, and/or
wherein the weight ratio of the lauryl methacrylate to the one or more polyfunctional crosslinkers is from 98:2 to 99.9:0.1, e.g., from 98.5:1.5 to 99.9: 0.1; from 99:1 to 99.9:0.1, from 99.1:0.9 to 99.9:0.1, from 99.2: 0.8 to 99.9:0.1; from 99.3:0.7 to 99.9:0.1, from 99.4:0.6 to 99.9:0.1, from 99.5:0.5 to 99.9:0.1, from 99.6:0.4 to 99.9:0.1, from 99.7:0.3 to 99.9:0.1, or from 99.8:0.2 to 99.9:0.1.

17. The condom of any one of claims 1-16, wherein the adhesive layer is transparent.

## Patentansprüche

1. Kondom, umfassend:
eine Barriereschicht, die geeignet ist, einen Durchgang von Sperma zu verhindern und eine innere Oberfläche und eine äußere Oberfläche umfasst, und
eine Haftschicht, die an mindestens einem Abschnitt der inneren Oberfläche der Barriereschicht haftet; wobei:
das Kondom so ausgebildet ist, dass die Haftschicht an der Eichel des Penis eines menschlichen Subjekts haftet,
das Kondom so ausgebildet ist, dass es eine fraktionierte Abdeckung des Penis bereitstellt, und
die Haftschicht einen Haftklebstoff umfasst, der ein stimuli-responsives Polymer umfasst, das aus einem oder mehreren Monomeren und einem oder mehreren polyfunktionellen Vernetzern gebildet ist, wobei das stimuli-responsive Polymer ein vernetztes Polymer ist, das ein heterogenes Vernetzungsnetzwerk aufweist, und wobei
beim Aufbringen des Kondoms auf den Penis die Haftschicht an der Eichel des Penis haftet und
die Haftschicht nach einer Aufbringung eines Stimulus auf das Kondom von der Eichel des Penis entfernt werden kann.

2. Kondom nach Anspruch 1, wobei das Kondom so bemessen und geformt ist, dass das Kondom, wenn es auf den Penis aufgebracht wird, den Eichelkranz und/oder den Schaft des Penis nicht berührt.

3. Kondom nach Anspruch 1 oder 2, wobei die Barriereschicht: eine Membran oder einen Film umfasst; und/oder
Folgendes umfasst: Naturlatexkautschuk, Synthesekautschuk, amorphes Polyurethan, semikristalline Polyurethane (einschließlich verschiedener thermoplastischer Polyurethane, Polyethylen, Polypropylen, Polydimethylsiloxan und anderer Silikonkautschuke), Polyethylenterephthalat, Poly(vinylchlorid), Polyisopren, vulkanisiertes Polyisopren oder andere vulkanisierte oder vernetzte Kautschuke, Ethylenvinylacetat, Poly(vinylacetat), elastomere oder flexible Materialien oder eine Kombination davon, bevorzugt Naturlatexkautschuk, Synthesekautschuk oder Polyurethane; und/oder
eine Dicke von Folgendem aufweist: 0,001 mm bis 2 mm, z. B. von 0,001 mm bis 1,5 mm, von 0,001 mm bis 1 mm, von 0,001 mm bis 0,5 mm, von 0,001 mm bis 0,1 mm, von 0,001 mm bis 0,01 mm, von 0,025 mm bis 0,25 mm, wie zum Beispiel von 0,025 mm bis 0,2 mm, von 0,025 mm bis 0,15 mm, von 0,025 mm bis 0,1 mm oder von 0,025 mm bis 0,05 mm.

4. Kondom nach einem der Ansprüche 1-3, wobei die Barriereschicht ferner ein Reservoir umfasst, das geeignet bemessen und geformt ist, um Sperma zu sammeln, der aus dem Penis ejakuliert wird, wobei das Reservoir bevorzugt distal zu der Harnröhrenöffnung des Penis ausgebildet ist.

5. Kondom nach Anspruch 4, wobei das Reservoir kugelförmig oder zylindrisch ist und/oder
wobei sich das Reservoir selbst bildet, wenn es dem Druck der Ejakulation durch den Penis ausgesetzt ist, und/oder
wobei das Reservoir eine Polymerbeschichtung umfasst, die bei Kontakt mit Sperma quillt oder geliert,
wobei die Polymerbeschichtung bevorzugt Chitosan, Alginat, Polyacrylsäure, vernetzte Polyacrylsäure, Natriumpolyacrylat, vernetztes Natriumpolyacrylat oder eine Kombination davon umfasst.

6. Kondom nach einem der Ansprüche 1-5, wobei das Kondom oder die Barriereschicht eine ebene oder gekrümmte Geometrie aufweist, die aus einem Quadrat, einem Kreis, einem Oval, einer Halbkugel, einem Rechteck, einem Polygon oder einem gekrümmten Polygon ausgewählt ist, bevorzugt:
wobei die Barriereschicht eine Kreisgeometrie aufweist,
wobei bevorzugt der Radius des Kreises mindestens etwa 0,5 cm beträgt, z. B. etwa 1,0 cm, etwa 2,0 cm, etwa 3,0 cm oder etwa 5,0 cm; oder
wobei die Barriereschicht eine Rechteckgeometrie aufweist,
wobei das Rechteck bevorzugt eine Länge von 0,5 cm bis 5 cm und eine Breite von 0,5 cm bis 5 cm aufweist, und/oder
wobei das Kondom oder die Barriereschicht planar ist.

7. Kondom nach einem der Ansprüche 1-6, ferner umfassend: ein Gleitmittel auf der äußeren Oberfläche der Barriereschicht, bevorzugt
wobei das Gleitmittel aus Folgendes ausgewählt ist: einem Gleitmittel auf Wasserbasis, einem Gleitmittel auf Siliciumbasis, einem Gleitmittel auf Polysaccharidbasis, einem natürlichen Gleitmittel und einem Gleitmittel auf Ölbasis; und/oder
ein Spermizid auf der äußeren Oberfläche der Barriereschicht in Form einer Lösung oder eines Gels, bevorzugt
wobei das Spermizid aus Nonoxynol-9, Octoxynol-9, Benzalkoniumchlorid, Milchsäure, Menfegol und Kombinationen davon ausgewählt ist.

8. Kondom nach einem der Ansprüche 1-7, ferner umfassend einen Elastomerring, der an dem Außenabschnitt der inneren Oberfläche der Barriereschicht oder dem Rand der Barriereschicht befestigt ist, bevorzugt
ferner umfassend einen oder mehrere vorstehende Arme, die mit dem Elastomerring verbunden sind.

9. Kondom nach einem der Ansprüche 1-8, wobei die Haftschicht: nur an einem Abschnitt der inneren Oberfläche der Barriereschicht haftet, bevorzugt
wobei die Haftschicht an dem Außenabschnitt der inneren Oberfläche der Barriereschicht haftet; oder
mit der inneren Oberfläche der Barriereschicht koextensiv ist.

10. Kondom nach einem der Ansprüche 1-9, wobei die Haftschicht in einer gemusterten Ausbildung vorliegt und/oder
wobei die Haftschicht eine Dicke von 0,1 Mikrometern bis 3.000 Mikrometern aufweist, z. B. von 1 Mikrometer bis 2.000 Mikrometern, von 25 Mikrometern bis 1.000 Mikrometern, von 25 Mikrometern bis 750 Mikrometern oder von 25 Mikrometern bis 500 Mikrometern.

11. Kondom nach einem der Ansprüche 1-10, wobei das stimuli-responsive Polymer eine Glasübergangstemperatur (Tg) von -100 bis 100 °C aufweist, bevorzugt von 0 °C bis 50 °C, bevorzugt von 0 °C bis 40 °C, bevorzugter von 5 °C bis 40 °C.

12. Kondom nach einem oder mehreren der Ansprüche 1-11, wobei das vernetzte Polymer eine oder mehrere C6- bis C30-Seitenketten oder ein oder mehrere freie C6- bis C30-Kettenenden umfasst, bevorzugt wobei die C6- bis C30-Seitenketten oder freie C6- bis C30-Kettenenden C6-bis C30-Alkylseitenketten sind, bevorzugt C12- bis C18-Alkylseitenketten.

13. Kondom nach einem der Ansprüche 1-12, wobei das vernetzte Polymer ein semiinterpenetrierendes Netzwerk ist oder das vernetzte Copolymer ein interpenetrierendes Netzwerk ist.

14. Kondom nach einem der Ansprüche 1-13, wobei das stimuli-responsive Polymer Monomere umfasst, die aus Folgenden ausgewählt sind: Acrylatmonomeren, Methacrylatmonomeren, Vinylethermonomeren, Allylmonomeren, Thiolmonomeren, Epoxidmonomeren, Aminmonomeren, elektronenreichen Monomeren, elektronenarmen Monomeren, Lactammonomeren, Lactonmonomeren, Alkoholmonomeren, Carbonsäuremonomeren, Isocyanatmonomeren, Diels-Alder-Monomeren, Ringöffnungsmetathesemonomeren oder einer Kombination davon, bevorzugt:
wobei das stimuli-responsive Polymer (i) Acrylatmonomere umfasst, bevorzugt:
wobei die Acrylatmonomere C6-C30-Alkylacrylatmonomere sind, bevorzugt C8-C20-Alkylacrylatmonomere, bevorzugt C8-C20-Alkylacrylatmonomere, bevorzugter C8-C16-Alkylacrylatmonomere, bevorzugt
wobei die C8-C30-Alkylacrylatmonomere aus Folgendem ausgewählt sind: Octylacrylat, Nonylacrylat, Decylacrylat, Undecylacrylat, Dodecylacrylat, Tridecylacrylat, Tetradecylacrylat, Pentadecylacrylat, Hexadecylacrylat, Heptadecylacrylat, Octadecylacrylat, Nonadecylacrylat, Eicosylacrylat, Heneicosylacrylat, Docosylacrylat, Tricosylacrylat, Tetracosylacrylat, Pentacosylacrylat, Hexacosylacrylat, Heptacosylacrylat, Octacosylacrylat, Nonacosylacrylat, Triacontylacrylat und Kombinationen davon; oder
wobei die Acrylatmonomere aus Folgendem ausgewählt sind: Methylacrylat, Ethylacrylat, Butylacrylat, 2-Ethylhexylacrylat, Isobutylacrylat, Methoxyethylacrylat, Hydroxyethylacrylat, Hydroxypropylacrylat, ethoxyliertem (2)-Hydroxyethylacrylat, Glycidylacrylat, Methacrylsäure, Methylmethacrylat, Ethylmethacrylat, Butylmethacrylat, 2-Hydroxyethylmethacrylat, Cyclohexylmethacrylat, Poly(ethylenglycol)methacrylat, Poly(ethylenglycol)diacrylat, Poly(ethylenglycol)dimethacrylat, Trimethylolpropantriacrylat, Triethylenglycoldiacrylat, Tetraethylenglycoldiacrylat, Neopentylglycoldiacrylat, Diethylenglycoldiacrylat, Dipentaerythrithexaacrylat, ethoxyliertem Trimethylolpropantriacrylat, propoxyliertem Glycerintriacrylat, Stearylacrylat, Laurylacrylat, Isodecylacrylat, Acrylsäure, Ethylenglycoldiacrylat (EGDA), Triethylenglycoldiacrylat (TEGDA), Propylenglycoldiacrylat (PGDA), Butandioldiacrylat (BDDA), Neopentylglycoldiacrylat (NPGDA), Pentaerythritoltetraacrylat (PETA), 1,4-Butandioldiacrylat (BDA), Di(trimethylolpropan)tetraacrylat (DTMPTA), Bisphenol-A-ethoxylatdiacrylat (BPAEDA), ethoxyliertes Bisphenol-A-diacrylat (EBPA), Decandioldiacrylat, Polyethylenglycoldiacrylat (PEGDA), Trimethylolpropantriacrylat (TMPTA), Diethylenglycoldiacrylat (DEGDA) und 1,6-Hexandioldiacrylat (HDDA), Trimethylolpropantriacrylat (TMPTA), Tripropylenglycoldiacrylat (TPGDA), Pentaerythritoltriacrylat (PETA), Dipentaerythritolpentaacrylat (DPEPA), Tris(2-hydroxyethyl)isocyanurattriacrylat (THEIC-TA), Triethylenglycoldiacrylat (TEGDMA), Triallylisocyanurat (TAIC), Triethylenglycoldiacrylat (TEGDA), ethoxyliertes Trimethylolpropandiacrylat (ETMPTA), Triallylcyanurat (TAC) und Kombinationen davon und/oder
wobei das stimuli-responsive Polymer mindestens 10 Gew.-% Acrylatmonomere umfasst, z. B. mindestens 50 Gew.-%, mindestens 65 Gew.-%, mindestens 95 Gew.-%, mindestens 96 Gew.-%, mindestens 97 Gew.-%, mindestens 98 Gew.-% oder mindestens 99 Gew.-%;
(ii) Methacrylatmonomeren, bevorzugt:
wobei die Methacrylatmonomere C6-C30-Alkylmethacrylatmonomere sind, bevorzugt C8-C30-Alkylmethacrylatmonomere, bevorzugter C8-C20-Alkylmethacrylatmonomere, bevorzugter C8-C16-Alkylmethacrylatmonomere, bevorzugt
wobei die C8-C30-Alkylmethacrylatmonomere aus Folgendem ausgewählt sind: Octylmethacrylat, Nonylmethacrylat, Decylmethacrylat, Undecylmethacrylat, Dodecylmethacrylat, Tridecylmethacrylat, Tetradecylmethacrylat, Pentadecylmethacrylat, Hexadecylmethacrylat, Heptadecylmethacrylat, Octadecylmethacrylat, Nonadecylmethacrylat, Eicosylmethacrylat, Heneicosylmethacrylat, Docosylmethacrylat, Tricosylmethacrylat, Tetracosylmethacrylat, Pentacosylmethacrylat, Hexacosylmethacrylat, Heptacosylmethacrylat, Octacosylmethacrylat, Nonacosylmethacrylat, Triacontylmethacrylat und Kombinationen davon; oder
wobei die Methacrylatmonomere aus Folgendem ausgewählt sind: Ethylmethacrylat, Butylmethacrylat, 2-Hydroxyethylmethacrylat, Cyclohexylmethacrylat, Isobornylmethacrylat, Stearylmethacrylat, Laurylmethacrylat, Isodecylmethacrylat, Tetrahydrofurfurylmethacrylat, Glycerylmethacrylat, Trimethylolpropantrimethacrylat, Trimethylolpropantriacrylat, Pentaerythrittriacrylat, Pentaerythrittetramethacrylat, Poly(ethylenglycol)monomethylethermethacrylat, Poly(ethylenglycol)monomethyletheracrylat, Poly(ethylenglycol)diacrylat, Poly(ethylenglycol)dimethacrylat, Poly(ethylenglycol)monoacrylat, ethoxyliertem Bisphenol-A-dimethacrylat, ethoxyliertes Bisphenol-A-diacrylat, ethoxyliertes Trimethylolpropantriacrylat, Hydroxypropylmethacrylat, Methacrylsäure, Acryloyloxyethyltrimethylammoniumchlorid, Diethylaminoethylmethacrylat, Butylaminoethylmethacrylat, N,N-Dimethylaminoethylmethacrylat, Methacryloyloxyethylphthalat, Cyclopropylmethacrylat und Kombinationen davon und/oder
wobei das stimuli-responsive Polymer Poly(laurylmethacrylat) umfasst oder
wobei das stimuli-responsive Polymer Poly(laurylmethacrylat) und ein oder mehrere andere Polymethacrylate umfasst oder
wobei der stimuli-responsive Polymerhaftklebstoff Poly(laurylmethacrylat) und ein oder mehrere Polyacrylate umfasst und/oder
wobei das stimuli-responsive Polymer mindestens 10 Gew.-% Methacrylatmonomere umfasst, z. B. mindestens 50 Gew.-%, mindestens 65 Gew.-%, mindestens 95 Gew.-%, mindestens 96 Gew.-%, mindestens 97 Gew.-%, mindestens 98 Gew.-% oder mindestens 99 Gew.-%;
(iii) Vinylethermonomeren, ausgewählt aus Divinylether von Ethylenglykol, Divinylether von Diethylenglykol, Divinylether von Triethylenglykol, Divinylether von Polyethylenglykol (DVE-PEG), Divinylether von Polypropylenglykol (DVE-PPG), Divinylether von Poly(ethylenglykol)methylether (DVE-PEGME), Divinylether von Poly(ethylenglykol)butylether (DVE), Divinylether von Poly(ethylenglykol)phenylether (DVE-PEGPhE), Divinylether von Glycerin (DVE-Gly), Divinylether von 1,4-Cyclohexandimethanol (DVE-CHDM), Divinylether von Neopentylglykol (DVE-NPG) und Kombinationen davon, bevorzugt
wobei das stimuli-responsive Polymer ferner Folgendes umfasst: Acrylatmonomere, Methacrylatmonomere, Allylmonomere, Thiolmonomere, Epoxymonomere, Aminmonomere, elektronenreiche Monomere, elektronenarme Monomere, Lactammonomere, Lactonmonomere, Alkoholmonomere, Carbonsäuremonomere, Isocyanatmonomere, Diels-Alder-Monomere, Ringöffnungsmetathesemonomere oder eine Kombination davon;
(iv) Allylmonomeren, ausgewählt aus Diallylphthalat (DAP), Diallylmaleat (DAM), Diallylsuccinat (DAS), Diallylfumarat (DAF), Diallyladipat (DAA), Diallylsebacat (DAS), Diallylterephthalat (DAT), Diallylisophthalat (DAI), Diallylitaconat (DAI), Diallylcarbonat (DAC), Diallyldiglykolat (DADG), Diallyltris(2-hydroxyethyl)isocyanurat (DATHEIC), Triallylcyanurat (TAC), Triallylisocyanurat (TAIC), Triallyltrimellitat (TATM), Triallylcitrat (TAC), Triallylphosphat (TAP), Triallylamin (TAA), Triallylcyanid (TACN), Triallylbenzol-1,2,4-tricarboxylat (TABTC), Triallyltrimesat (TATM), Tris(2-hydroxyethyl)isocyanurattriallylether (THEIC-TAE) und Kombinationen davon, bevorzugt
wobei das stimuli-responsive Polymer ferner Folgendes umfasst: Acrylatmonomere, Methacrylatmonomere, Vinylethermonomer, Thiolmonomere, Epoxymonomere, Aminmonomere, elektronenreiche Monomere, elektronenarme Monomere, Lactammonomere, Lactonmonomere, Alkoholmonomere, Carbonsäuremonomere, Isocyanatmonomere, Diels-Alder-Monomere, Ringöffnungsmetathesemonomere oder eine Kombination davon;
(v) Thiolmonomeren, ausgewählt aus 3-Mercaptopropionsäure; Thioglykolsäure; 3-Mercapto-1-propanol; 2-Mercaptoethanol; 2-(2-Mercaptoethoxy)ethanol; 2-(2-Mercaptopropionylamino)ethanol; 2-(2-Mercaptosuccinyl)ethylacrylat; 3-(2-Mercaptopropionylamino)propionsäure; 3-(Mercaptopropyl)trimethoxysilan; 2,2'-(Ethylenbis(thio))diethanol; 3-Mercaptopropyltrimethoxysilan; 3-Mercaptopropylmethyldimethoxysilan; 3-(2,2-Dithiobis(ethylthio)propionylamino)propionsäure; 3,6,9-Trioxadecanethiol; 3-Mercapto-1,2-propandiol; 2,2'-Dithiodiethanol; N-Acetyl-L-cystein; L-Cystein; 2-(2-Mercaptoethyl)pyridin; 4-(2-Mercaptoethyl)morpholin; 3-Mercapto-1,2,4-triazol; Thiophenol; Pentaerythritol-tetrakis(3-mercaptopropionat) (PETMP); Trimethylolpropan-tris(3-mercaptopropionat) (TMPMP); Triethanolamin-tris(3-mercaptopropionat) (TEAMP); Tris(2-hydroxyethyl)isocyanurat-tris(3-mercaptopropionat) (THEICMP); Bis(3-mercaptopropyl)sulfid (BMPS); 1,2-Ethanedithiol (EDT); 1,3-Propanedithiol; 1,4-Butanedithiol; 1,6-Hexanedithiol; 1,8-Octanedithiol und Kombinationen davon, bevorzugt
wobei das stimuli-responsive Polymer ferner Folgendes umfasst: Acrylatmonomere, Methacrylatmonomere, Vinylethermonomer, Allylmonomere, Epoxymonomere, Aminmonomere, elektronenreiche Monomere, elektronenarme Monomere, Lactammonomere, Lactonmonomere, Alkoholmonomere, Carbonsäuremonomere, Isocyanatmonomere, Diels-Alder-Monomere, Ringöffnungsmetathesemonomere oder eine Kombination davon;
(vi) Epoxidmonomeren, ausgewählt aus Bisphenol-A-Diglycidylether (BADGE), Bisphenol-F-Diglycidylether (BFDGE), Novolac-Diglycidylether (NGDE), Phenol-Novolac-diglycidylether (PNGDE), cycloaliphatischen Epoxidharzen, Glycidylethern aliphatischer Alkohole, Glycidylethern aromatischer Alkohole, Triglycidylisocyanurat (TGIC), Diglycidylether von 1,4-Butandiol (BDDGE), Diglycidylether von Neopentylglykol (NPGDGE), Diglycidylether von Propylenglykol (PGDGE), epoxidiertem Sojaöl (ESO), epoxidiertem Leinöl (ELO), Epoxidharzen auf Dicyclopentadienbasis, tetrafunktionellen Epoxidharzen, Epoxid-Phenol-Novolak-Harzen und Kombinationen davon, bevorzugt
ferner umfassend Acrylatmonomere, Methacrylatmonomere, Vinylethermonomer, Allylmonomere, Thiolmonomere, Aminmonomere, elektronenreiche Monomere, elektronenarme Monomere, Lactammonomere, Lactonmonomere, Alkoholmonomere, Carbonsäuremonomere, Isocyanatmonomere, Diels-Alder-Monomere, Ringöffnungsmetathesemonomere oder eine Kombination davon;
(vii) Aminmonomeren, ausgewählt aus Ethylendiamin; Diethylentriamin; Triethylentetramin; Tetraethylenpentamin; Polyethylenimin; Diaminopropan; Diaminobutan; Diaminopentan; Diethylentriaminpentaessigsäure (DTPA); Tris(2-aminoethyl)amin; N-(2-Aminoethyl)piperazin; N-(3-Aminopropyl)morpholin; N,N-Dimethylaminopropylamin; N,N-Dimethylethylendiamin; 1,3-Diaminopropan; Isophorondiamin; Jeffamin D-230; Jeffamin T-403; Jeffamin M-207; Jeffamin EDR-148; und Kombinationen davon, bevorzugt
wobei das stimuli-responsive Polymer ferner Folgendes umfasst: Acrylatmonomere, Methacrylatmonomere, Vinylethermonomer, Allylmonomere, Thiolmonomere, Epoxymonomere, elektronenreiche Monomere, elektronenarme Monomere, Lactammonomere, Lactonmonomere, Alkoholmonomere, Carbonsäuremonomere, Isocyanatmonomere, Diels-Alder-Monomere, Ringöffnungsmetathesemonomere oder eine Kombination davon;
(viii) elektronenreichen Monomeren, ausgewählt aus Vinylethern (z. B. Vinylmethylether, Vinylethylether), Vinylacetat, Allylalkohol, Allylamin, N-Methylolacrylamid, N-Methylolmethacrylamid, N-Methylolallylamin, N-Methylolvinylacetamid, Acroleindiethylacetal, Acroleindiethylketal, Diacetonacrylamid, 2-Hydroxyethylacrylat, 2-Hydroxyethylmethacrylat, 2-Hydroxypropylacrylat, 2-Hydroxypropylmethacrylat, 2,3-Dihydroxypropylmethacrylat, Glycidylmethacrylat, Glycidylacrylat, Tetrahydrofurfurylmethacrylat und N-Vinylpyrrolidon, n-Vinylformamid, n-Vinylpyridin, Styrol, Styrolderivate und Kombinationen davon, bevorzugt
wobei das stimuli-responsive Polymer ferner Folgendes umfasst: Acrylatmonomere, Methacrylatmonomere, Vinylethermonomer, Allylmonomere, Thiolmonomere, Epoxymonomere, Aminmonomere, elektronenarme Monomere, Lactammonomere, Lactonmonomere, Alkoholmonomere, Carbonsäuremonomere, Isocyanatmonomere, Diels-Alder-Monomere, Ringöffnungsmetathesemonomere oder eine Kombination davon;
(ix) elektronenarmen Monomeren, ausgewählt aus Acrylnitril, Methacrylnitril, Methylmethacrylat, Acrylsäure, Methacrylsäure, Maleinsäureanhydrid, Itaconsäure, Fumarsäure, Acrylamid, Methacrylamid, N-Vinylcarbazol, Vinylidenchlorid, Vinylchlorid, Vinylsulfonsäure, Vinylacetat, Styrol, Alpha-Methylstyrol, Maleimid, N-Phenylmaleinimid und N-Butylmaleinimid, Maleinsäureanhydrid und Kombinationen davon, bevorzugt
wobei das stimuli-responsive Polymer ferner Folgendes umfasst: Acrylatmonomere, Methacrylatmonomere, Vinylethermonomer, Allylmonomere, Thiolmonomere, Epoxymonomere, Aminmonomere, elektronenreiche Monomere, Lactammonomere, Lactonmonomere, Alkoholmonomere, Carbonsäuremonomere, Isocyanatmonomere, Diels-Alder-Monomere, Ringöffnungsmetathesemonomere oder eine Kombination davon;
(x) Lactammonomeren, ausgewählt aus Caprolactam, Valerolactam, Enantholactam, Capryllactam, Laurinlactam, Prolactam, Butyrolactam, Methionyllactam, Methoxyethyllactam, Methoxyethylmethionyllactam, Dimethylaminoethyllactam, Dimethylaminoethylmethionyllactam, Dimethylaminoethylacryloyllactam, Dimethylaminoethylmethacryloyllactam, N-Vinylpyrrolidon, N-Methylpyrrolidon, N-Ethylpyrrolidon und Kombinationen davon, bevorzugt
wobei das stimuli-responsive Polymer Folgendes umfasst: Acrylatmonomere, Methacrylatmonomere, Vinylethermonomer, Allylmonomere, Thiolmonomere, Epoxymonomere, Aminmonomere, elektronenreiche Monomere, elektronenarme Monomere, Lactonmonomere, Alkoholmonomere, Carbonsäuremonomere, Isocyanatmonomere, Diels-Alder-Monomere, Ringöffnungsmetathesemonomere oder eine Kombination davon;
(xi) Lactonmonomeren, ausgewählt aus β-Propiolacton, γ-Butyrolacton, 8-Valerolacton, ε-Caprolacton, ω-Pentadecalacton, β-Butyrolacton, 8-Decalacton, ε-Decalacton, γ-Decalacton, 8-Dodecalacton, γ-Dodecalacton, α-Methylen-γ-butyrolacton, β-Methyl-γ-butyrolacton, β-Methyl-γ-valerolacton, γ-Hexalacton und Kombinationen davon, bevorzugt
wobei das stimuli-responsive Polymer ferner Folgendes umfasst: Acrylatmonomere, Methacrylatmonomere, Vinylethermonomer, Allylmonomere, Thiolmonomere, Epoxymonomere, Aminmonomere, elektronenreiche Monomere, elektronenarme Monomere, Lactammonomere, Alkoholmonomere, Carbonsäuremonomere, Isocyanatmonomere, Diels-Alder-Monomere, Ringöffnungsmetathesemonomere oder eine Kombination davon;
(xii) Alkoholmonomeren, ausgewählt aus Ethylenglycol; Propylenglycol; 1,3-Butandiol; 1,4-Butandiol; 1,5-Pentandiol; 1,6-Hexandiol; 1,10-Decandiol; Neopentylglycol; Diethylenglycol; Triethylenglycol; Tetraethylenglycol; Polyethylenglycol (PEG); Polypropylenglycol (PPG); Polycaprolactondiol; Polymethylolpropan; Hydroxypivalylhydroxymethylbutyrat (HPHMB); 1,4-Cyclohexandimethanol; und Kombinationen davon, bevorzugt
wobei das stimuli-responsive Polymer ferner Folgendes umfasst: Acrylatmonomere, Methacrylatmonomere, Vinylethermonomer, Allylmonomere, Thiolmonomere, Epoxymonomere, Aminmonomere, elektronenreiche Monomere, elektronenarme Monomere, Lactammonomere, Lactonmonomere, Carbonsäuremonomere, Isocyanatmonomere, Diels-Alder-Monomere, Ringöffnungsmetathesemonomere oder eine Kombination davon;
(xiii) Carbonsäuremonomeren, ausgewählt aus Adipinsäure, Bernsteinsäure, Glutarsäure, Sebacinsäure, Malonsäure, Phthalsäure, Isophthalsäure, Terephthalsäure, Fumarsäure, Maleinsäure, Itaconsäure, Zitronensäure, 1,4-Cyclohexandicarbonsäure, 1,3-Cyclohexandicarbonsäure, Dodecandisäure und Kombinationen davon, bevorzugt
wobei das stimuli-responsive Polymer ferner Folgendes umfasst: Acrylatmonomere, Methacrylatmonomere, Vinylethermonomer, Allylmonomere, Thiolmonomere, Epoxymonomere, Aminmonomere, elektronenreiche Monomere, elektronenarme Monomere, Lactammonomere, Lactonmonomere, Alkoholmonomere, Isocyanatmonomere, Diels-Alder-Monomere, Ringöffnungsmetathesemonomere oder eine Kombination davon;
(xiv) Isocyanatmonomeren, ausgewählt aus Toluylendiisocyanat (TDI), Diphenylmethandiisocyanat (MDI), Hexamethylendiisocyanat (HDI), Isophorondiisocyanat (IPDI), 1,6-Hexamethylendiisocyanat (HMDI), 4,4'-Methylenbis(cyclohexylisocyanat) (H12MDI), Naphthalindiisocyanat (NDI), 2,4-Toluylendiisocyanat (2,4-TDI), 2,6-Toluylendiisocyanat (2,6-TDI), Polymethylenpolyphenylisocyanat (PAPI), Desmodur N-100, Desmodur L-75, Desmodur HL, Desmodur H, Desmodur VP, Desmodur Z und Kombinationen davon, bevorzugt
wobei das stimuli-responsive Polymer ferner Folgendes umfasst: Acrylatmonomere, Methacrylatmonomere, Vinylethermonomer, Allylmonomere, Thiolmonomere, Epoxymonomere, Aminmonomere, elektronenreiche Monomere, elektronenarme Monomere, Lactammonomere, Lactonmonomere, Alkoholmonomere, Carbonsäuremonomere, Diels-Alder-Monomere, Ringöffnungsmetathesemonomere oder eine Kombination davon;
(xv) Diels-Alder-Monomeren, ausgewählt aus Maleinsäureanhydrid, Furan, Cyclopentadien, N-Phenylmaleinimid, Anthracen, N-Ethylmaleinimid, N-Phenylnorbornen, N,N-Dimethylmaleinimid, 2,5-Dimethylfuran, Tetracyanoethylen, Methylvinylketon und Kombinationen davon, bevorzugt
wobei das stimuli-responsive Polymer ferner Folgendes umfasst: Acrylatmonomere, Methacrylatmonomere, Vinylethermonomer, Allylmonomere, Thiolmonomere, Epoxymonomere, Aminmonomere, elektronenreiche Monomere, elektronenarme Monomere, Lactammonomere, Lactonmonomere, Alkoholmonomere, Carbonsäuremonomere, Isocyanatmonomere, Ringöffnungsmetathesemonomere oder eine Kombination davon; oder
(xvi) Ringöffnungsmetathesemonomeren, ausgewählt aus Norbornen, Dicyclopentadien (DCPD), Cycloocten, Tetracyclododecen (TCD), Cyclopenten, Cyclohepten, Cyclohexen, Bicyclo[2.2.1]hept-2-en, Bicyclo[2.2.2]oct-5-en, Tricyclo[5.2.1.0(2,6)]dec-8-en (TCD-Dien) und Kombinationen davon, bevorzugt
wobei das stimuli-responsive Polymer ferner Folgendes umfasst: Acrylatmonomere, Methacrylatmonomere, Vinylethermonomer, Allylmonomere, Thiolmonomere, Epoxymonomere, Aminmonomere, elektronenreiche Monomere, elektronenarme Monomere, Lactammonomere, Lactonmonomere, Alkoholmonomere, Carbonsäuremonomere, Isocyanatmonomere, Diels-Alder-Monomere oder eine Kombination davon.

15. Kondom nach einem der Ansprüche 1-14, wobei der polyfunktionelle Vernetzer aus Folgendem ausgewählt ist: einem difunktionellen Vernetzer, einem trifunktionellen Vernetzer oder einem tetrafunktionellen Vernetzer, bevorzugt
wobei der polyfunktionelle Vernetzer ein trifunktioneller Vernetzer ist, bevorzugt
wobei der trifunktionelle Vernetzer ein Acrylatvernetzer ist;
Poly(ethylenglykol)diacrylat, Trimethylolpropantriacrylat; ethoxyliertes Trimethyolpropantriacrylat; Pentaerythritoltetraacrylat; ethoxyliertes Pentaerythritoltetraacrylat; Dipentaerithrotolhexaacrylat; ethoxyliertes Dipentaerithrotolhexaacrylat; Di-, Tri-, Tetra-, Penta-oder Hexaepoxide; Polythiole; Polyalkene; Tris(2-acryloxyethyl)isocyanat, ε-Caprolactonmodifiziertes Tris(2-acryloxyethyl)isocyanurat, ethoxyliertes Glycerintriacrylat, ethoxyliertes Glycerintriacrylat, Pentaerythritoltriacrylat und Kombinationen davon und/oder
wobei das stimuli-responsive Polymer 0,001 bis 29 Gew.-% umfasst, bevorzugt 0,1 Gew.-
% bis 1,5 Gew.-% polyfunktionellen Vernetzer, bevorzugt 0,2 Gew.-% bis 1 Gew.-%, bevorzugter 0,4 Gew.-% bis 0,8 Gew.-%, und/oder
wobei das Gewichtsverhältnis des einen oder der mehreren Monomere zu dem einen oder den mehreren polyfunktionellen Vernetzern von 98:2 bis 99,9:0,1 beträgt, z. B. von 98,5:1,5 bis 99,9:0,1; von 99:1 bis 99,9:0,1, von 99,1:0,9 bis 99,9:0,1, von 99,2:0,8 bis 99,9:0,1; von 99,3:0,7 bis 99,9:0,1, von 99,4:0,6 bis 99,9:0,1, von 99,5:0,5 bis 99,9:0,1, von 99,6:0,4 bis 99,9:0,1, von 99,7:0,3 bis 99,9:0,1 oder von 99,8:0,2 bis 99,9:0,1.

16. Kondom nach Anspruch 1, wobei das stimuli-responsive Polymer ein Poly(laurylmethacrylat)-Polymer umfasst, das mit einem oder mehreren polyfunktionellen Vernetzern vernetzt ist, bevorzugt:
wobei der polyfunktionelle Vernetzer aus einem difunktionellen Vernetzer, einem trifunktionellen Vernetzer oder einem tetrafunktionellen Vernetzer ausgewählt ist, bevorzugt
wobei der polyfunktionelle Vernetzer ein trifunktioneller Vernetzer ist, bevorzugt
wobei der trifunktionelle Vernetzer ein Acrylatvernetzer ist; oder
wobei der polyfunktionelle Vernetzer aus Folgendem ausgewählt ist: Poly(ethylenglykol)diacrylat, Trimethylolpropantriacrylat; ethoxyliertes Trimethyolpropantriacrylat; Pentaerythritoltetraacrylat; ethoxyliertes Pentaerythritoltetraacrylat; Dipentaerithrotolhexaacrylat; ethoxyliertes Dipentaerithrotolhexaacrylat; Di-, Tri-, Tetra-, Penta-oder Hexaepoxide; Polythiole; Polyalkene; Tris(2-acryloxyethyl)isocyanat, ε-Caprolactonmodifiziertes Tris(2-acryloxyethyl)isocyanurat, ethoxyliertes Glycerintriacrylat, ethoxyliertes Glycerintriacrylat, Pentaerythritoltriacrylat und Kombinationen davon und/oder
wobei das Gewichtsverhältnis des Laurylmethacrylats zu dem einen oder den mehreren polyfunktionellen Vernetzern von 98:2 bis 99,9:0,1 beträgt, z. B. von 98,5:1,5 bis 99,9:0,1; von 99:1 bis 99,9:0,1, von 99,1:0,9 bis 99,9:0,1, von 99,2:0,8 bis 99,9:0,1; von 99,3:0,7 bis 99,9:0,1, von 99,4:0,6 bis 99,9:0,1, von 99,5:0,5 bis 99,9:0,1, von 99,6:0,4 bis 99,9:0,1, von 99,7:0,3 bis 99,9:0,1 oder von 99,8:0,2 bis 99,9:0,1.

17. Kondom nach einem der Ansprüche 1-16, wobei die Haftschicht transparent ist.

## Revendications

1. Préservatif comprenant :
une couche barrière appropriée pour interdire le passage du sperme et comprenant une surface interne et une surface externe, et
une couche adhésive collée à au moins une partie de la surface interne de la couche barrière :
ledit préservatif étant conçu pour l'adhérence de la couche adhésive au gland du pénis d'un sujet humain,
ledit préservatif étant conçu pour fournir une couverture fractionnée du pénis, et
ladite couche adhésive comprenant un adhésif qui comprend un polymère sensible aux stimuli formé d'un ou de plusieurs monomères et d'un ou de plusieurs agents de réticulation polyfonctionnels, ledit polymère sensible aux stimuli étant un polymère réticulé présentant un réseau de réticulation hétérogène, et
lorsque ledit préservatif est appliqué sur le pénis, ladite couche adhésive adhérant au gland du pénis, et
ladite couche adhésive pouvant être retirée du gland du pénis après l'application d'un stimulus sur le préservatif.

2. Préservatif selon la revendication 1, ledit préservatif étant dimensionné et formé de sorte que, lorsqu'il est appliqué sur le pénis, le préservatif n'entre pas en contact avec la couronne et/ou le corps du pénis.

3. Préservatif selon la revendication 1 ou 2, ladite couche barrière : comprenant une membrane ou un film ; et/ou
comprenant du caoutchouc de latex naturel, du caoutchouc synthétique, du polyuréthane amorphe, des polyuréthanes semi-cristallins (y compris divers polyuréthanes thermoplastiques, le polyéthylène, le polypropylène, le polydiméthylsiloxane et les autres caoutchoucs de silicone), du polytéréphtalate d'éthylène, du polychlorure de vinyle, du polyisoprène, du polyisoprène vulcanisé ou d'autres caoutchoucs vulcanisés ou réticulés, de l'éthylène-acétate de vinyle, du polyacétate de vinyle, des matériaux élastomères ou souples, ou une combinaison de ceux-ci, de préférence
du caoutchouc de latex naturel, du caoutchouc synthétique ou des polyuréthanes ; et/ou
présentant une épaisseur de 0,001 mm à 2 mm, par exemple de 0,001 mm à 1,5 mm, de 0,001 mm à 1 mm, de 0,001 mm à 0,5 mm, de 0,001 mm à 0,1 mm, de 0,001 mm à 0,01 mm, de 0,025 mm à 0,25 mm, comme par exemple de 0,025 mm à 0,2 mm, de 0,025 mm à 0,15 mm, de 0,025 mm à 0,1 mm, ou de 0,025 mm à 0,05 mm.

4. Préservatif selon l'une quelconque des revendications 1 à 3, ladite couche barrière comprenant en outre un réservoir dimensionné et formé de manière appropriée pour recueillir le sperme éjaculé du pénis, de préférence,
ledit réservoir étant conçu de manière distale par rapport à l'ouverture urétrale du pénis.

5. Préservatif selon la revendication 4, ledit réservoir étant sphérique ou cylindrique, et/ou
ledit réservoir se formant automatiquement lorsqu'il est soumis à la pression de l'éjaculation par le pénis, et/ou
ledit réservoir comprenant un revêtement polymère qui gonfle ou gélifie lorsqu'il est mis en contact avec du sperme, de préférence
ledit revêtement polymère comprenant du chitosane, de l'alginate, de l'acide polyacrylique, de l'acide polyacrylique réticulé, du polyacrylate de sodium, du polyacrylate de sodium réticulé ou une combinaison de ceux-ci.

6. Préservatif selon l'une quelconque des revendications 1 à 5, ledit préservatif ou ladite couche barrière présentant une géométrie plane ou incurvée choisie parmi un carré, un cercle, un ovale, un hémisphère, un rectangle, un polygone ou un polygone curviligne, de préférence :
ladite couche barrière présentant une géométrie circulaire, de préférence
ledit rayon du cercle étant d'au moins environ 0,5 cm, par exemple, d'environ 1,0 cm, d'environ 2,0 cm, d'environ 3,0 cm ou d'environ 5,0 cm ; ou
ladite couche barrière présentant une géométrie rectangulaire, de préférence
ledit rectangle présentant une longueur de 0,5 cm à 5 cm et une largeur de 0,5 cm à 5 cm, et/ou
ledit préservatif ou ladite couche barrière étant planaire.

7. Préservatif selon l'une quelconque des revendications 1 à 6, comprenant en outre : un lubrifiant sur la surface externe de la couche barrière, de préférence,
ledit lubrifiant étant choisi parmi un lubrifiant à base d'eau, un lubrifiant à base de silicium, un lubrifiant à base de polysaccharide, un lubrifiant naturel et un lubrifiant à base d'huile ; et/ou
un spermicide sur la surface externe de la couche barrière sous la forme d'une solution ou d'un gel, de préférence,
ledit spermicide étant choisi parmi le nonoxynol-9, l'octoxynol-9, le chlorure de benzalkonium, l'acide lactique, le menfégol et des combinaisons de ceux-ci.

8. Préservatif selon l'une quelconque des revendications 1 à 7, comprenant en outre un anneau élastomère fixé à la partie externe de la surface interne de la couche barrière ou au bord de la couche barrière, comprenant de préférence en outre un ou plusieurs bras saillants reliés à l'anneau élastomère.

9. Préservatif selon l'une quelconque des revendications 1 à 8, ladite couche adhésive étant : collée à seulement une partie de la surface interne de la couche barrière, de préférence,
ladite couche adhésive étant collée à la partie externe de la surface interne de la couche barrière ; ou
coextensif avec la surface interne de la couche barrière.

10. Préservatif selon l'une quelconque des revendications 1 à 9, ladite couche adhésive se trouvant dans une configuration à motifs, et/ou
ladite couche adhésive présentant une épaisseur allant de 0,1 micron à 3 000 microns, par exemple, allant de 1 micron à 2 000 microns, allant de 25 microns à 1 000 microns, allant de 25 microns à 750 microns, ou allant de 25 microns à 500 microns.

11. Préservatif selon l'une quelconque des revendications 1 à 10, ledit polymère sensible aux stimuli présentant une température de transition vitreuse (Tg) de -100 à 100°C, de préférence de 0°C à 50°C, de préférence de 0°C à 40°C, plus préférablement de 5°C à 40°C.

12. Préservatif selon l'une quelconque ou plusieurs des revendications 1 à 11, ledit polymère réticulé comprenant une ou plusieurs chaînes latérales en C6 à C30 ou une ou plusieurs extrémités de chaîne pendantes en C6 à C30, de préférence lesdites chaînes latérales en C6 à C30 ou lesdites extrémités de chaîne pendantes en C6 à C30 étant des chaînes latérales alkyle en C6 à C30, de préférence des chaînes latérales alkyle en C12 à C18.

13. Préservatif selon l'une quelconque des revendications 1 à 12, ledit polymère réticulé étant un réseau semi-interpénétrant ou ledit copolymère réticulé étant un réseau interpénétrant.

14. Préservatif selon l'une quelconque des revendications 1 à 13, ledit polymère sensible aux stimuli comprenant des monomères choisis parmi des monomères d'acrylate, des monomères de méthacrylate, des monomères d'éther vinylique, des monomères d'allyle, des monomères de thiol, des monomères d'époxy, des monomères d'amine, des monomères riches en électrons, des monomères pauvres en électrons, des monomères de lactame, des monomères de lactone, des monomères d'alcool, des monomères d'acide carboxylique, des monomères d'isocyanate, des monomères de Diels-Alder, des monomères de métathèse avec ouverture de cycle, ou une combinaison de ceux-ci, de préférence :
ledit polymère sensible aux stimuli comprenant (i) des monomères d'acrylate, de préférence :
lesdits monomères d'acrylate étant des monomères d'acrylate d'alkyle en C6 à C30, de préférence des monomères d'acrylate d'alkyle en C8 à C20, de préférence des monomères d'acrylate d'alkyle en C8 à C20, plus préférablement des monomères d'acrylate d'alkyle en C8 à C16, de préférence
lesdits monomères d'acrylate d'alkyle en C8 à C30 étant choisis parmi l'acrylate d'octyle, l'acrylate de nonyle, l'acrylate de décyle, l'acrylate d'undécyle, l'acrylate de dodécyle, l'acrylate de tridécyle, l'acrylate de tétradécyle, l'acrylate de pentadécyle, l'acrylate d'hexadécyle, l'acrylate d'heptadécyle, l'acrylate d'octadécyle, l'acrylate de nonadécyle, l'acrylate d'éicosyle, l'acrylate d'hénéicosyle, l'acrylate de docosyle, l'acrylate de tricosyle, l'acrylate de tétracosyle, l'acrylate de pentacosyle, l'acrylate d'hexacosyle, l'acrylate d'heptacosyle, l'acrylate d'octacosyle, l'acrylate de nonacosyle, l'acrylate de triacontyle, et leurs combinaisons ; ou
lesdits monomères d'acrylate étant choisis parmi l'acrylate de méthyle, l'acrylate d'éthyle, l'acrylate de butyle, l'acrylate de 2-éthylhexyle, l'acrylate d'isobutyle, l'acrylate de méthoxyéthyle, l'acrylate d'hydroxyéthyle, l'acrylate d'hydroxypropyle, l'acrylate d'hydroxyéthyle éthoxylé (2), l'acrylate de glycidyle, l'acide méthacrylique, le méthacrylate de méthyle, le méthacrylate d'éthyle, le méthacrylate de butyle, le méthacrylate de 2-hydroxyéthyle, le méthacrylate de cyclohexyle, le méthacrylate de polyéthylène glycol, le diacrylate de polyéthylène glycol, le diméthacrylate de polyéthylène glycol, le triacrylate de triméthylolpropane, le diacrylate de triéthylène glycol, le diacrylate de tétraéthylène glycol, le diacrylate de néopentyle glycol, le diacrylate de diéthylène glycol, l'hexaacrylate de dipentaérythritol, le triacrylate de triméthylolpropane éthoxylé, le triacrylate de glycérol propoxylé, l'acrylate de stéaryle, l'acrylate de lauryle, l'acrylate d'isodécyle, l'acide acrylique, le diacrylate d'éthylène glycol (EGDA), le diacrylate de triéthylène glycol (TEGDA), le diacrylate de propylène glycol (PGDA), le diacrylate de butanediol (BDDA), le diacrylate de néopentylglycol (NPGDA), le tétraacrylate de pentaérythritol (PETA), le diacrylate de 1,4-butanediol (BDA), le tétraacrylate de di(triméthylolpropane) (DTMPTA), le diacrylate d'éthoxylate de bisphénol A (BPAEDA), le diacrylate de bisphénol A éthoxylé (EBPA), le diacrylate de décanediol, le diacrylate de polyéthylène glycol (PEGDA), le triacrylate de triméthylolpropane (TMPTA), le diacrylate de diéthylène glycol (DEGDA) et le diacrylate de 1,6-hexanediol (HDDA), le triacrylate de triméthylolpropane (TMPTA), le diacrylate de tripropylène glycol (TPGDA), le triacrylate de pentaérythritol (PETA), le pentaacrylate de dipentaérythritol (DPEPA), le triacrylate d'isocyanurate de tris(2-hydroxyéthyle) (THEIC-TA), le diméthacrylate de triéthylène glycol (TEGDMA), l'isocyanurate de triallyle (TAIC), le diacrylate de triéthylène glycol (TEGDA), le triacrylate de trimethylolpropane éthoxylé (ETMPTA), le cyanurate de triallyle (TAC), et leurs combinaisons, et/ou
ledit polymère sensible aux stimuli comprenant au moins 10 % en poids de monomères d'acrylate, par exemple, au moins 50 % en poids, au moins 65 % en poids, au moins 95 % en poids, au moins 96 % en poids, au moins 97 % en poids, au moins 98 % en poids ou au moins 99 % en poids ;
(ii) des monomères de méthacrylate, de préférence :
lesdits monomères de méthacrylate étant des monomères de méthacrylate d'alkyle en C6 à C30, de préférence des monomères de méthacrylate d'alkyle en C8 à C30, plus préférablement des monomères de méthacrylate d'alkyle en C8 à C20, plus préférablement des monomères de méthacrylate d'alkyle en C8 à C16, de préférence
lesdits monomères de méthacrylate d'alkyle en C8 à C30 étant choisis parmi le méthacrylate d'octyle, le méthacrylate de nonyle, le méthacrylate de décyle, le méthacrylate d'undécyle, le méthacrylate de dodécyle, le méthacrylate de tridécyle, le méthacrylate de tétradécyle, le méthacrylate de pentadécyle, le méthacrylate d'hexadécyle, le méthacrylate d'heptadécyle, le méthacrylate d'octadécyle, le méthacrylate de nonadécyle, le méthacrylate d'éicosyle, le méthacrylate d'hénéicosyle, le méthacrylate de docosyle, le méthacrylate de tricosyle, le méthacrylate de tétracosyle, le méthacrylate de pentacosyle, le méthacrylate d'hexacosyle, le méthacrylate d'heptacosyle, le méthacrylate d'octacosyle, le méthacrylate de nonacosyle, le méthacrylate de triacontyle, et leurs combinaisons ; ou
lesdits monomères de méthacrylate étant choisis parmi le méthacrylate d'éthyle, le méthacrylate de butyle, le méthacrylate de 2-hydroxyéthyle, le méthacrylate de cyclohexyle, le méthacrylate d'isbornyle, le méthacrylate de stéaryle, le méthacrylate de lauryle, le méthacrylate d'isodécyle, le méthacrylate de tétrahydrofurfuryle, le méthacrylate de glycéryle, le triméthacrylate de triméthylolpropane, le triacrylate de triméthylolpropane, le triacrylate de pentaérythritol, le tétraméthacrylate de pentaérythritol, le méthacrylate d'éther monométhylique de polyéthylène glycol, l'acrylate d'éther monométhylique de polyéthylène glycol, le diacrylate de polyéthylène glycol, le diméthacrylate de polyéthylène glycol, le monoacrylate de polyéthylène glycol, le diméthacrylate de bisphénol A éthoxylé, le diacrylate de bisphénol A éthoxylé, le triacrylate de triméthylolpropane éthoxylé, le méthacrylate d'hydroxypropyle, l'acide méthacrylique, le chlorure d'acryloyloxyéthyltriméthylammonium, le méthacrylate de diéthylaminoéthyle, le méthacrylate de butylaminoéthyle, le méthacrylate de N,N-diméthylaminoéthyle, le phtalate de méthacryloyloxyéthyle, le méthacrylate de cyclopropyle, et leurs combinaisons, et/ou
ledit polymère sensible aux stimuli comprenant du polyméthacrylate de lauryle, ou
ledit polymère sensible aux stimuli comprenant du polyméthacrylate de lauryle et un ou plusieurs autres polyméthacrylates, ou
ledit adhésif polymère sensible aux stimuli comprenant du polyméthacrylate de lauryle et un ou plusieurs polyacrylates, et/ou
ledit polymère sensible aux stimuli comprenant au moins 10 % en poids de monomères de méthacrylate, par exemple, au moins 50 % en poids, au moins 65 % en poids, au moins 95 % en poids, au moins 96 % en poids, au moins 97 % en poids, au moins 98 % en poids ou au moins 99 % en poids ;
(iii) des monomères d'éther vinylique choisis parmi l'éther divinylique de l'éthylène glycol, l'éther divinylique du diéthylène glycol, l'éther divinylique du triéthylène glycol, l'éther divinylique du polyéthylène glycol (DVE-PEG), l'éther divinylique du polypropylène glycol (DVE-PPG), l'éther divinylique de l'éther méthylique de polyéthylène glycol (DVE-PEGME), l'éther divinylique de l'éther butylique de polyéthylène glycol (DVE), l'éther divinylique de l'éther phénylique du polyéthylène glycol (DVE-PEGPhE), l'éther divinylique du glycérol (DVE-Gly), l'éther divinylique du 1,4-cyclohexanediméthanol (DVE-CHDM), l'éther divinylique du néopentyl glycol (DVE-NPG), et leurs combinaisons, de préférence
ledit polymère sensible aux stimuli comprenant en outre des monomères d'acrylate, des monomères de méthacrylate, des monomères d'allyle, des monomères de thiol, des monomères d'époxy, des monomères d'amine, des monomères riches en électrons, des monomères pauvres en électrons, des monomères de lactame, des monomères de lactone, des monomères d'alcool, des monomères d'acide carboxylique, des monomères d'isocyanate, des monomères de Diels-Alder, des monomères de métathèse avec ouverture de cycle, ou une combinaison de ceux-ci ;
(iv) des monomères d'allyle choisis parmi le phtalate de diallyle (DAP), le maléate de diallyle (DAM), le succinate de diallyle (DAS), le fumarate de diallyle (DAF), l'adipate de diallyle (DAA), le sébacate de diallyle (DAS), le téréphtalate de diallyle (DAT), l'isophtalate de diallyle (DAI), l'itaconate de diallyle (DAI), le carbonate de diallyle (DAC), le diglycolate de diallyle (DADG), le tris(2-hydroxyéthyl) isocyanurate de diallyle (DATHEIC), le cyanurate de triallyle (TAC), l'isocyanurate de triallyle (TAIC), le trimellitate de triallyle (TATM), le citrate de triallyle (TAC), le phosphate de triallyle (TAP), la triallylamine (TAA), le cyanure de triallyle (TACN), le benzène-1,2,4-tricarboxylate de triallyle (TABTC), le trimésate de triallyle (TATM), l'éther triallylique du tris(2-hydroxyéthyl)isocyanurate, et les combinaisons de ceux-ci, de préférence
ledit polymère sensible aux stimuli comprenant en outre des monomères d'acrylate, des monomères de méthacrylate, un monomère d'éther vinylique, des monomères de thiol, des monomères d'époxy, des monomères d'amine, des monomères riches en électrons, des monomères pauvres en électrons, des monomères de lactame, des monomères de lactone, des monomères d'alcool, des monomères d'acide carboxylique, des monomères d'isocyanate, des monomères de Diels-Alder, des monomères de métathèse avec ouverture de cycle, ou une combinaison de ceux-ci ;
(v) des monomères de thiol choisis parmi l'acide 3-mercaptopropionique ; l'acide thioglycolique ; le 3-mercapto-1-propanol ; le 2-mercaptoéthanol ; le 2-(2-mercaptoéthoxy)éthanol ; le 2-(2-mercaptopropionylamino)éthanol ; l'acrylate de 2-(2-mercaptosuccinyl)éthyle ; l'acide 3-(2-mercaptopropionylamino)propionique ; le 3-(mercaptopropyl)triméthoxysilane ; le 2,2'-(éthylènebis(thio))diéthanol ; le 3-mercaptopropyltriméthoxysilane ; le 3-mercaptopropylméthyldiméthoxysilane ; l'acide 3-(2,2-dithiobis(éthylthio)propionylamino)propionique ; le 3,6,9-trioxadécanéthiol ; le 3-mercapto-1,2-propanediol ; le 2,2'-dithiodiéthanol ; la N-acétyl-L-cystéine ; la L-cystéine ; la 2-(2-mercaptoéthyl)pyridine ; la 4-(2-mercaptoéthyl)morpholine ; le 3-mercapto-1,2,4-triazole ; le thiophénol ; le tetrakis(3-mercaptopropionate) de pentaérythritol (PETMP); le tris(3-mercaptopropionate) de triméthylolpropane (TMPMP) ; le tris(3-mercaptopropionate) de triéthanolamine (TEAMP) ; le tris(3-mercaptopropionate) de tris(2-hydroxyéthyl)isocyanurate (THEICMP) ; le sulfure de bis(3-mercaptopropyle) (BMPS) ; le 1,2-éthanedithiol (EDT) ; le 1,3-propanedithiol ; le 1,4-butanedithiol ; le 1,6-hexanedithiol ; le 1,8-octanedithiol et leurs combinaisons, de préférence
ledit polymère sensible aux stimuli comprenant en outre des monomères d'acrylate, des monomères de méthacrylate, un monomère d'éther vinylique, des monomères d'allyle, des monomères d'époxy, des monomères d'amine, des monomères riches en électrons, des monomères pauvres en électrons, des monomères de lactame, des monomères de lactone, des monomères d'alcool, des monomères d'acide carboxylique, des monomères d'isocyanate, des monomères de Diels-Alder, des monomères de métathèse avec ouverture de cycle, ou une combinaison de ceux-ci ;
(vi) des monomères d'époxy choisis parmi l'éther diglycidylique du bisphénol A (BADGE), l'éther diglycidylique du bisphénol F (BFDGE), l'éther diglycidylique du novolaque (NGDE), l'éther diglycidylique du novolaque de phénol (PNGDE), les résines époxydes cycloaliphatiques, les éthers glycidyliques d'alcools aliphatiques, les éthers glycidyliques d'alcools aromatiques, l'isocyanurate de triglycidyle (TGIC), l'éther diglycidylique du 1,4-butanediol (BDDGE), l'éther diglycidylique du néopentyl glycol (NPGDGE), l'éther diglycidylique du propylène glycol (PGDGE), l'huile de soja époxydée (ESO), l'huile de lin époxydée (ELO), les résines époxydes à base de dicyclopentadiène, les résines époxydes tétrafonctionnelles, les résines époxyphénoliques du novolaque, et leurs combinaisons, de préférence
comprenant en outre des monomères d'acrylate, des monomères de méthacrylate, un monomère d'éther vinylique, des monomères d'allyle, des monomères de thiol, des monomères d'amine, des monomères riches en électrons, des monomères pauvres en électrons, des monomères de lactame, des monomères de lactone, des monomères d'alcool, des monomères d'acide carboxylique, des monomères d'isocyanate, des monomères de Diels-Alder, des monomères de métathèse avec ouverture de cycle, ou une combinaison de ceux-ci ;
(vii) des monomères d'amine choisis parmi l'éthylènediamine ; la diéthylènetriamine ; la triéthylènetétramine ; la tétraéthylènepentamine ; la polyéthylèneimine ; le diaminopropane ; le diaminobutane ; le diaminopentane ; l'acide diéthylènetriaminepentaacétique (DTPA) ; la tris(2-aminoéthyl)amine ; la N-(2-aminoéthyl)pipérazine ; la N-(3-aminopropyl)morpholine ; la N,N-diméthylaminopropylamine ; la N,N-diméthyléthylènediamine ; le 1,3-diaminopropane ; l'isophoronediamine ; la Jeffamine D-230 ; la Jeffamine T-403 ; la Jeffamine M-207 ; la Jeffamine EDR-148 ; et leurs combinaisons, de préférence
ledit polymère sensible aux stimuli comprenant en outre des monomères d'acrylate, des monomères de méthacrylate, un monomère d'éther vinylique, des monomères d'allyle, des monomères de thiol, des monomères d'époxy, des monomères riches en électrons, des monomères pauvres en électrons, des monomères de lactame, des monomères de lactone, des monomères d'alcool, des monomères d'acide carboxylique, des monomères d'isocyanate, des monomères de Diels-Alder, des monomères de métathèse avec ouverture de cycle, ou une combinaison de ceux-ci ;
(viii) des monomères riches en électrons choisis parmi les éthers vinyliques (par exemple, l'éther méthylique de vinyle, l'éther éthylique de vinyle), l'acétate de vinyle, l'alcool allylique, l'amine d'allyle, le N-méthylolacrylamide, le N-méthylolméthacrylamide, la N-méthylolallylamine, le N-méthylolvinylacétamide, l'acétal diéthylique d'acroléine, le cétal diéthylique d'acroléine, l'acrylamide de diacétone, l'acrylate de 2-hydroxyéthyle, le méthacrylate de 2-hydroxyéthyle, l'acrylate de 2-hydroxypropyle, le méthacrylate de 2-hydroxypropyle, le méthacrylate de 2,3-dihydroxypropyle, le méthacrylate de glycidyle, l'acrylate de glycidyle, le méthacrylate de tétrahydrofurfururyle, et la N-vinylpyrrolidone, le N-vinylformamide, la N-vinylpyridine, le styrène, les dérivés du styrène, et leurs combinaisons, de préférence
ledit polymère sensible aux stimuli comprenant en outre des monomères d'acrylate, des monomères de méthacrylate, un monomère d'éther vinylique, des monomères d'allyle, des monomères de thiol, des monomères d'époxy, des monomères d'amine, des monomères pauvres en électrons, des monomères de lactame, des monomères de lactone, des monomères d'alcool, des monomères d'acide carboxylique, des monomères d'isocyanate, des monomères de Diels-Alder, des monomères de métathèse avec ouverture de cycle, ou une combinaison de ceux-ci ;
(ix) des monomères pauvres en électrons choisis parmi l'acrylonitrile, le méthacrylonitrile, le méthacrylate de méthyle, l'acide acrylique, l'acide méthacrylique, l'anhydride maléique, l'acide itaconique, l'acide fumarique, l'acrylamide, le méthacrylamide, le N-vinylcarbazole, le chlorure de vinylidène, le chlorure de vinyle, l'acide vinylsulfonique, l'acétate de vinyle, le styrène, l'alpha-méthylstyrène, le maléimide, le N-phénylmaléimide et le N-butylmaléimide, l'anhydride maléique et leurs combinaisons, de préférence
ledit polymère sensible aux stimuli comprenant en outre des monomères d'acrylate, des monomères de méthacrylate, un monomère d'éther vinylique, des monomères d'allyle, des monomères de thiol, des monomères d'époxy, des monomères d'amine, des monomères riches en électrons, des monomères de lactame, des monomères de lactone, des monomères d'alcool, des monomères d'acide carboxylique, des monomères d'isocyanate, des monomères de Diels-Alder, des monomères de métathèse avec ouverture de cycle, ou une combinaison de ceux-ci ;
(x) des monomères de lactame choisis parmi le caprolactame, le valérolactame, l'énantholactame, le capryllactame, le laurinlactame, le prolactame, le butyrolactame, le méthionyl lactame, le méthoxyéthyl lactame, le méthoxyéthyl méthionyl lactame, le diméthylaminoéthyl lactame, le diméthylaminoéthyl méthionyl lactame, le diméthylaminoéthyl acryloyl lactame, le diméthylaminoéthyl méthacryloyl lactame, la N-vinylpyrrolidone, la N-méthylpyrrolidone, la N-éthylpyrrolidone et leurs combinaisons, de préférence
ledit polymère sensible aux stimuli comprenant des monomères d'acrylate, des monomères de méthacrylate, un monomère d'éther vinylique, des monomères d'allyle, des monomères de thiol, des monomères d'époxy, des monomères d'amine, des monomères riches en électrons, des monomères pauvres en électrons, des monomères de lactone, des monomères d'alcool, des monomères d'acide carboxylique, des monomères d'isocyanate, des monomères de Diels-Alder, des monomères de métathèse avec ouverture de cycle, ou une combinaison de ceux-ci ;
(xi) des monomères de lactone choisis parmi la β-propiolactone, la γ-butyrolactone, la 8-valérolactone, la ε-caprolactone, la ω-pentadécalactone, la β-butyrolactone, la 8-décalactone, la ε-décalactone, la γ-décalactone, la 8-dodécalactone, la γ-dodécalactone, l'α-méthylène-γ-butyrolactone, la β-méthyl-γ-butyrolactone, la β-méthyl-γ-valérolactone, la γ-hexalactone et leurs combinaisons, de préférence
ledit polymère sensible aux stimuli comprenant en outre des monomères d'acrylate, des monomères de méthacrylate, un monomère d'éther vinylique, des monomères d'allyle, des monomères de thiol, des monomères d'époxy, des monomères d'amine, des monomères riches en électrons, des monomères pauvres en électrons, des monomères de lactame, des monomères d'alcool, des monomères d'acide carboxylique, des monomères d'isocyanate, des monomères de Diels-Alder, des monomères de métathèse avec ouverture de cycle, ou une combinaison de ceux-ci ;
(xii) des monomères d'alcool choisis parmi l'éthylène glycol ; le propylène glycol ; le 1,3-butanediol ; le 1,4-butanediol ; le 1,5-pentanediol ; le 1,6-hexanediol ; le 1,10-décanediol ; le néopentyl glycol ; le diéthylène glycol ; le triéthylène glycol ; le tétraéthylène glycol ; le polyéthylène glycol (PEG) ; le polypropylène glycol (PPG) ; le polycaprolactone diol ; le polyméthylolpropane ; l'hydroxyméthylbutyrate d''ydroxypivalyle (HPHMB) ; le 1,4-cyclohexanediméthanol ; et leurs combinaisons, de préférence
ledit polymère sensible aux stimuli comprenant en outre des monomères d'acrylate, des monomères de méthacrylate, un monomère d'éther vinylique, des monomères d'allyle, des monomères de thiol, des monomères d'époxy, des monomères d'amine, des monomères riches en électrons, des monomères pauvres en électrons, des monomères de lactame, des monomères de lactone, des monomères d'acide carboxylique, des monomères d'isocyanate, des monomères de Diels-Alder, des monomères de métathèse avec ouverture de cycle, ou une combinaison de ceux-ci ;
(xiii) des monomères d'acide carboxylique choisis parmi l'acide adipique, l'acide succinique, l'acide glutarique, l'acide sébacique, l'acide malonique, l'acide phtalique, l'acide isophtalique, l'acide téréphtalique, l'acide fumarique, l'acide maléique, l'acide itaconique, l'acide citrique, l'acide 1,4-cyclohexanedicarboxylique, l'acide 1,3-cyclohexanedicarboxylique, l'acide dodécanedioïque et leurs combinaisons, de préférence
ledit polymère sensible aux stimuli comprenant en outre des monomères d'acrylate, des monomères de méthacrylate, un monomère d'éther vinylique, des monomères d'allyle, des monomères de thiol, des monomères d'époxy, des monomères d'amine, des monomères riches en électrons, des monomères pauvres en électrons, des monomères de lactame, des monomères de lactone, des monomères d'alcool, des monomères d'isocyanate, des monomères de Diels-Alder, des monomères de métathèse avec ouverture de cycle, ou une combinaison de ceux-ci ;
(xiv) des monomères d'isocyanate choisis parmi le diisocyanate de toluène (TDI), le diisocyanate de diphénylméthane (MDI), le diisocyanate d'hexaméthylène (HDI), le diisocyanate d'isophorone (IPDI), le diisocyanate de 1,6-hexaméthylène (HMDI), le 4,4'-méthylènebis(isocyanate de cyclohexyle) (H12MDI), le diisocyanate de naphtalène (NDI), le diisocyanate de 2,4-toluène (2,4-TDI), le diisocyanate de 2,6-toluène (2,6-TDI), le polyphényl isocyanate de polyméthylène (PAPI), le Desmodur N-100, le Desmodur L-75, le Desmodur HL, le Desmodur H, le Desmodur VP, le Desmodur Z et leurs combinaisons, de préférence
ledit polymère sensible aux stimuli comprenant en outre des monomères d'acrylate, des monomères de méthacrylate, un monomère d'éther vinylique, des monomères d'allyle, des monomères de thiol, des monomères d'époxy, des monomères d'amine, des monomères riches en électrons, des monomères pauvres en électrons, des monomères de lactame, des monomères de lactone, des monomères d'alcool, des monomères d'acide carboxylique, des monomères de Diels-Alder, des monomères de métathèse avec ouverture de cycle, ou une combinaison de ceux-ci ;
(xv) des monomères de Diels-Alder choisis parmi l'anhydride maléique, le furane, le cyclopentadiène, le N-phénylmaléimide, l'anthracène, le N-éthylmaléimide, le N-phénylnorbornène,le N, N-diméthylmaléimide, le 2,5-diméthylfurane, le tétracyanoéthylène, la méthylvinylcétone et leurs combinaisons, de préférence
ledit polymère sensible aux stimuli comprenant en outre des monomères d'acrylate, des monomères de méthacrylate, un monomère d'éther vinylique, des monomères d'allyle, des monomères de thiol, des monomères d'époxy, des monomères d'amine, des monomères riches en électrons, des monomères pauvres en électrons, des monomères de lactame, des monomères de lactone, des monomères d'alcool, des monomères d'acide carboxylique, des monomères d'isocyanate, des monomères de métathèse avec ouverture de cycle, ou une combinaison de ceux-ci ; ou
(xvi) des monomères de métathèse avec ouverture de cycle choisis parmi le norbornène, le dicyclopentadiène (DCPD), le cyclooctène, le tétracyclododécène (TCD), le cyclopentène, le cycloheptène, le cyclohexène, le bicyclo[2.2.1]hept-2-ène, le bicyclo[2.2.2]oct-5-ène, le tricyclo[5.2.1.0(2,6)]déc-8-ène (TCD-Diène) et leurs combinaisons, de préférence
ledit polymère sensible aux stimuli comprenant en outre des monomères d'acrylate, des monomères de méthacrylate, un monomère d'éther vinylique, des monomères d'allyle, des monomères de thiol, des monomères d'époxy, des monomères d'amine, des monomères riches en électrons, des monomères pauvres en électrons, des monomères de lactame, des monomères de lactone, des monomères d'alcool, des monomères d'acide carboxylique, des monomères d'isocyanate, des monomères de Diels-Alder, ou une combinaison de ceux-ci.

15. Préservatif selon l'une quelconque des revendications 1 à 14, ledit agent de réticulation polyfonctionnel étant choisi parmi : un agent de réticulation difonctionnel, un agent de réticulation trifonctionnel, ou un agent de réticulation tétrafonctionnel, de préférence
ledit agent de réticulation polyfonctionnel étant un agent de réticulation trifonctionnel, de préférence
ledit agent de réticulation trifonctionnel étant un agent de réticulation acrylate ;
le diacrylate de polyéthylène glycol, le triacrylate de triméthylolpropane ; le triacrylate de triméthyolpropane éthoxylé ; le tétraacrylate de pentaérythritol ; le tétraacrylate de pentaérythritol éthoxylé ; l'hexaacrylate de dipentaérithrotol ; l'hexaacrylate de dipentaérithrotol éthoxylé ; les di-, tri-, tétra-, penta- ou hexa-époxydes ; les polythiols ; les polyalcènes ; l'isocyanulate de tris(2-acryloxyéthyle), l'isocyanurate de tris(2-acryloxyéthyle) modifié par la ε-caprolactone, le triacrylate de glycérine éthoxylé, le triacrylate de glycérine éthoxylé, le triacrylate de pentaérythritol, et leurs combinaisons, et/ou
ledit polymère sensible aux stimuli comprenant de 0,001 à 29 % en poids, de préférence de 0,1 % en poids à 1,5 % en poids d'un agent de réticulation polyfonctionnel, de préférence de 0,2 % en poids à 1 % en poids, plus préférablement de 0,4 % en poids à 0,8 % en poids, et/ou
ledit rapport pondéral desdits un ou plusieurs monomères auxdits un ou plusieurs agents de réticulation polyfonctionnels étant de 98:2 à 99,9:0,1, par exemple de 98,5:1,5 à 99,9:0,1 ; de 99:1 à 99,9:0,1, de 99,1:0,9 à 99,9:0,1, de 99,2:0,8 à 99,9:0,1 ; de 99,3:0,7 à 99,9:0,1, de 99,4:0,6 à 99,9:0,1, de 99,5:0,5 à 99,9:0,1, de 99,6:0,4 à 99,9:0,1, de 99,7:0,3 à 99,9:0,1, ou de 99,8:0,2 à 99,9:0,1.

16. Préservatif selon la revendication 1, ledit polymère sensible aux stimuli comprenant un polymère de polyméthacrylate de lauryle réticulé avec un ou plusieurs agents de réticulation polyfonctionnels, de préférence :
ledit agent de réticulation polyfonctionnel étant choisi parmi un agent de réticulation difonctionnel, un agent de réticulation trifonctionnel, ou un agent de réticulation tétrafonctionnel, de préférence
ledit agent de réticulation polyfonctionnel étant un agent de réticulation trifonctionnel, de préférence
ledit agent de réticulation trifonctionnel étant un agent de réticulation acrylate ;
ledit agent de réticulation trifonctionnel étant choisi parmi le diacrylate de polyéthylène glycol, le triacrylate de triméthylolpropane ; le triacrylate de triméthyolpropane éthoxylé ; le tétraacrylate de pentaérythritol ; le tétraacrylate de pentaérythritol éthoxylé ; l'hexaacrylate de dipentaérithrotol ; l'hexaacrylate de dipentaérithrotol éthoxylé ; les di-, tri-, tétra-, penta- ou hexa-époxydes ; les polythiols ; les polyalcènes ; l'isocyanulate de tris(2-acryloxyéthyle), l'isocyanurate de tris(2-acryloxyéthyle) modifié par la ε-caprolactone, le triacrylate de glycérine éthoxylé, le triacrylate de glycérine éthoxylé, le triacrylate de pentaérythritol, et leurs combinaisons, et/ou
ledit rapport pondéral du méthacrylate de lauryle auxdits un ou plusieurs agents de réticulation polyfonctionnels étant de 98:2 à 99,9:0,1, par exemple de 98,5:1,5 à 99,9:0,1 ; de 99:1 à 99,9:0,1, de 99,1:0,9 à 99,9:0,1, de 99,2:0,8 à 99,9:0,1 ; de 99,3:0,7 à 99,9:0,1, de 99,4:0,6 à 99,9:0,1, de 99,5:0,5 à 99,9:0,1, de 99,6:0,4 à 99,9:0,1, de 99,7:0,3 à 99,9:0,1, ou de 99,8:0,2 à 99,9:0,1.

17. Préservatif selon l'une quelconque des revendications 1 à 16, ladite couche adhésive étant transparente.
